# EUROPEAN PATENT APPLICATION

(11) **EP 2 108 406 A1**
(43) Date of publication of application: **14.10.2009**
(21) Application number: 08103498.5
(22) Date of filing: 11.04.2008
(51) Int. Cl.: A61Q 5/06, A61Q 5/10, A61K 8/49, C09B 29/12, C09B 29/14, C09B 29/16, C09B 29/36, C09B 29/033, C09B 29/036, C09B 29/039, C09B 29/042, C09B 29/045, C09B 29/048

(54) **Anionic dyes and hair dye compositions containing them**

(71) Applicant: DyStar Textilfarben GmbH & Co. Deutschland KG, 65926 Frankfurt (DE)
(72) Inventor: Barbieru, Roxana, 65929 Frankfurt (DE); Russ, Werner, 65439 Flörsheim-Wicker (DE)
(74) Representative: Muley, Ralf

(57) **Abstract**

The present invention refers to dyestuffs of the formula (I) 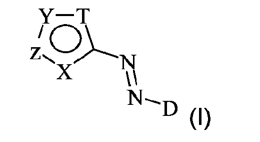 and its tautomeric forms, wherein
D is a group of the formula D¹, D², D³, D⁴ or D⁵ 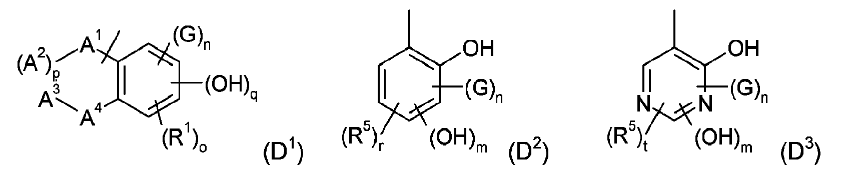 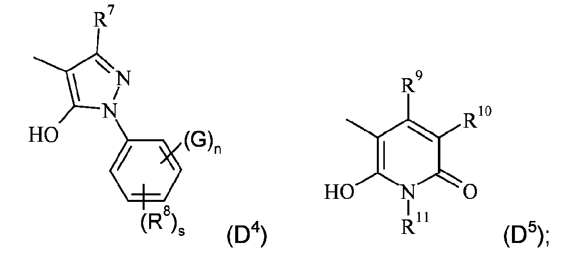 wherein T, X, Y, Z, A¹ to A⁴, G, R¹, R⁵, R⁷ to R¹¹, m, n, o, p, q, r, s and t are defined as given in claim 1, a process for their manufacture and hair dye compositions containing them.

## Description

The present invention relates to anionic dyes and dye compositions containing them for coloring keratin fibers.

For dyeing of keratin fibers, especially human hair two main coloring methods are usually used. In the first method, the dyeing is achieved with so-called oxidative coloring systems or permanent colorants by using a mixture of various developers and couplers and an oxidizing agent. The developers and couplers are usually small and colorless molecules with good water solubility, which diffuse into the hair and then in the presence of oxidizing agents undergo chemical reactions to form larger chromophore molecules, which are then trapped inside of the hair shaft. Furthermore direct dyes can be added to affect the final shade or to obtain special coloristic effects. This coloring technology has been used for over 50 years with good results, especially in covering natural gray shades. Nevertheless there are some critical issues with this first method such as toxicological and dermatological issues (sensitization), limited performance in fashion shade area, poor fastness properties and lack of options for some shade areas such as the blue area.
The second dyeing process is based on the exclusive use of direct substantive dyes that are applied to the fibers using suitable formulations. Compared to the oxidative systems, the hair direct dye formulations are accompanied by the drawback that the color imparted to the hair typically displays low durability. Furthermore many direct dyes are not stable to oxidizing agents at alkaline pH values greater than or equal to 9 necessary for bleaching the hair, so that direct colorants are generally not able to lighten and impart color to the hair in one step.
EP 1 366 752 A1, EP 1 849 454 A1 and JP 2004-262888 disclose hair dye compositions which contain direct dissociative azo dyes. Such dyes can strongly impart the hair with a vivid color without decomposition of the dye upon hair dyeing. However, said compositions still have drawbacks and consequently there is a constant need for new dyestuffs which provide improved colour and toxicological properties.

Surprisingly, it has now been found, that azo dyes containing particular structural characteristics such as special functional groups in specific configuration to each other and bearing anionic groups can dye keratin fibers in a broad pH-range inclusively high alkaline despite their anionic charges in uniform, very intense, brilliant and durable shades of high wash fastness. In addition these new anionic direct dyes are stable towards oxidizing agents inclusively to alkaline peroxide and thus can be also used in oxidative coloring systems of acidic, neutral or alkaline pH.

Accordingly, the present invention refers to dyestuffs of the formula (I) 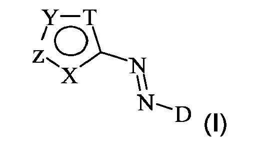 and its tautomeric forms, wherein D is a group of the formula D¹, D², D³, D⁴ or D⁵ 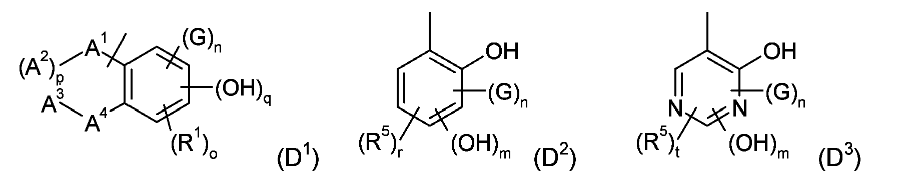 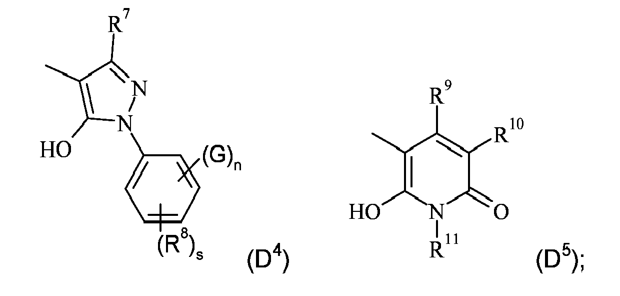 X is oxygen, sulphur, nitrogen, -N(R¹²)- or -C(R¹³)-;
Y is oxygen, sulphur, -C(R¹⁴)-, nitrogen or -N(R¹⁵)-;
Z is nitrogen or -C(R¹⁶)-;
T is nitrogen, sulphur or -C(R¹⁷)-; with the proviso that at least two of the groups X, Y, Z and T are heteroatoms; each of A¹, A², A³ and A⁴, independently, is oxygen, nitrogen, -N(R¹⁸)-, -C(R¹⁹)=, -C(R²⁰)(R²¹)- or -C(=R²²)-;
G is -SO₃M or -COOM;
M is hydrogen or a cosmetically acceptable, water solubilizing inorganic or organic cation;
n is an integer from 0 to 4, with the proviso, that when n is 0, at least one of the
groups A¹, A², A³, A⁴, X, Y Z and T carries a group G;
m is an integer from 0 to 3;
p is an integer from 0 to 1;
q is an integer from 1 to 4;
r is an integer from 0 to 4;
s is an integer from 0 to 3;
t is an integer from 0 to 2;
o is an integer from 1 to 3;
wherein in the group of the formula D² m+n+r is from 0 to 4 and in the group of the formula D³ m+n+r is from 0 to 2;
each of R¹¹, R¹², R¹⁵, R¹⁸ and R²³, independently, is hydrogen; alkyl; arylalkyl; alkoxyalkyl; alkylthioalkyl; poly(oxyalkylene)alkyl; N-acyl-aminoalkyl; N-alkylsulfonyl-aminoalkyl; aminothiocarbonyl-aminoalkyl; aminocarbonyl-aminoalkyl; alkoxyalkylaminoalkyl; alkylthioalkyl-aminoalkyl; aminoalkyloxyalkyl; N-monoalkylamino-alkyloxyalkyl; N,N-dialkyl-aminoalkoxyalkyl; N-arylamino-alkoxyalkyl; N,N-diarylamino-alkoxyalkyl; N-alkyl-N-aryl-amino-alkoxyalkyl; aminoalkylthioalkyl; N-monoalkylamino-alkylthioalkyl; N,N-dialkylamino-alkylthioalkyl; N-arylamino-alkylthioalkyl; N,N-diarylamino-alkylthioalkyl; N-alkyl-N-aryl-amino-alkylthioalkyl; alkenyl; alkynyl; cycloalkyl; cycloalkylalkyl; aryl; aryloxyalkyl; arylthioalkyl; heteroaryl; heteroarylalkyl; heterocycloalkyl; heterocycloalkylalkyl; acyl; benzoyl; alkoxycarbonyl; aryloxycarbonyl; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; acylthio; alkylsulfonyl or arylsulfonyl;
or is alkyl substituted by one or more substituents selected from the group consisting of G, hydroxy, amino, N-monoalkyl-amino, N,N-dialkyl-amino, N-monoaryl-amino, N,N-diaryl-amino, N-alkyl-N-aryl-amino, halogen, cyano, nitro, alkoxycarbonyl, alkoxythiocarbonyl; acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoaryl-carbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl and N-monoalkyl-N-monoarylsulfamoyl;
each of R¹, R⁵, R⁷, R⁸, R⁹, R¹⁰, R¹³, R¹⁴, R¹⁶, R¹⁷, R¹⁹, R²⁰ and R²¹, independently, is hydrogen; alkyl; trifluoromethyl; alkenyl; alkynyl; cycloalkyl; aryl; heteroaryl; heterocycloalkyl; halogen; cyano; nitro; alkoxy; aryloxy; formyl; iminomethyl; N-alkyl-iminomethyl; N-cycloalkyl-iminomethyl; N-aryl-iminomethyl; hydroxyiminomethyl; thioacyl; arylcarbonyl, arylthiocarbonyl; acyloxy; arylcarbonyloxy; acylamino; N-alkyl-acylamino; N-cycloalkyl-acylamino; N-arylacylamino; alkylsulfonylamino; cycloalkylsulfonylamino, N-alkyl-alkylsulfonylamino; N-alkyl-cycloalkylsulfonylamino; N-cycloalkyl-alkylsulfonylamino; N-cycloalkyl-cycloalkyl-sulfonylamino; N-arylalkylsulfonylamino; N-arylcycloalkylsulfonylamino; arylsulfonylamino; N-alkyl-arylsulfonylamino; N-cycloalkyl-arylsulfonylamino; N-arylarylsulfonylamino; arylcarbonylamino; arylthiocarbonylamino; N-alkyl- arylthiocarbonylamino; N-alkyl-arylcarbonylamino; N-cycloalkyl-arylthiocarbonylamino; N-cycloalkyl-arylcarbonylamino; N-aryl-arylthiocarbonylamino; N-aryl-arylcarbonylamino; carbamoyl; N-monocycloalkyl-carbamoyl; N-monoalkyl-carbamoyl; N,N-dicycloalkyl-carbamoyl; N,N-dialkyl-carbamoyl; N-monoaryl-carbamoyl; N,N-diaryl-carbamoyl; N-monocycloalkyl-N-monoarylcarbamoyl; N-monoalkyl-N-monoarylcarbamoyl; alkoxycarbonyl; aryloxycarbonyl; amino;
monocycloalkyl-amino; monoalkyl-amino; di(cyclo)alkyl-amino; dialkyl-amino; monoaryl-amino; diaryl-amino; monocycloalkylmonoarylamino; monoalkylmonoarylamino; aminothiocarbonylamino; aminocarbonylamino; N-N-dicycloalkyl-aminothiocarbonylamino; N-N-dialkyl-aminothiocarbonylamino; N-N-dicycloalkyl-aminocarbonylamino; N-N-dialkyl-aminocarbonylamino; N,N-diaryl-aminothiocarbonylamino; N,N-diaryl-aminocarbonylamino; N,N-dialkylaminothio-carbonyl-N-alkylamino; N,N-dialkylaminocarbonyl-N-alkylamino; alkoxycarbonylamino; aryloxycarbonylamino; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; aminosulfonylamino; N-monocycloalkyl-aminosulfonylamino; N-monoalkyl-aminosulfonylamino; N,N-dicycloalkyl-aminosulfonylamino; N,N-dialkyl-aminosulfonylamino; N-monoaryl-aminosulfonylamino; N,N-diaryl-aminosulfonylamino; N-monocycloalkyl-N-monoarylaminosulfonyl-amino; N-monoalkyl-N-monoarylaminosulfonyl-amino; -S-G; alkylthio; arylthio; alkylsulfonyl; arylsulfonyl;
or is alkyl, N-monoalkylamino, N-monocycloalkylamino, N,N-dialkylamino, N,N-dicycloalkylamino, N-alkyl-N-cycloalkylamino, N-alkyl-N-arylamino, N-aryl-N-cycloalkylamino, alkoxy, alkylthio or alkylsulfonyl substituted by one or more substituents selected from the group consisting of hydroxy, cycloalkyl, heteroaryl, heterocycloalkyl, aryl, aryloxy, alkoxy, alkylthio, arykthio. poly(oxyalkylene), G, halogen, cyano, alkoxycarbonyl, alkoxythiocarbonyl, acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoarylcarbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl, N-monoalkyl-N-monoarylsulfamoyl, nitro, amino, acylamino, arylcarbonylamino, alkylsulfonylamino, arylsulfonylamino, aminocarbonylamino, aminothiocarbonylamino, N-monoalkylamino,
N-mono(hydroxyalkyl)amino, N,N-bis(hydroxyalkyl)amino, N-mono(alkoxyalkyl)amino,
N,N-bis(alkoxyalkyl)amino, N-mono(alkoxyalkyl)amino, N-mono(alkylthioalkyl)amino, N
bis(alkylthioalkyl)amino, N-monocycloalkylamino, N-monoaryl-amino, N,N-dialkyl-amino, N,N-dicycloalkylamino, N,N-diaryl-amino, N-alkyl-N-aryl-amino, N-cycloalkyl-N-aryl-amino, aminosulfonylamino, N-monocycloalkyl-aminosulfonylamino, N-monoalkyl-aminosulfonylamino, N,N-dicycloalkyl-aminosulfonylamino, N,N-dialkylaminosulfonylamino, N-monoaryl- aminosulfonylamino, N,N-diaryl-aminosulfonylamino, N-monocycloalkyl-N-monoarylaminosulfonyl-amino, N-monoalkyl-N-monoarylaminosulfonyl-amino and -S-G;
and wherein the substituents mentioned above can themselves be substituted by G; and
R⁷, R⁹, R¹⁹, R¹³, R¹⁴, R¹⁶ and R¹⁷ in addition can represent G; and
R¹⁹, R²⁰ and R²¹ in addition can represent hydroxyl;
R²² is oxygen, sulphur or -NR²³-;
wherein when X denotes -C-R¹³ and Z denotes -C-R¹⁶, or Y denotes -C-R¹⁴ and Z denotes -C-R¹⁶, or Y denotes -C-R¹⁴ and T denotes -C-R¹⁷, the groups R¹³ and R¹⁶ and R¹⁴ and R¹⁶ and R¹⁴ and R¹⁷, respectively, together with the carbon atoms to which they are bonded can form a 3- to 8 membered carbocyclic or heterocylic ring, which is saturated, unsaturated or partially unsaturated, and which is unsubstituted or substituted by one or more substituents selected from the group consisting of the substituents for R¹, R², R³, R⁴, R⁵, R⁷, R⁹, R¹⁰, R¹³, R¹⁴, R¹⁶, R¹⁷, R¹⁹, R²⁰ and R²¹ given above; and
wherein 3-hydroxy-4-(1H-imidazol-2-ylazo)-1-naphthalenesulfonic acid and 4-hydroxy-3-(1 H-imidazol-2-ylazo)-benzenesulfonic acid are excluded.

Alkyl groups appearing in this application may be straight-chain or branched and are preferably (C₁-C₆)-alkyl groups, for example methyl, ethyl, n-propyl, isopropyl, n-butyl, n-pentyl, isopentyl or n-hexyl. The same logic applies to alkoxy groups which accordingly are preferably (C₁-C₆)-alkoxy, for example methoxy and ethoxy, to thioalkoxy groups, which are preferably (C₁-C₆)-thioalkoxy, for example -SCH₃ or - SC₂H₅.

Cycloalkyl groups are preferably (C₃-C₈)-cycloalkyl and especially preferably cyclopentyl and cyclohexyl. The term cyloalkyl comprises for the purpose of the present application unsatured cycloalkyl groups as well. A preferred group of this type is cyclopentenyl.
Alkenyl groups may be straight-chain or branched and are preferably (C₂-C₆)-alkenyl groups for example vinyl, allyl, ethynyl and propargyl.
Aryl groups appearing in this application are preferably phenyl or naphthyl. The terms phenyl and naphthyl comprises unsubstituted phenyl and naphthyl as well as phenyl and naphthyl, which carry substituents. Preferred substituents are alkyl, heterocycloalkyl, hydroxyalkyl, halogen, hydroxyl, alkoxy, alkylthio, acyl, nitro, amino, monoalkylamino, dialkylamino, mono(hydroxyalkyl)amino, bis (hydroxyalkyl)amino, monoalkyl-mono(hydroxyalkyl)amino, carbamoyl, sulfamoyl, acylamino, aminocarbonylamino, aminosulfonylamino, alkoxycarbonyl and acyloxy.
Heteroaryl groups appearing in this application are preferably pyridine, pyrimidine, pyridazine, pyrazine, pyrrole, imidazole, pyrazole, 1,2,4-thiadiazole, 1,2,4-triazole, tetrazole, thiophene, thiazole, isothiazole, 1,3,4-thiadiazole, furane, oxazole or isoxazole.

Heterocycloalkyl groups are preferably pyrrolidine, piperidine, morpholine or piperazine. Halogen is preferably chlorine, bromine or fluorine. A cosmetically acceptable, water solubilizing inorganic or organic cation is for example a monovalent cation like sodium, potassium, lithium, ammonium or tetraalkylammonium or bi- or polyvalent cation, in which case M represent 1/v cation whereby v represents the valency of the cation. Examples of such cations are magnesium, calcium and aluminium.
Preferred meanings of M are sodium, potassium, lithium and ammonium.

In preferred dyestuffs of the formula (I) n is an integer from 1 to 3 and/or m is an integer from 0 to 2.

Further preferred dyestuffs of the formula (I) correspond to formulae (Ia) to (Im) 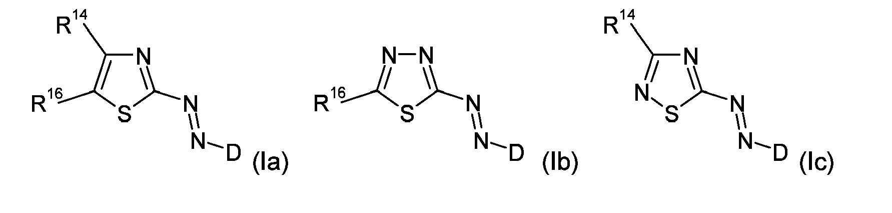 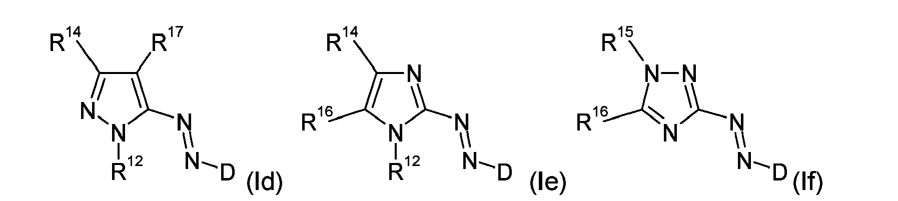 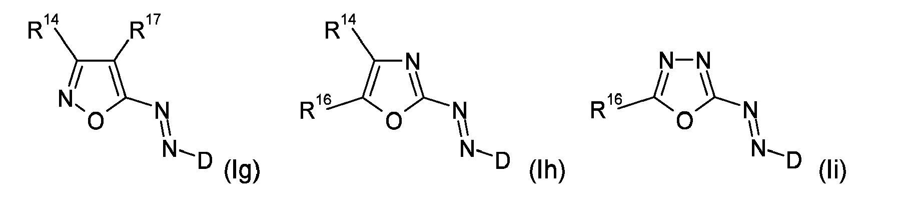 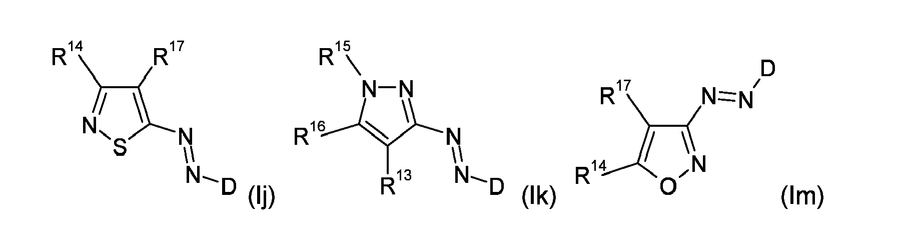 wherein R¹² to R¹⁷ are defined as given above.

Especially preferred dyestuffs of the formula (I) correspond to one of the formulae (Ia) to (Im) wherein
R¹² and R¹⁵, independently, are hydrogen or (C₁-C₄)-alkyl;
R¹³, R¹⁴, R¹⁶ and R¹⁷, independently, are hydrogen, (C₁-C₄)-alkyl, (C₁-C₄)-alkyl substituted by (C₁-C₄)-alkoxycarbonyl, trifluoromethyl, formyl, hydroxyiminoformyl, (C₁-C₄)-alkylcarbonyl, carboxy, aminocarbonyl, (C₁-C₄)-alkoxycarbonyl, chlorine,
(C₁-C₄)-thioalkoxy, (C₁-C₄)-thioalkoxy substituted by hydroxy, amino, mono-(C₁-C₄)-alkyl-amino, mono-(C₁-C₄)-alkyl-amino substituted by hydroxy or sulfo, di-(C₁-C₄)-alkylamino, di-(C₁-C₄)-alkyl-amino wherein one or both alkyl groups are substituted by hydroxy or sulfo, N-(C₁-C₄)-alkylcarbonyl-amino, N-(C₁-C₄)-alkyl-N-(C₁-C₄)-alkylcarbonyl-amino, (C₁-C₄)-alkyl-sulfonyl-amino, di-(C₁-C₄)-alkyl-amino-sulfonyl-amino, (C₁-C₄)-alkyl-sulfonyl, (C₁-C₄)-alkyl-sulfonyl substituted by sulfo, aminosulfonyl, di-(C₁-C₄)-alkyl-aminosulfonyl, aminocarbonylamino, aminothiocarbonylamino, di-(C₁-C₄)-alkyl-aminocarbonylamino, di-(C₁-C₄)-alkyl-aminothiocarbonylamino, di-(C₁-C₄)-alkyl-aminocarbonyl-(C₁-C₄)-alkylamino, di-(C₁-C₄)-alkyl-aminothiocarbonyl-(C₁-C₄)-alkyl-amino,
or wherein R¹³ and R¹⁶ or R¹⁴ and R¹⁶ or R¹⁴ and R¹⁷ together with the carbon atoms to which they are bonded form a phenyl ring which is unsubstituted or substituted by a substituent selected from the group consisting of (C₁-C₄)-alkyl, (C₁-C₄)-alkoxy, nitro, aminosulfonyl, (C₁-C₄)-alkyl-sulfonyl, (C₁-C₄)-alkyl-sulfonyl substituted by hydroxy and sulfo.

In further preferred dyestuffs of the formula (I) D corresponds to the formula (D¹)
wherein
A¹ to A⁴ are defined as given above, wherein, however, only one or two of which can denote nitrogen, -N(R¹⁸)-, or oxygen and the remainders are -C(R¹⁹)=,
-C(R²⁰)(R²¹)- or -C(=R²²)-;
G is defined as given above;
R¹, R¹⁹, R²⁰ and R²², independently, are hydrogen, halogen, nitro, formyl, (C₁-C₄)-alkyl, (C₁-C₄)-alkoxy, (C₂-C₄)-acyl, (C₂-C₄)-thioacyl, (C₁-C₄)-alkoxycarbonyl, (C₂-C₄)-acylamino, N-(C₁-C₄)-alkyl-(C₂-C₄)-acylamino, (C₁-C₄)-alkylsulfonylamino, N-(C₁-C₄)-alkyl-alkylsulfonylamino, benzoylamino, N-(C₁-C₄)-alkyl-benzoylamino, carbamoyl, N-mono(C₁-C₄)-alkyl-carbamoyl, N,N-di-(C₁-C₄)-alkylcarbamoyl, amino, mono-(C₁-C₄)-alkylamino, di-(C₁-C₄)-alkylamino, aminocarbonylamino, N,N-di-(C₁-C₄)-alkylamino-carbonylamino, N,N-di-(C₁-C₄)-alkylaminocarbonyl-(N-(C₁-C₄)-alkyl)amino, aminothiocarbonylamino, N,N-di-(C₁-C₄)-alkylaminothiocarbonyl-amino, N,N-di-(C₁-C₄)-alkylaminothiocarbonyl-(N-(C₁-C₄)-alkyl)amino, sulfamoyl group, N-mono-(C₁-C₄)-alkylsulfamoyl, N,N-di-(C₁-C₄)-alkylsulfamoyl, aminosulfonylamino, N,N-di-(C₁-C₄)-alkylaminosulfonylamino, sulphur substituted by -SO₃M, (C₁-C₄)-alkylthio, (C₁-C₄)-alkylsulfonyl, iminomethyl, N-(C₁-C₄)-alkyliminomethyl, hydroxyiminomethyl, and wherein the (C₁-C₄)-alkyl groups mentioned above can be substituted by one or more substituents selected from the group consisting of hydroxyl, (C₁-C₄)-alkoxy, thio-(C₁-C₄)-alkoxy, poly(oxyalkylene), -SO₃M, halogen, cyano, nitro, amino, N- (C₁-C₄)-monoalkyl-amino, N,N-di-(C₁-C₄)-alkyl-amino, (C₁-C₄)-alkoxy-(C₁-C₄)-alkylamino, thio-(C₁-C₄)-alkoxy-(C₁-C₄)-alkyl-amino, amino-(C₁-C₄)-alkyloxy, N-mono-(C₁-C₄)-alkylamino-(C₁-C₄)-alkyloxy, N,N-di-(C₁-C₄)-alkyl-amino-(C₁-C₄)-alkoxy, amino-(C₁-C₄)-alkylthioxy, N-mono-(C₁-C₄)-alkylamino-(C₁-C₄)-alkylthioxy and N,N-di-(C₁-C₄)-alkylamino-(C₁-C₄)-alkylthioxy; and
R¹⁹, R²⁰ and R²², independently, can in addition denote hydroxyl or -SO₃M;
R¹⁸ is hydrogen or (C₁-C₄)-alkyl;
R²² is oxygen;
q is an integer from 1 to 2;
n is an integer from 0 to 2;
p is an integer from 0 to 1; and o is 1.

In especially preferred dyestuffs of the formula (I) D corresponds to a group of the formula (D¹a) to (D¹w) 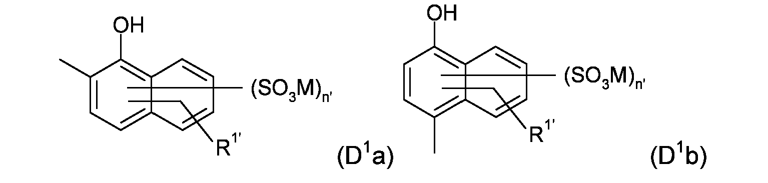 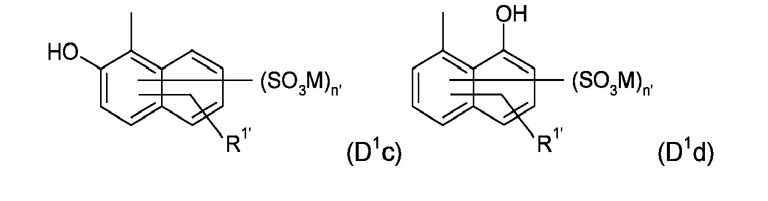 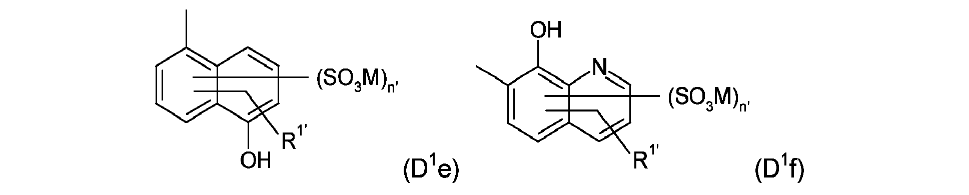 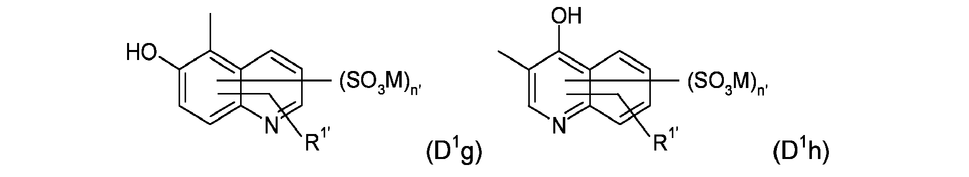 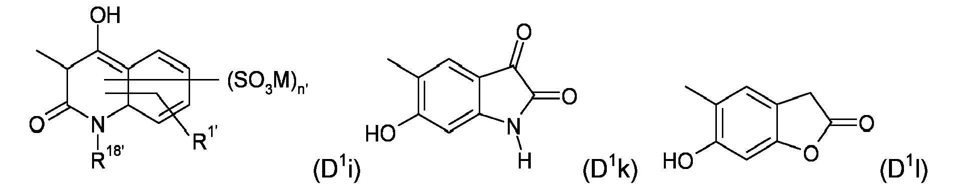 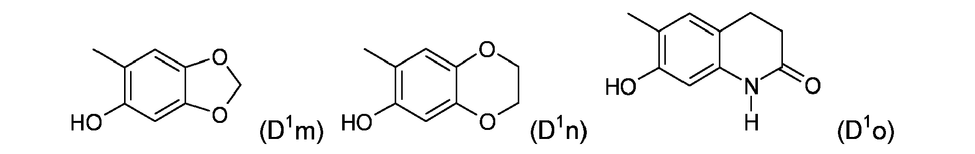 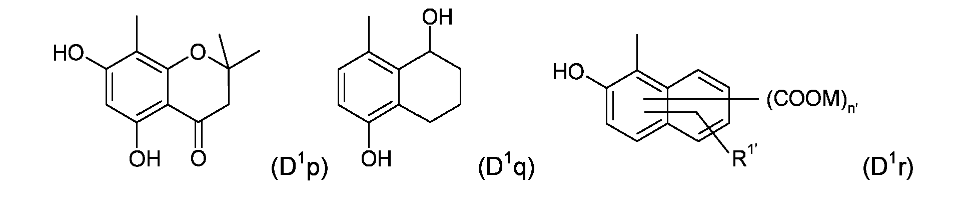 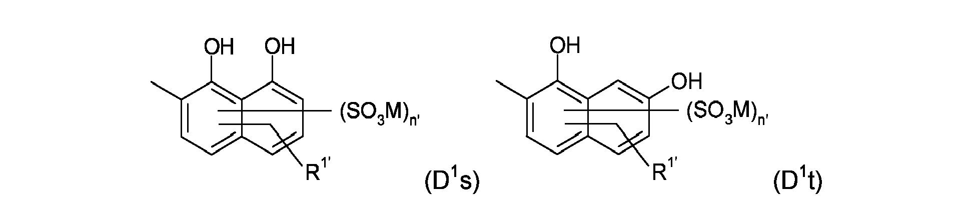 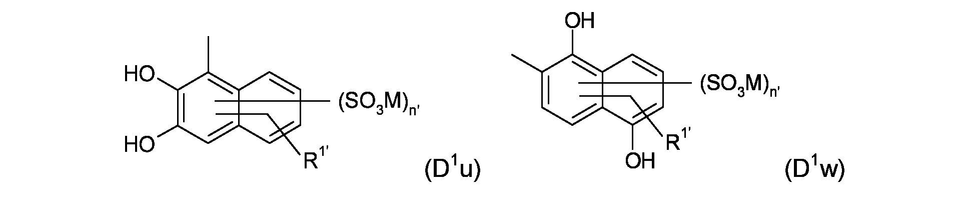 wherein
R^{1'} is hydrogen, (C₁-C₄)-alkoxy, amino, (C₁-C₄)-alkylcarbonyl-amino, benzoylamino, hydroxy, (C₁-C₄)-alkylamino or chlorine;
R^{18'} is hydrogen or (C₁-C₄)-alkyl;
M is defined as given above; and n' is an integer from 0 to 2.

Examples of dyestuffs of this type are the dyestuffs of the formulae (11) to (1176) and (1388) to (1395) 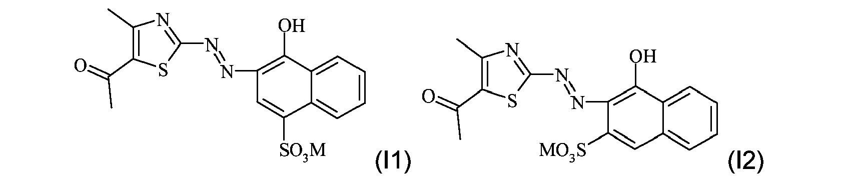 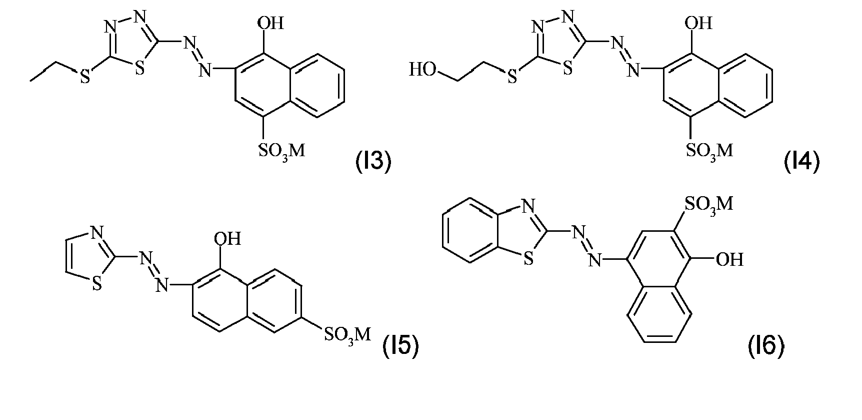 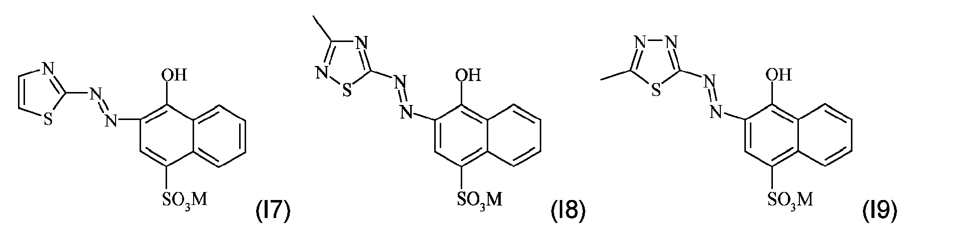 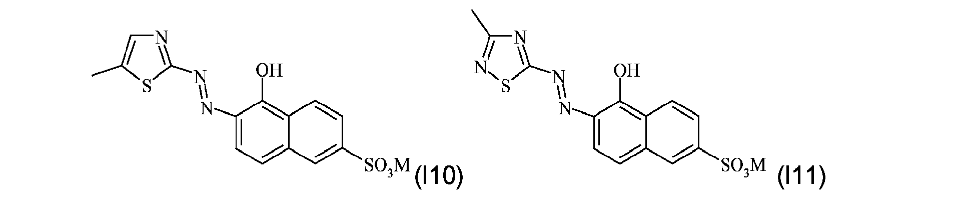 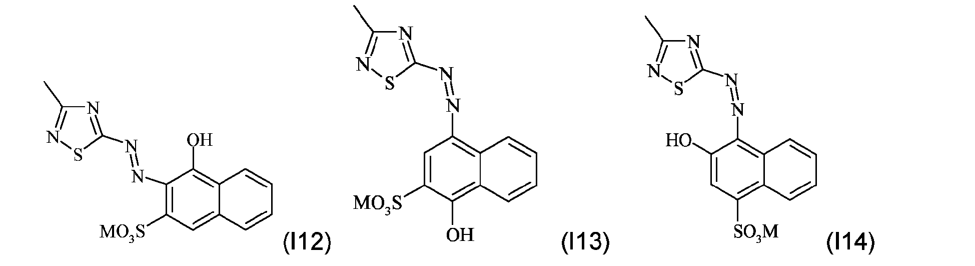 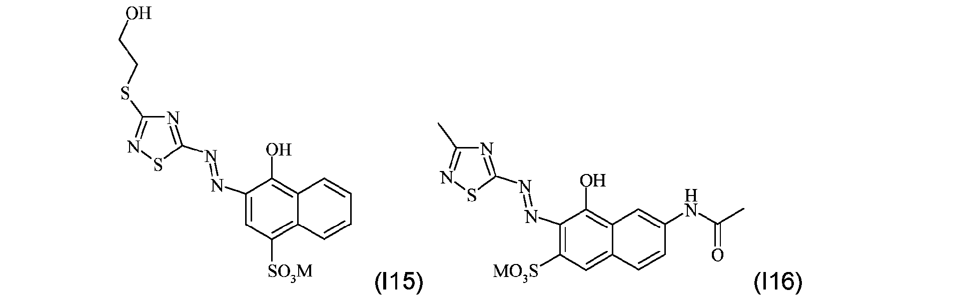 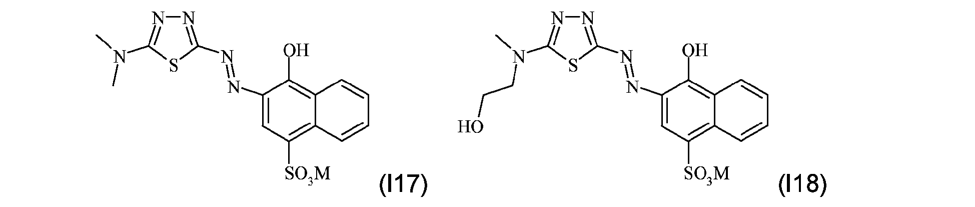 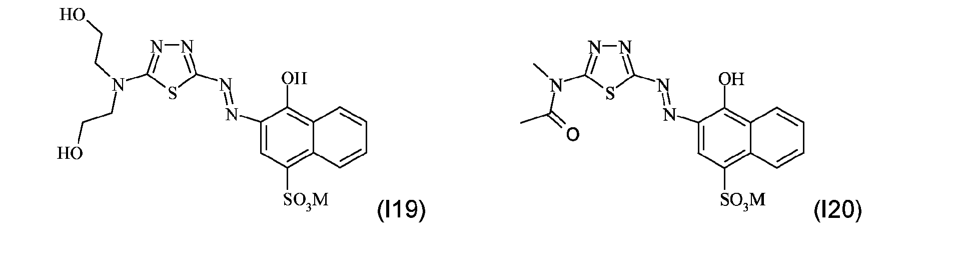 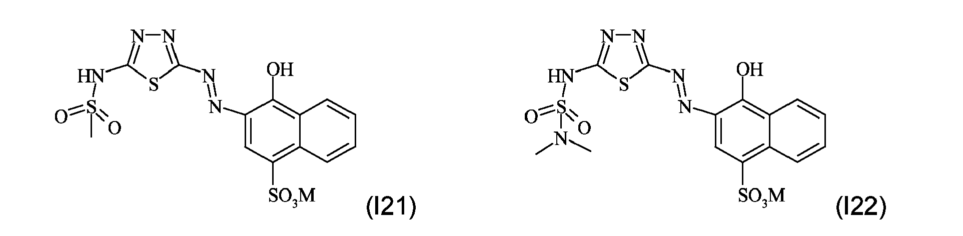 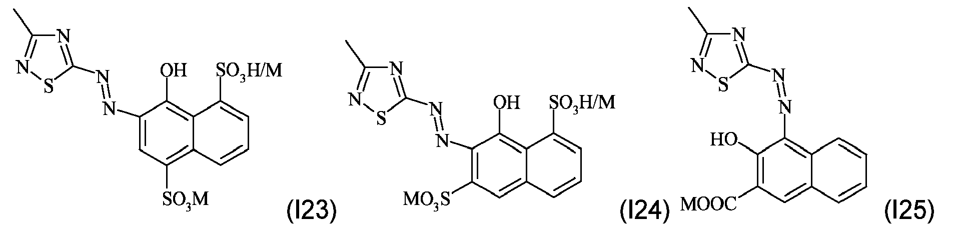 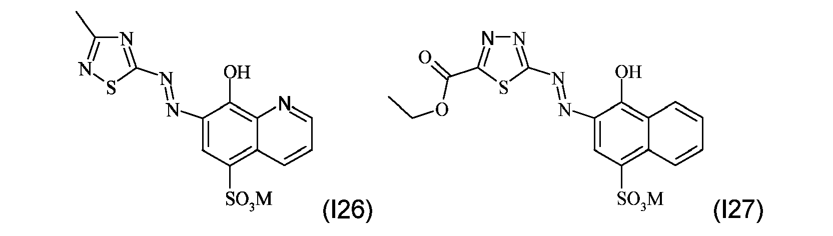 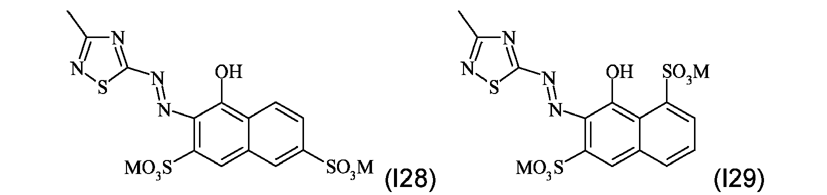 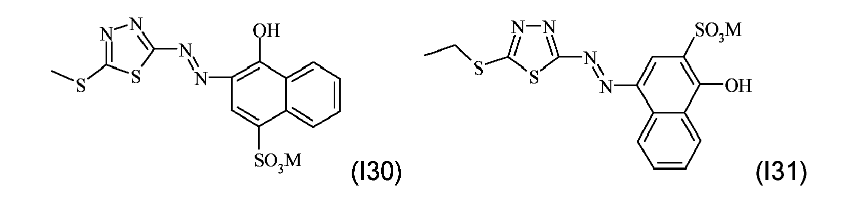 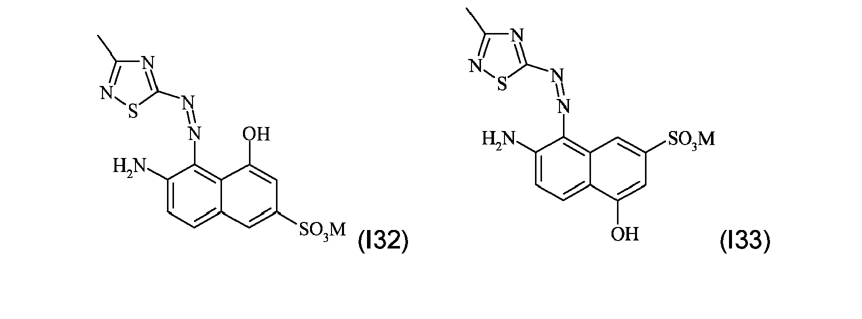 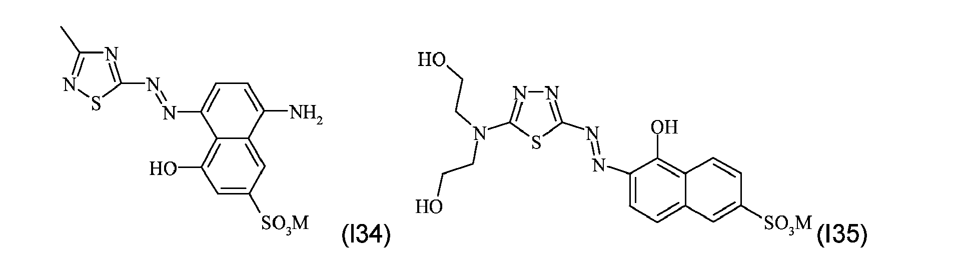 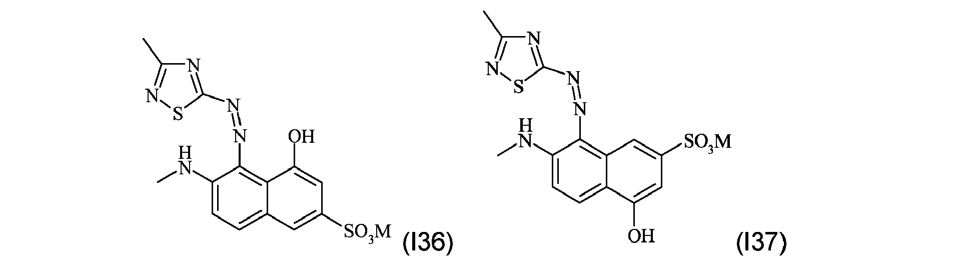 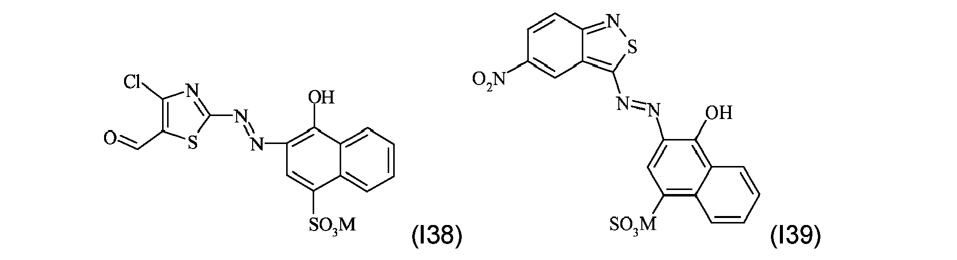 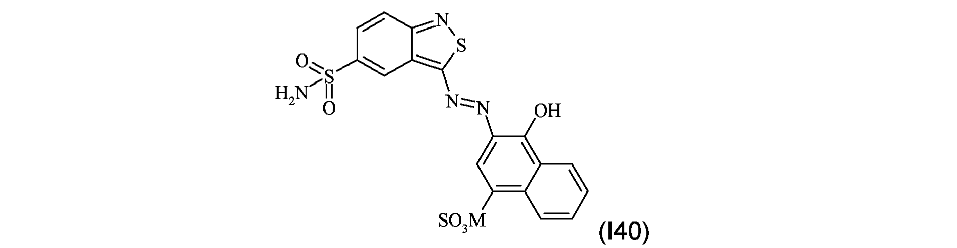 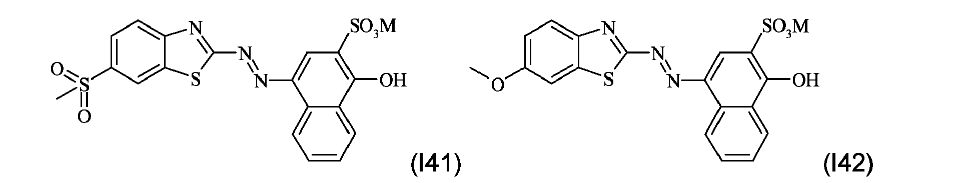 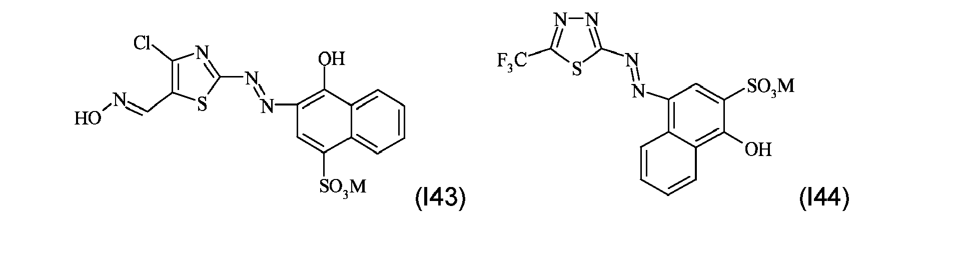 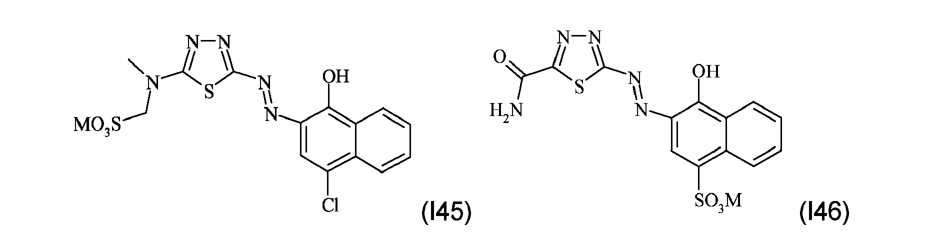 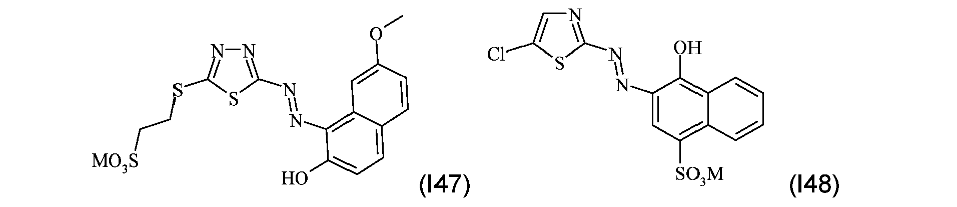 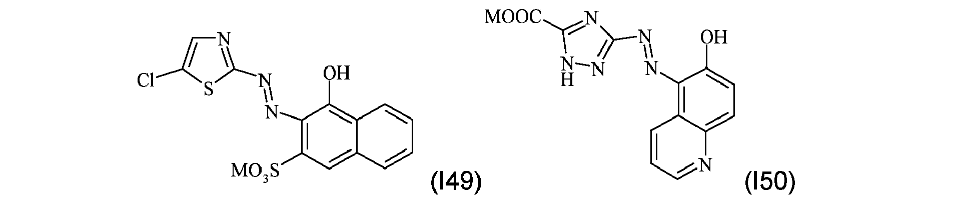 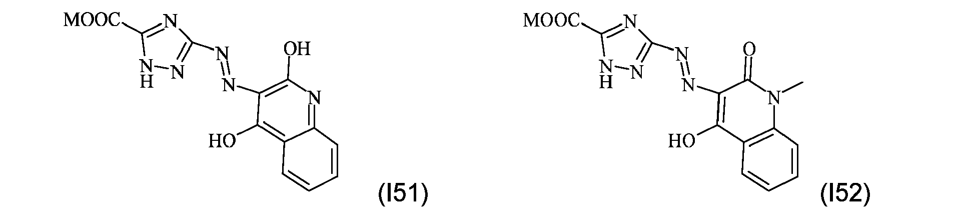 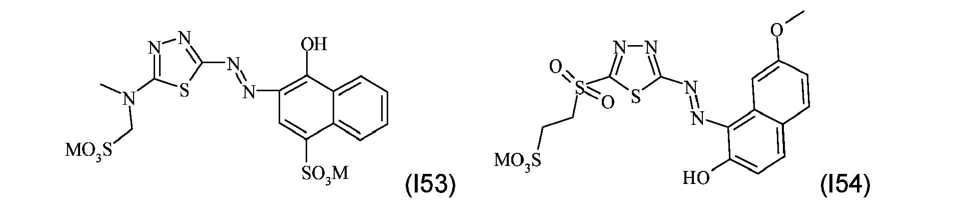 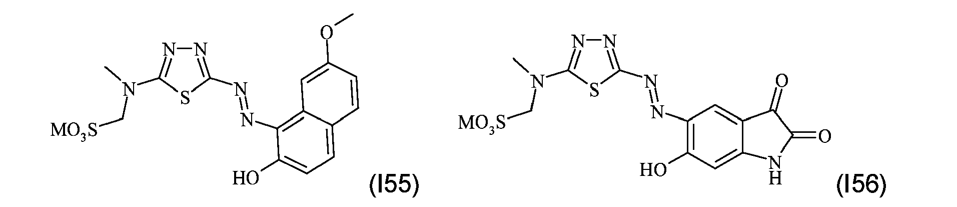 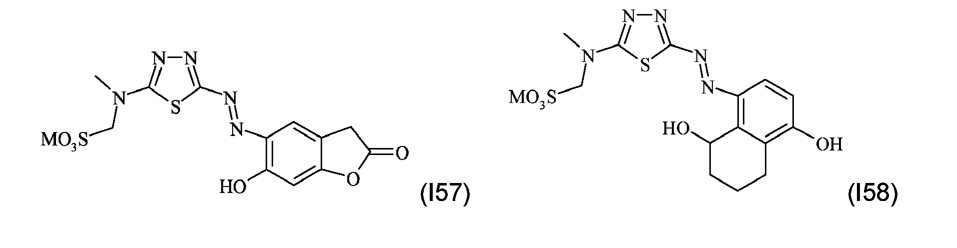 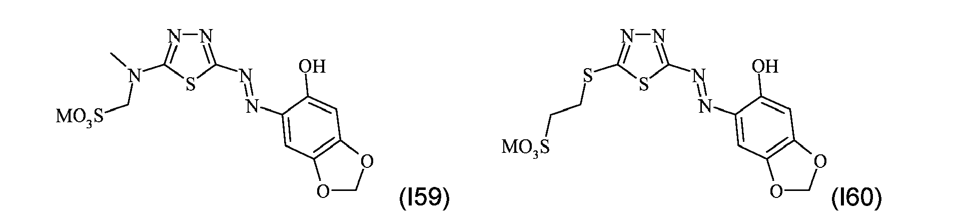 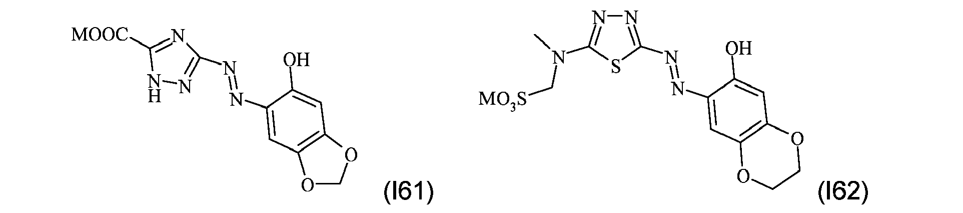 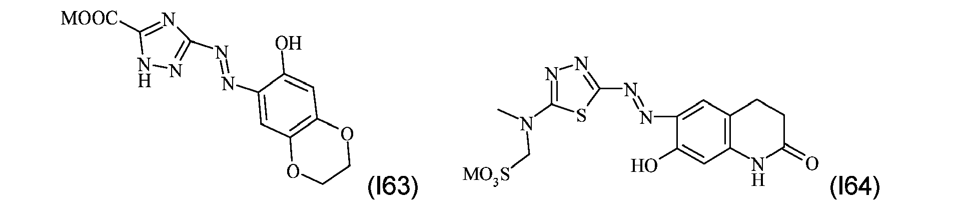 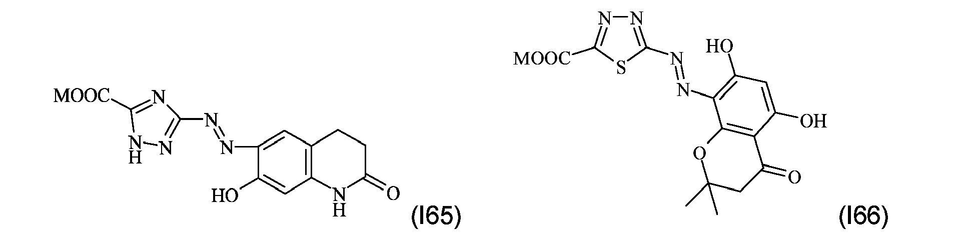 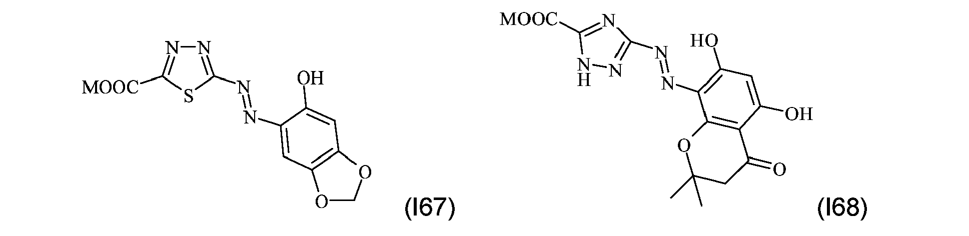 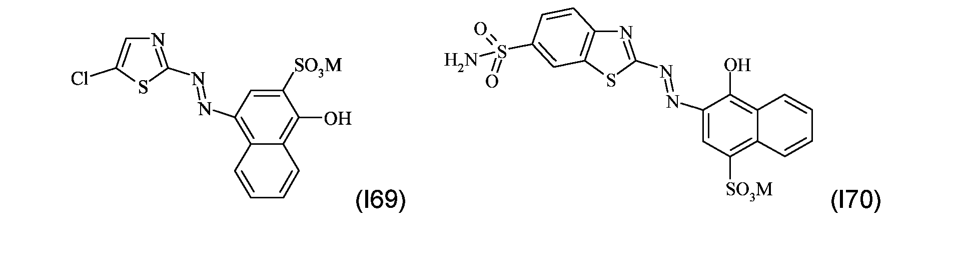 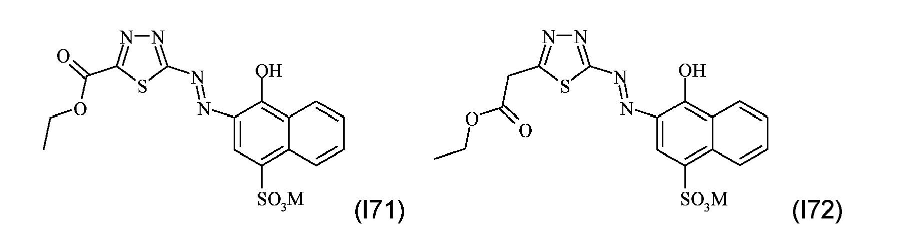 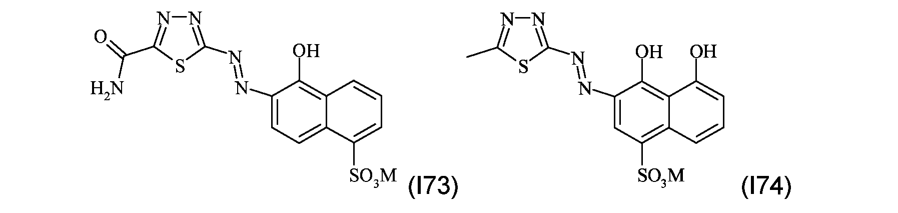 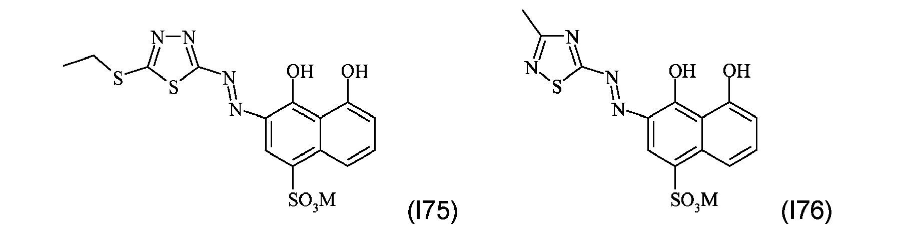 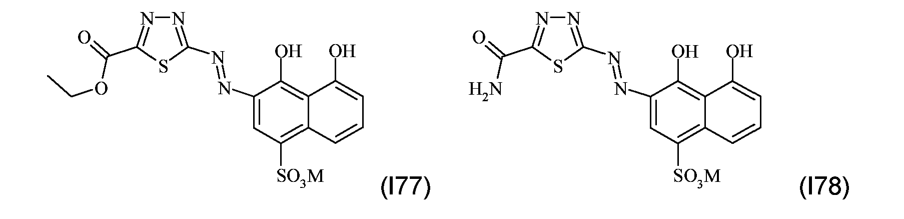 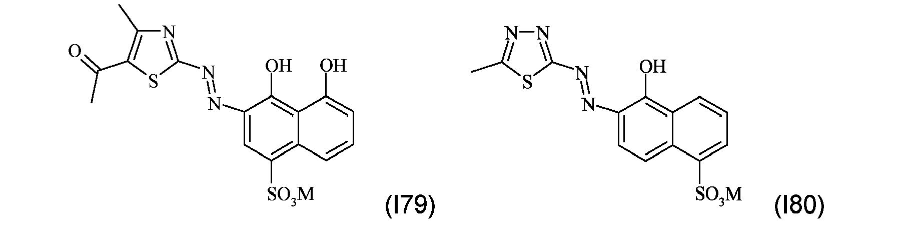 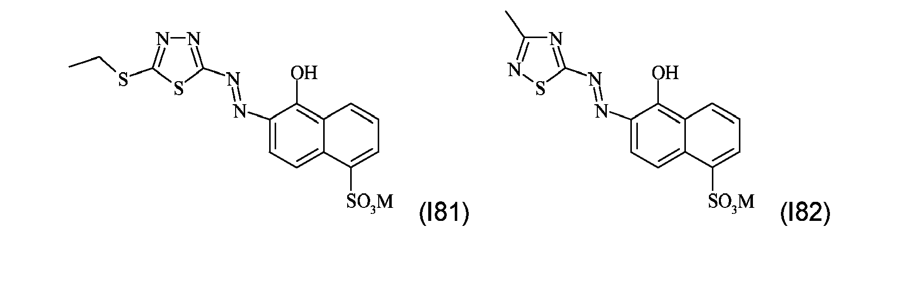 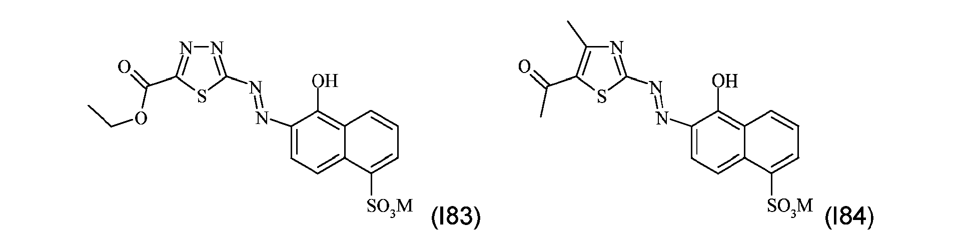 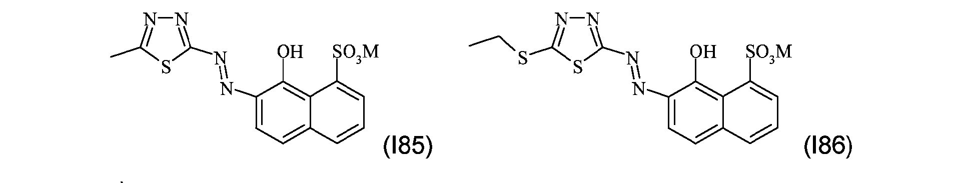 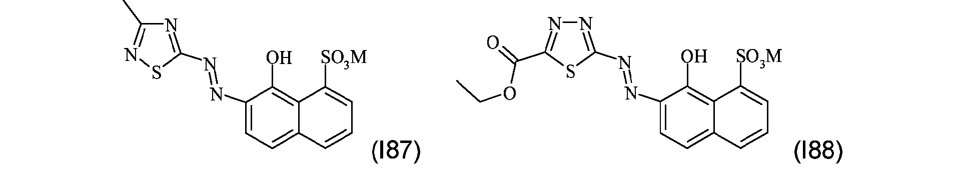 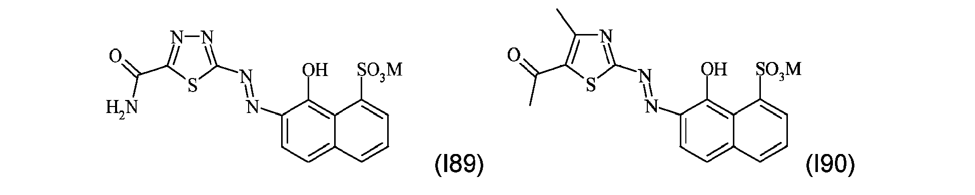 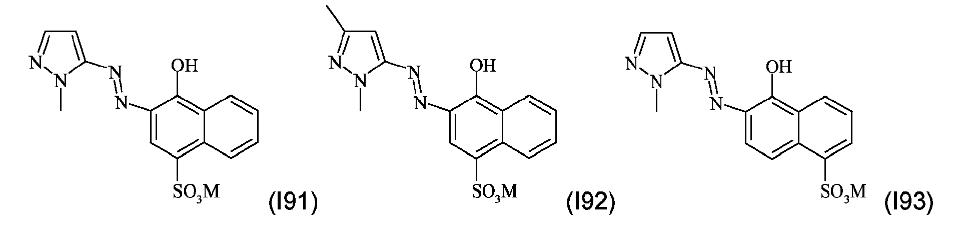 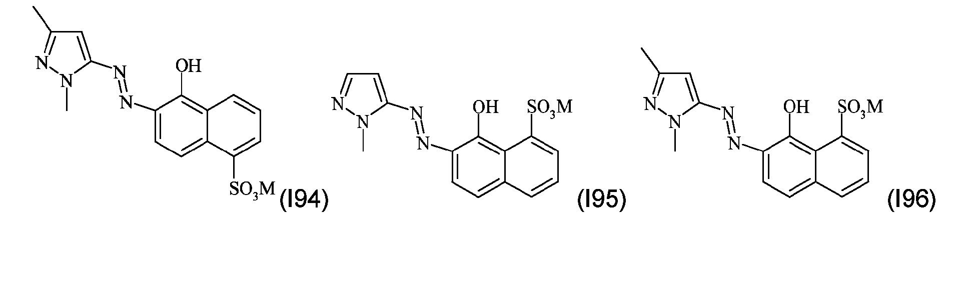 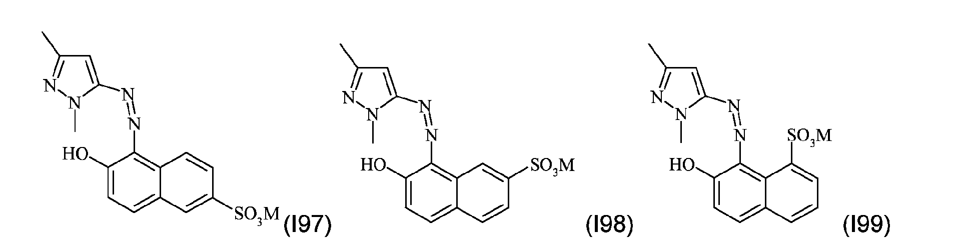 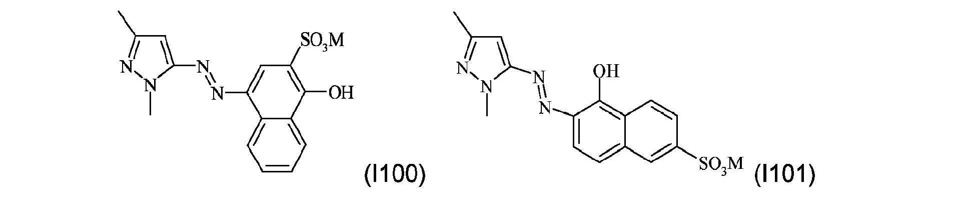 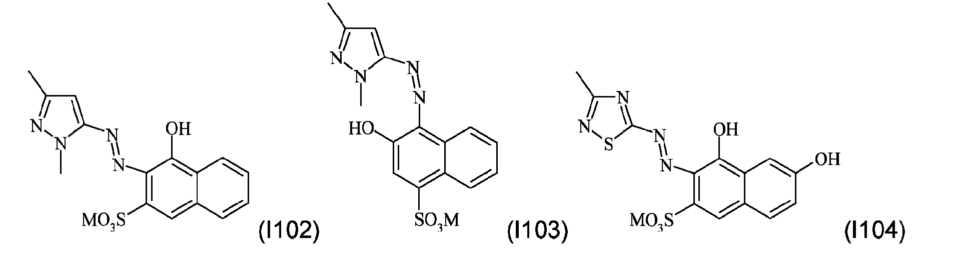 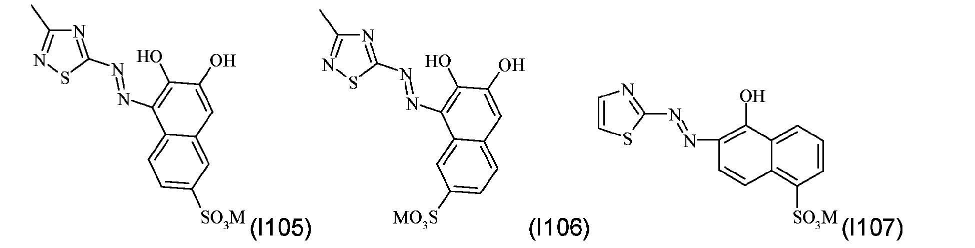 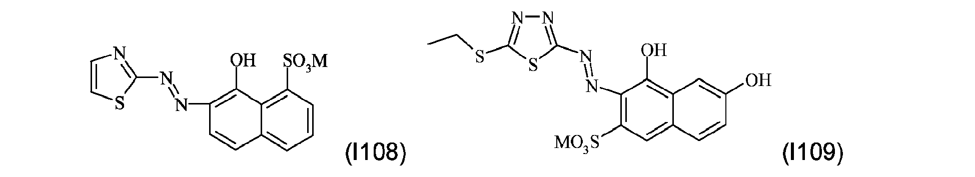 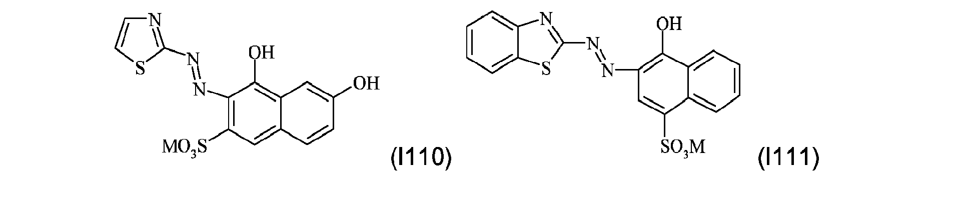 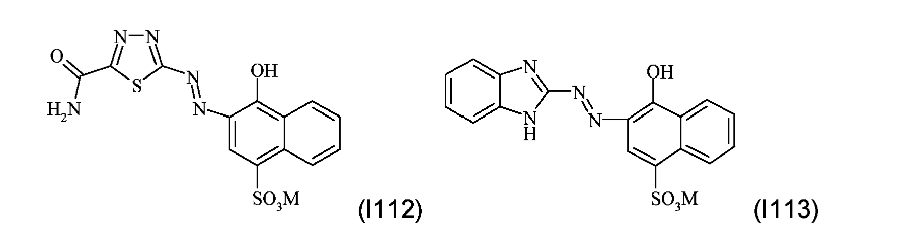 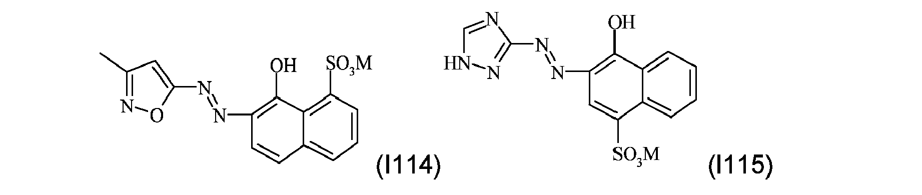 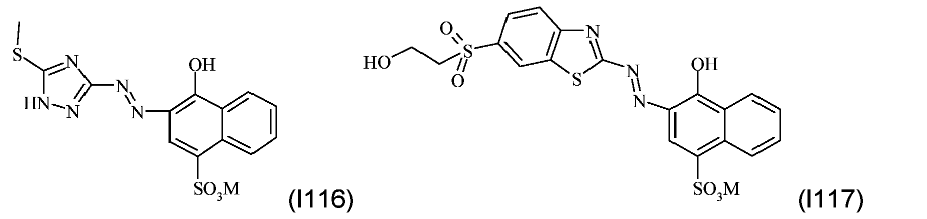 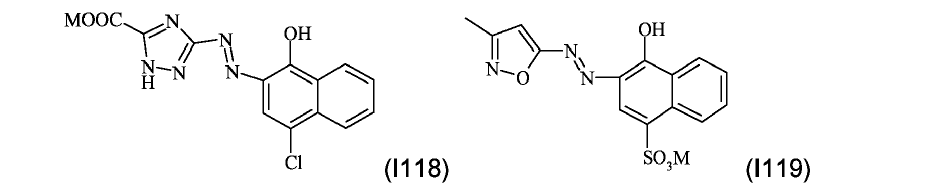 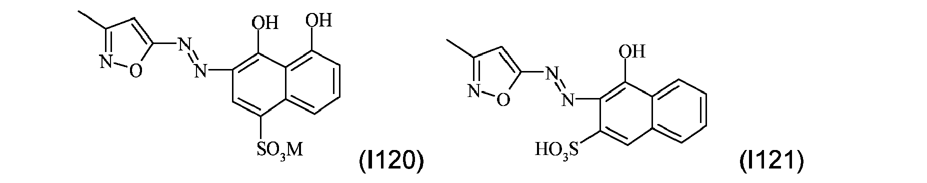 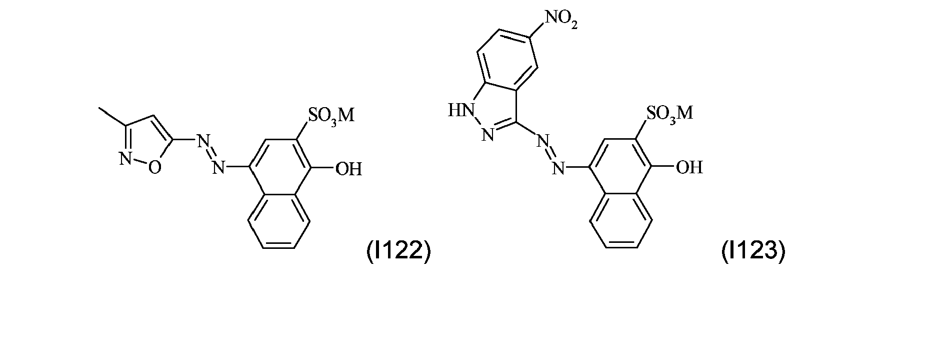 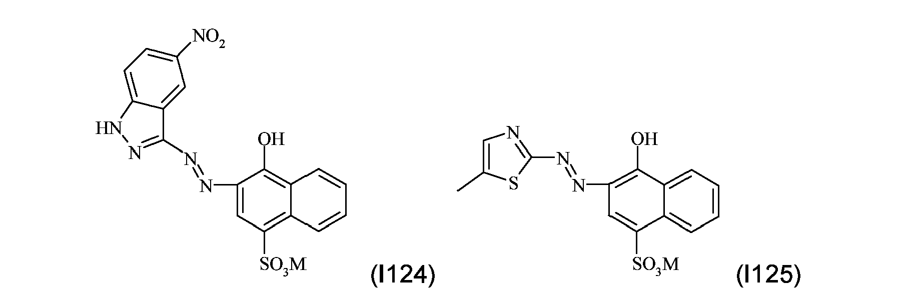 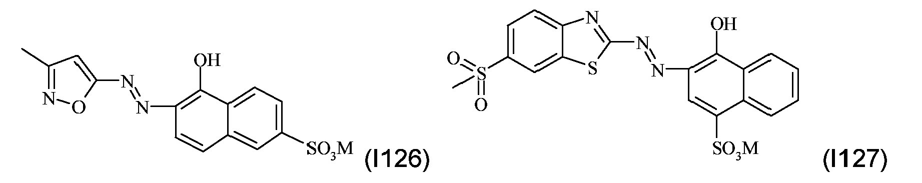 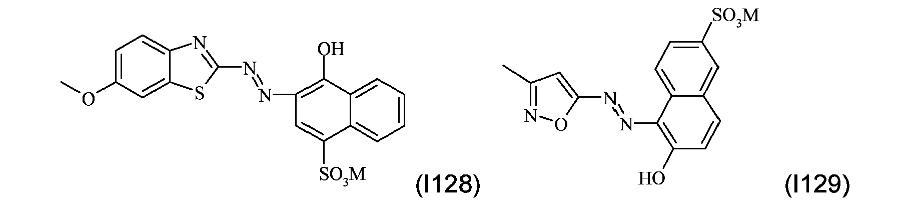 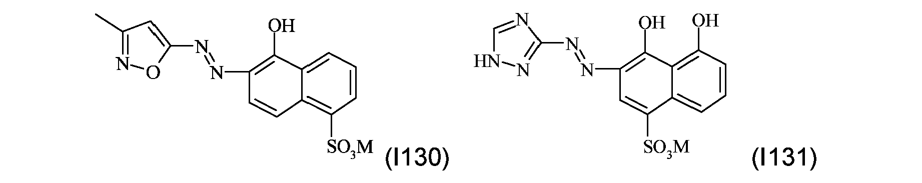 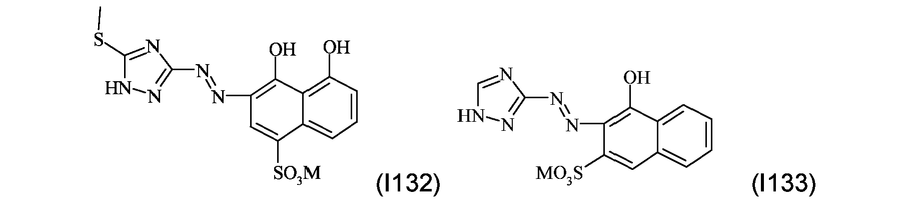 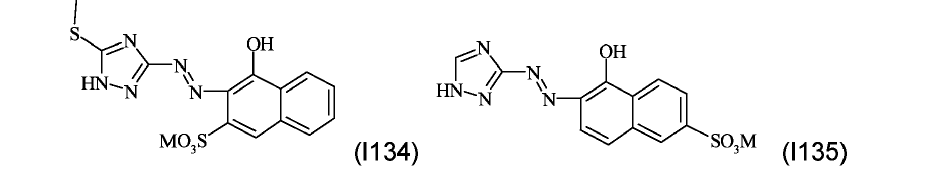 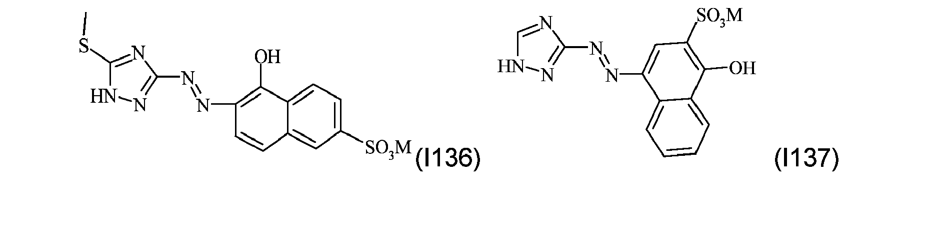 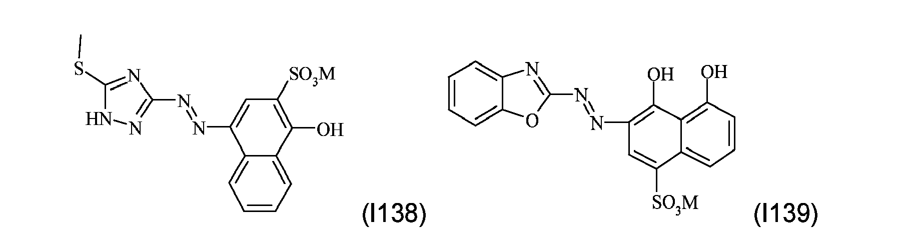 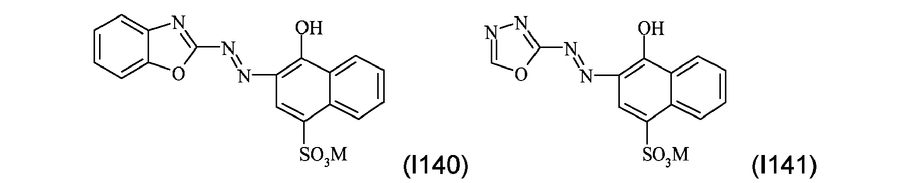 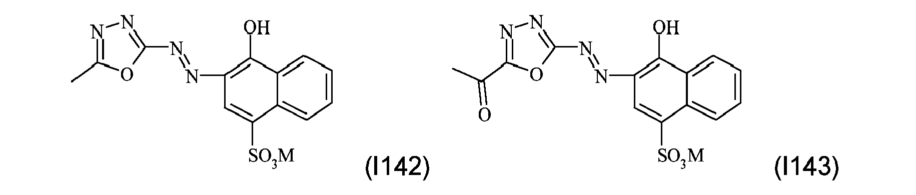 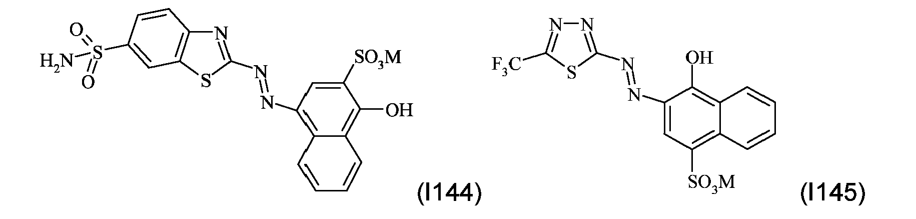 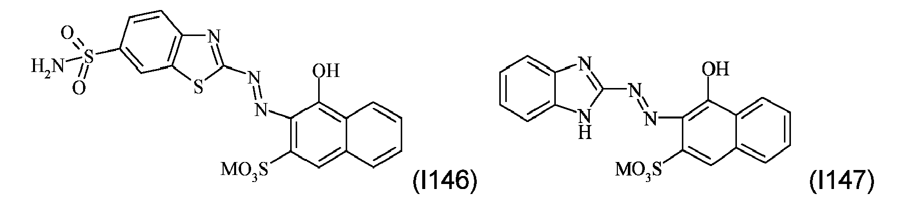 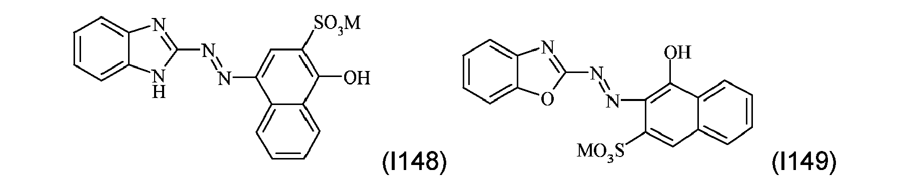 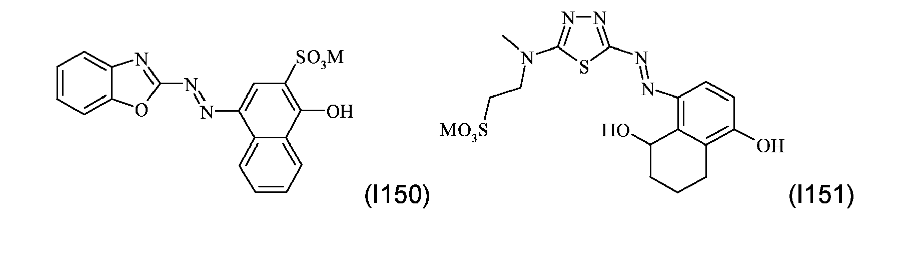 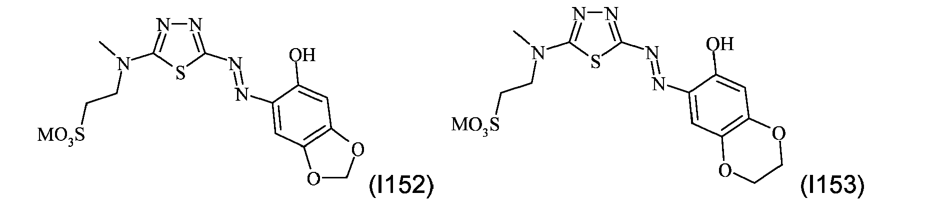 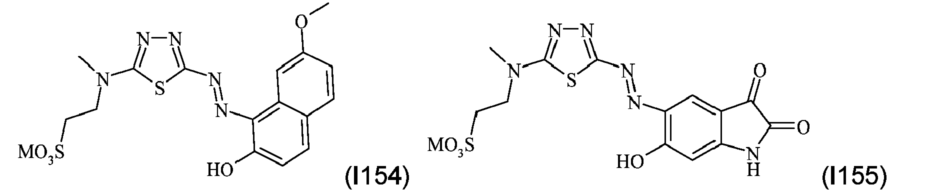 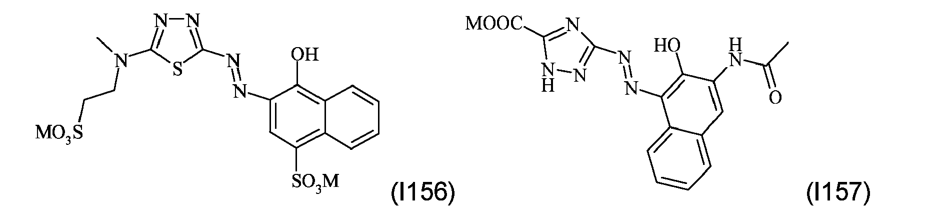 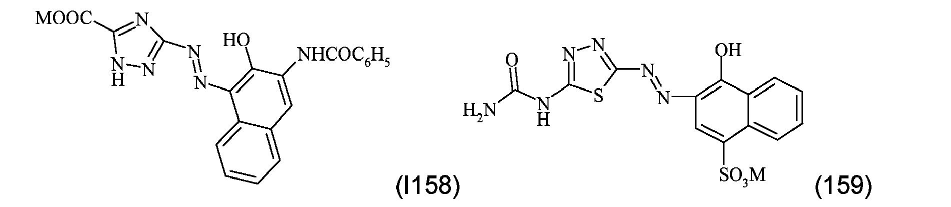 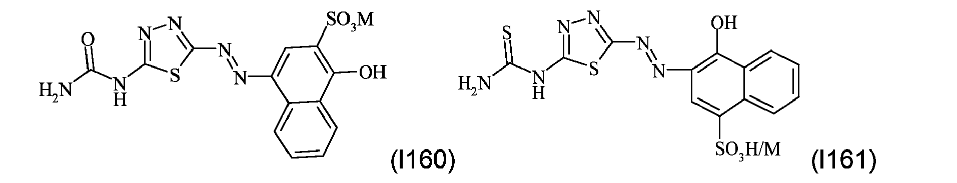 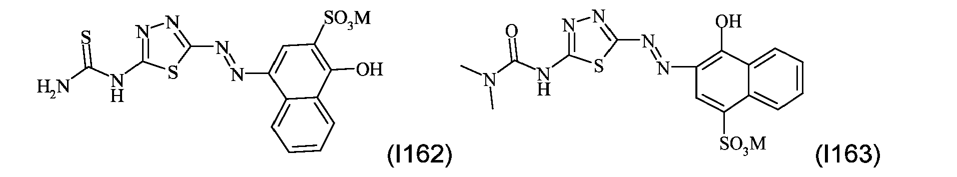 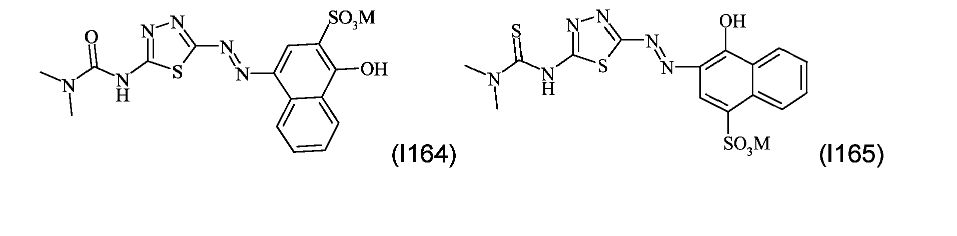 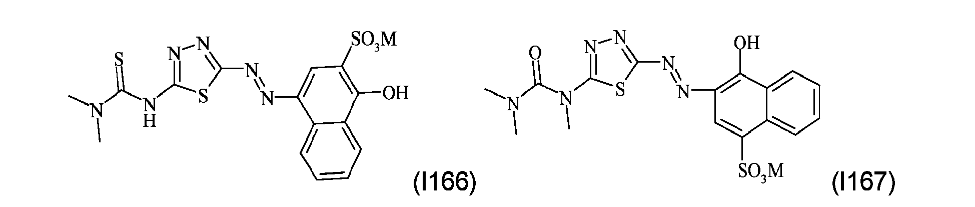 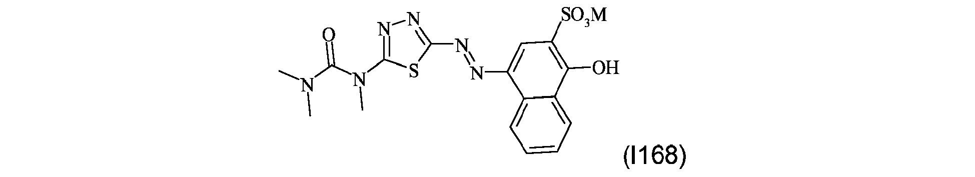 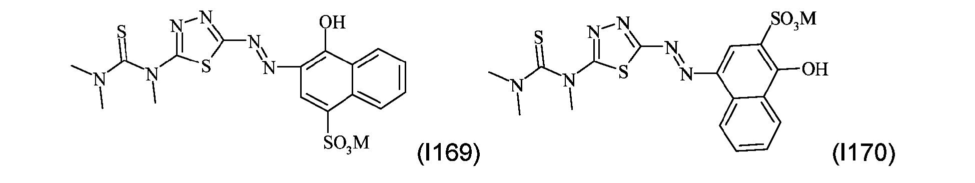 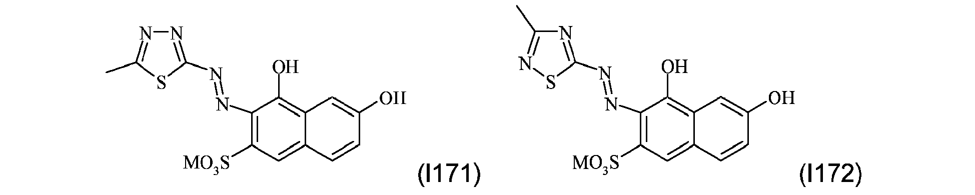 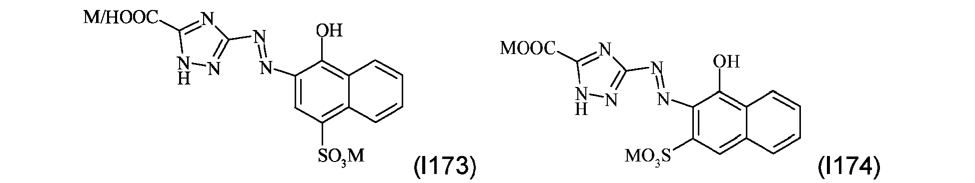 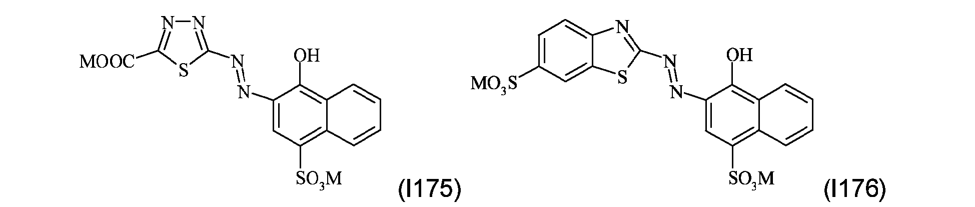 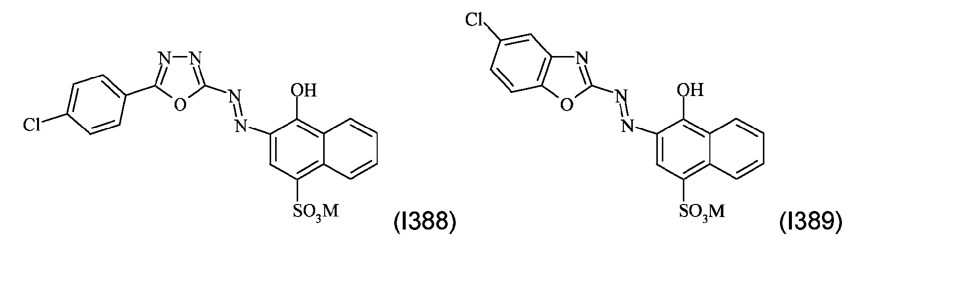 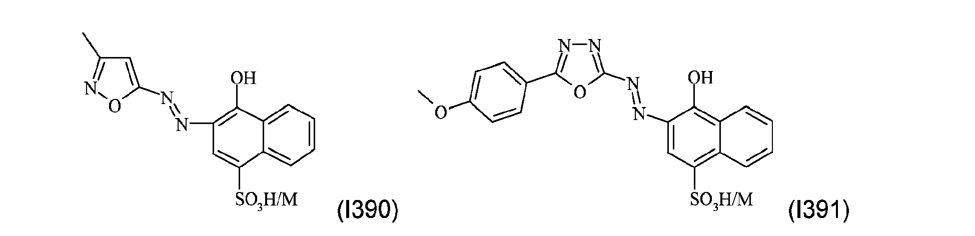 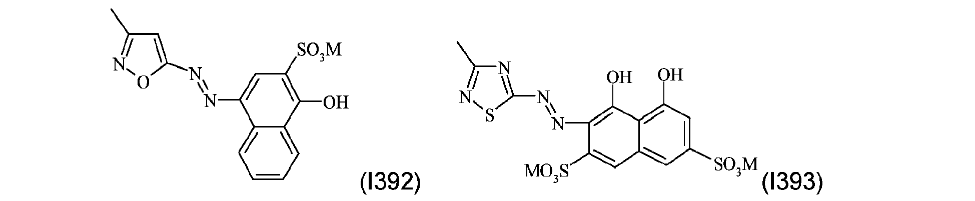 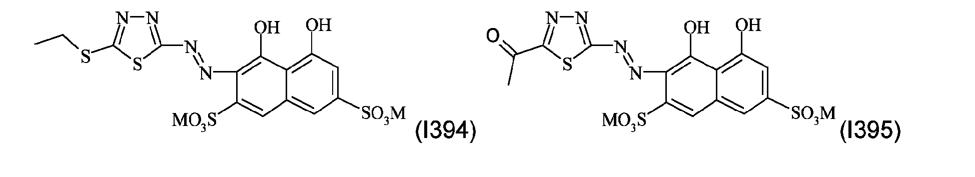 wherein M is hydrogen, sodium, potassium, lithium or ammonium.

In still further preferred dyestuffs of the formula (I) D corresponds to the formula (D²)
wherein
G is defined as given above;
n is an integer from 0 to 1;
m is an integer from 0 to 2;
r is an integer from 0 to 3;
R⁵ is hydrogen, halogen, cyano, nitro, formyl, (C₁-C₄)-alkyl; (C₁-C₄)-alkoxy, (C₂-C₄)-acyl, (C₁-C₄)-alkoxycarbonyl, (C₂-C₄)-acyloxy, (C₂-C₄)-acylamino, N-(C₁-C₄)-alkyl-(C₂-C₄)-acylamino, (C₁-C₄)-alkylsulfonylamino, N-(C₁-C₄)-alkyl-(C₁-C₄)-alkylsulfonylamino, carbamoyl, N-mono-(C₁-C₄)-alkylcarbamoyl, N,N-di-(C₁-C₄)-alkylcarbamoyl, amino, mono-(C₁-C₄)-alkyl-amino, mono-(C₃-C₈)-cycloalkyl-amino, monoarylamino, diarylamino, mono-(C₁-C₄)-alkylmono-arylamino, mono-(C₃-C₈)-cycloalkylmono-arylamino, aminothiocarbonylamino, aminocarbonylamino, N,N-di-(C₁-C₄)-alkylaminothiocarbonylamino, N,N-di-(C₁-C₄)-alkylaminocarbonylamino, N,N,N-tri-(C₁-C₄)-alkylaminothiocarbonylamino, N,N,N-tri-(C₁-C₄)-alkylaminocarbonylamino, sulfamoyl, N-mono-(C₁-C₄)-alkyl-sulfamoyl, N,N-di-(C₁-C₄)-alkylsulfamoyl, aminosulfonylamino, N,N-di-(C₁-C₄)-alkylaminosulfonylamino, sulphur substituted by - SO₃M, (C₁-C₄)-alkylthio, (C₁-C₄)-alkylsulfonyl, iminomethyl, N-(C₁-C₄)-alkylimino-methyl, hydroxyiminomethyl; and wherein the (C₁-C₄)-alkyl groups mentioned above can be substituted by one or more substituents selected from the group consisting of hydroxyl, (C₁-C₄)-alkoxy, thio-(C₁-C₄)-alkoxy, poly(oxyalkylene), -SO₃M, halogen, cyano, nitro, (C₁-C₄)-alkoxythiocarbonyl, (C₁-C₄)-alkoxycarbonyl, (C₁-C₄)-acyloxy, carbamoyl, sulfamoyl, amino, N-(C₂-C₄)-acylamino, N-(C₁-C₄)-alkylsulfonylamino, aminothiocarbonylamino, aminocarbonylamino, N-mono-(C₁-C₄)-alkyl-amino, N,N-di-(C₁-C₄)-alkyl-amino, (C₁-C₄)-alkoxy-(C₁-C₄)-alkylamino, thio-(C₁-C₄)-alkoxy-(C₁-C₄)-alkyl-amino, amino-(C₁-C₄)-alkoxy, N-mono-(C₁-C₄)-alkylamino-(C₁-C₄)-alkoxy, N,N-di-(C₁-C₄)-alkyl-amino-(C₁-C₄)-alkoxy, amino-(C₁-C₄)-alkylthioxy, N-mono-(C₁-C₄)-alkylamino-(C₁-C₄)-alkylthioxy and N,N-di-(C₁-C₄)-alkylamino-(C₁-C₄)-alkylthioxy.

In especially preferred dyestuffs of the formula (I) D corresponds to a group of the formula (D²a) to (D¹c) 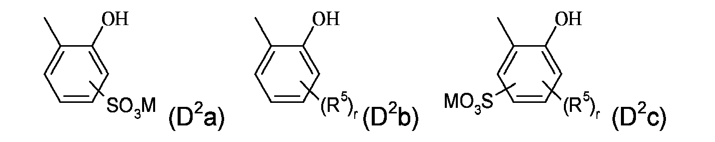 wherein
R⁵ is cyano, -COOM, (C₁-C₄)-alkyl, heteroaryl, heterocycloalkyl, (C₁-C₄)-alkoxy, N,N-di-(C₁-C₄)-alkyl-amino or N,N-di-(C₁-C₄)-alkyl-amino in which one or both alkyl groups are substituted by hydroxy, cyano, (C₁-C₄)-alkoxycarbonyl, aminocarbonyl, (C₁-C₄)-alkylcarbonyloxy, -COOM, -SO₃M, carboxyl or sulfo.
r is an intteger form 1 to 3; and
M is defined as given above.

Examples of dyestuffs of this type are the dyestuffs of the formulae (1177) to (1260) and (1396) to (1403). 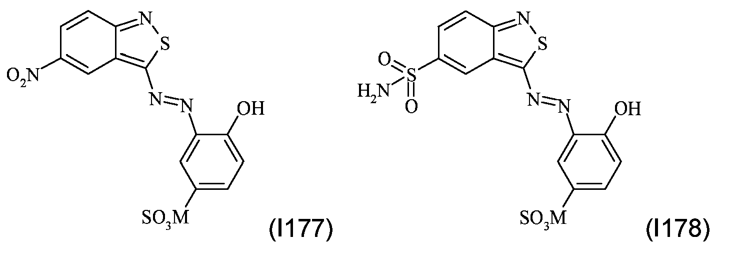 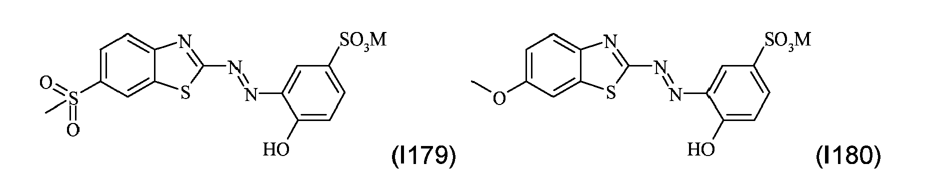 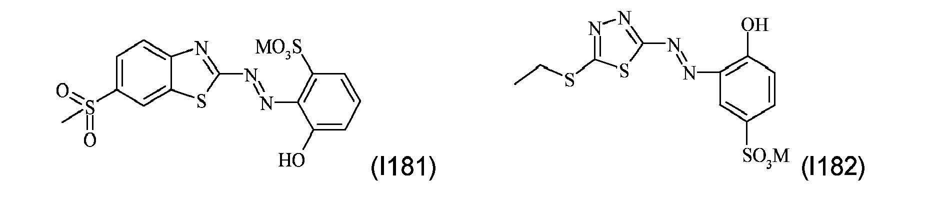 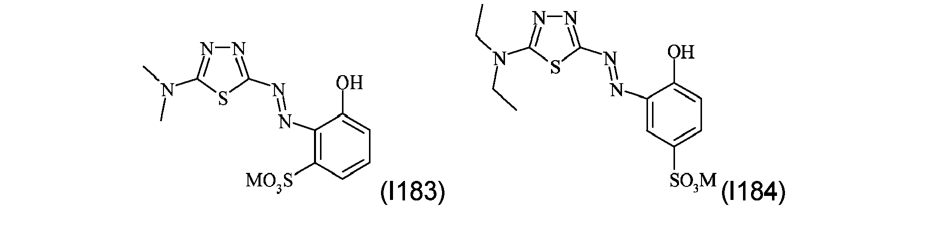 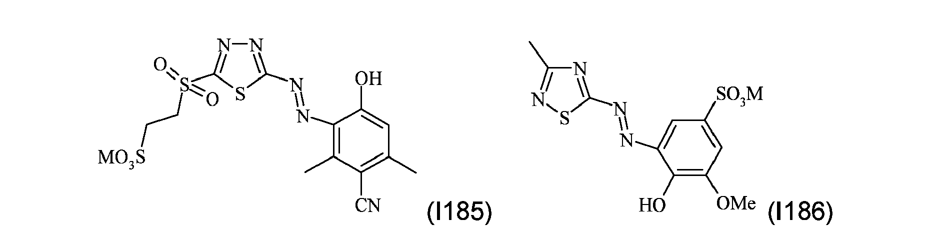 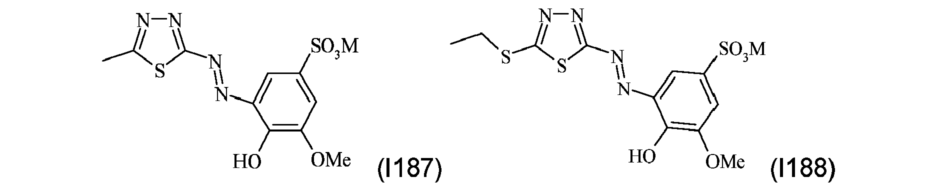 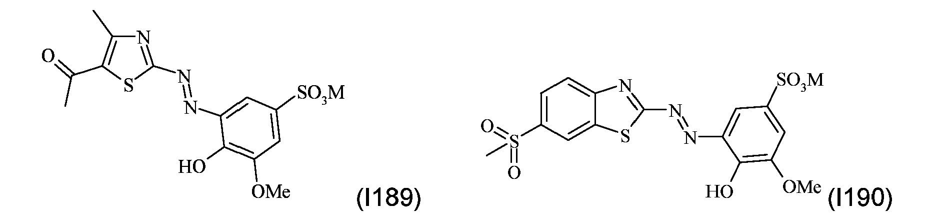 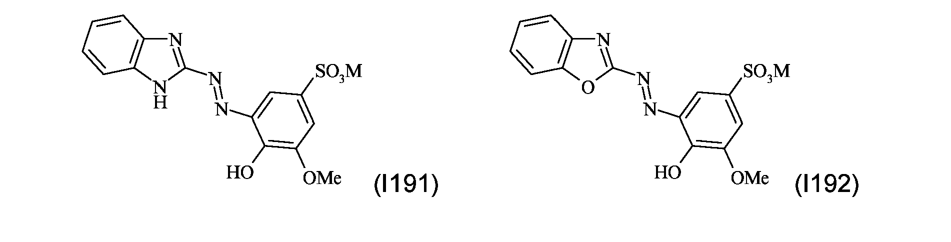 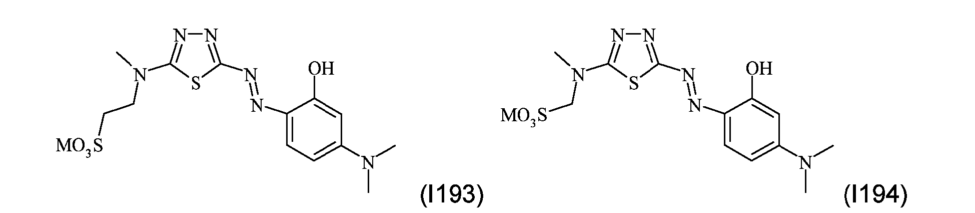 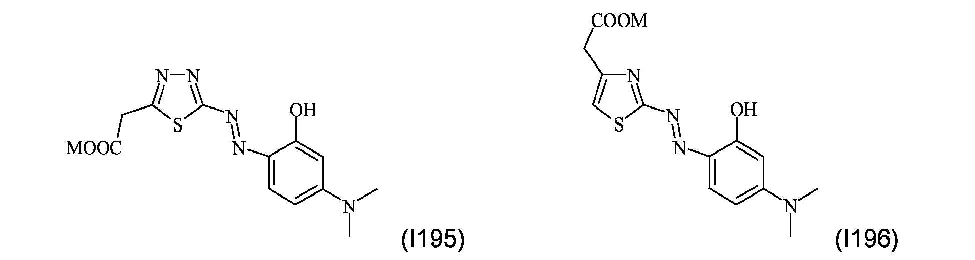 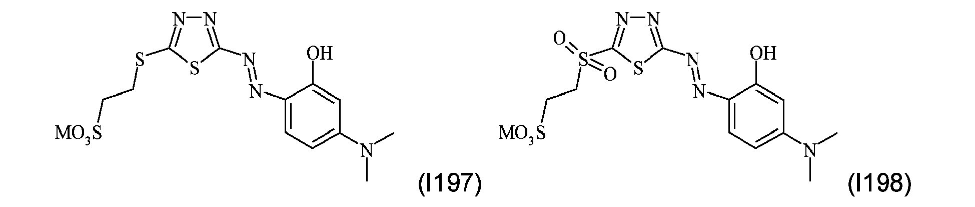 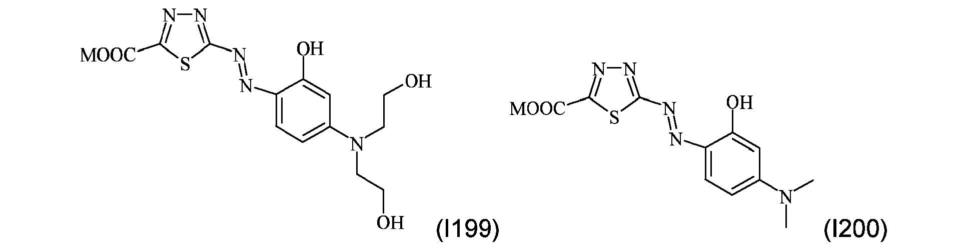 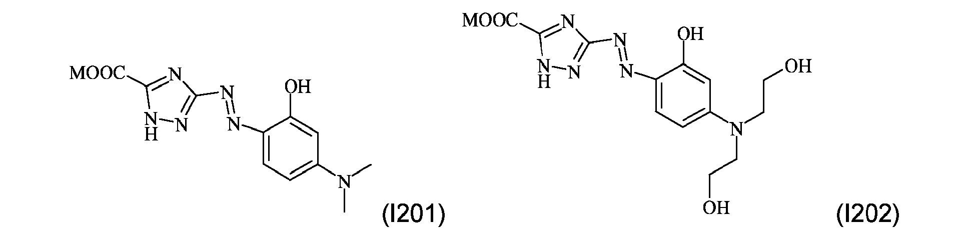 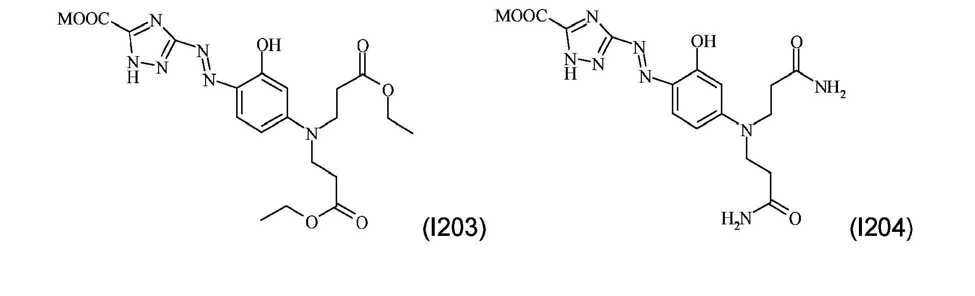 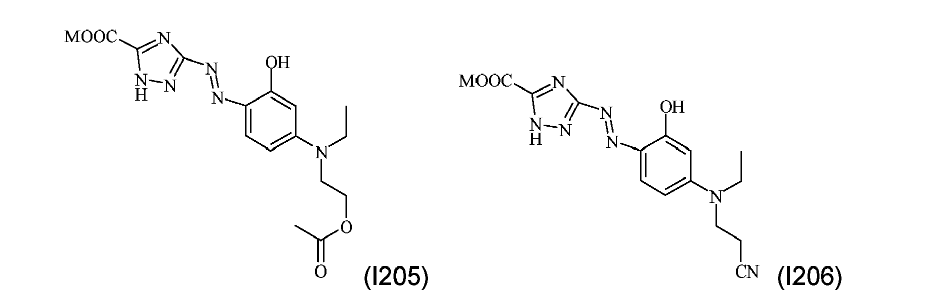 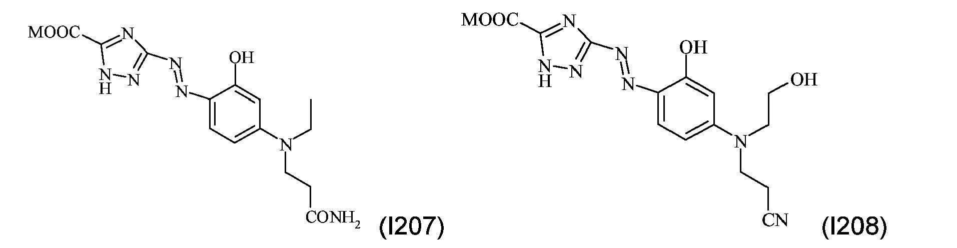 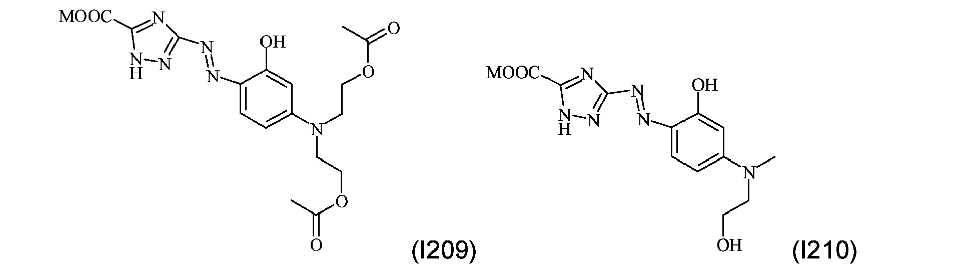 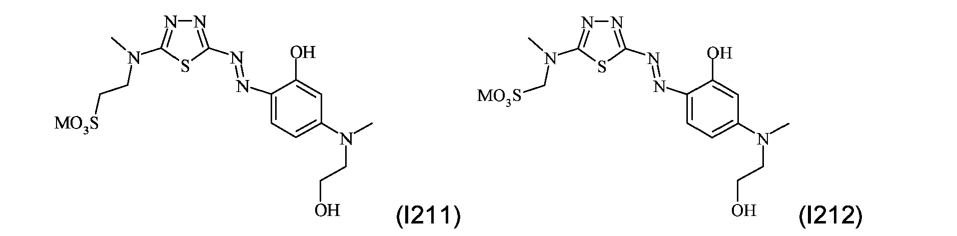 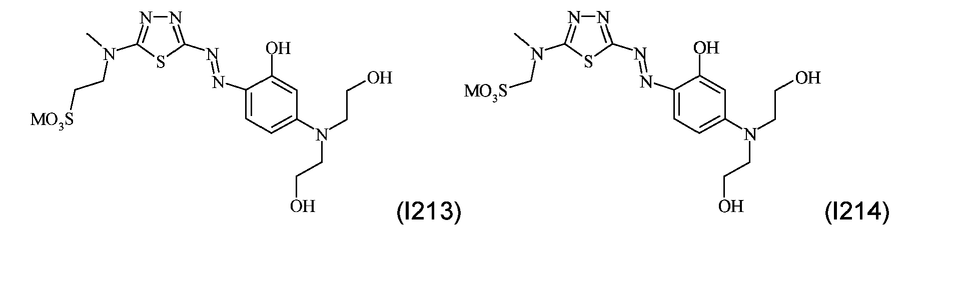 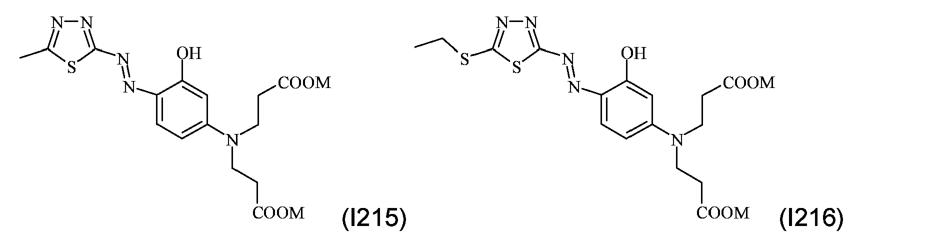 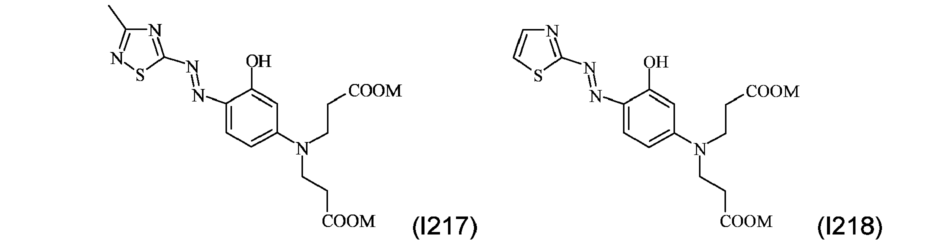 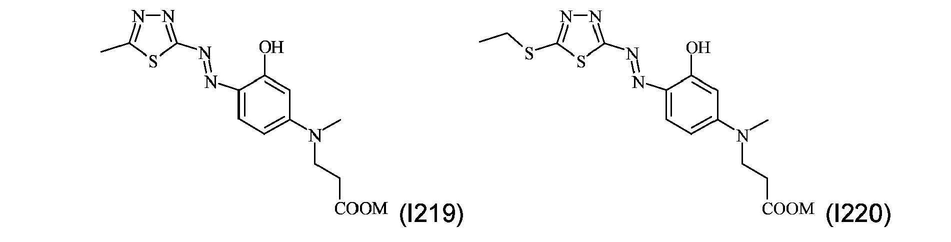 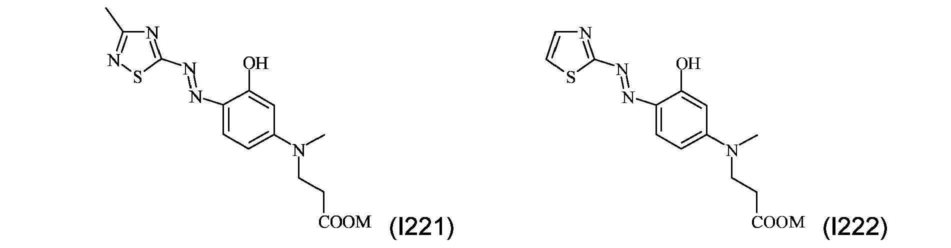 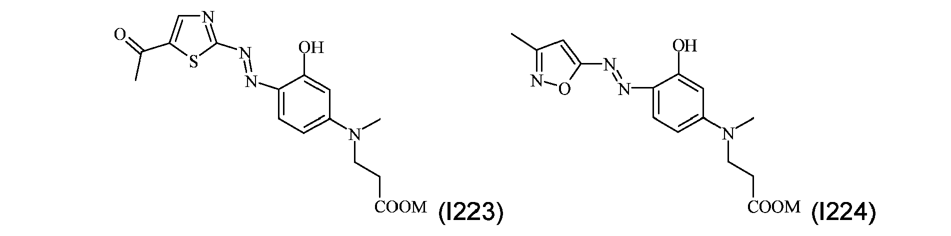 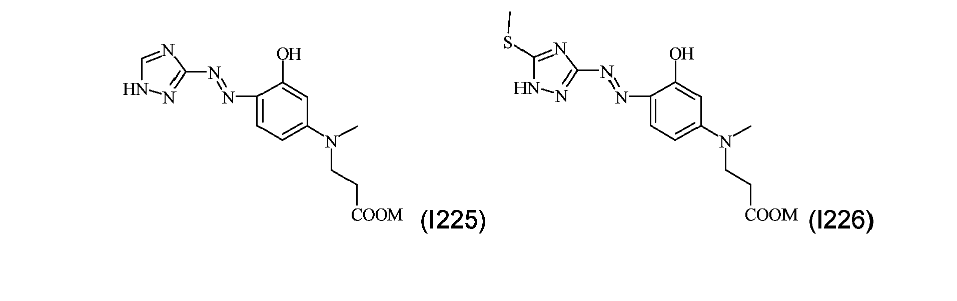 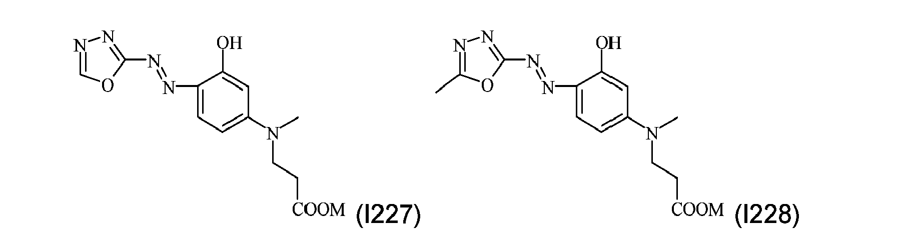 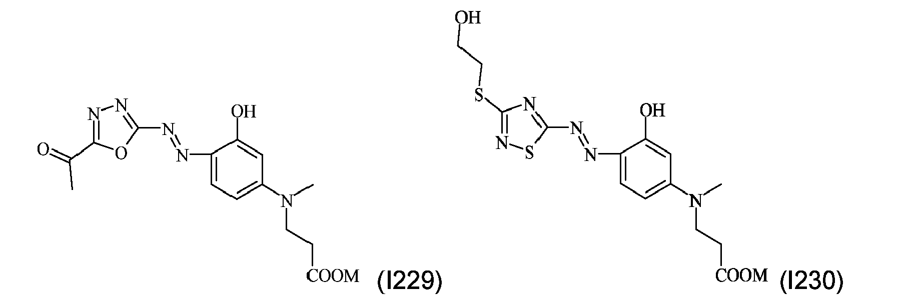 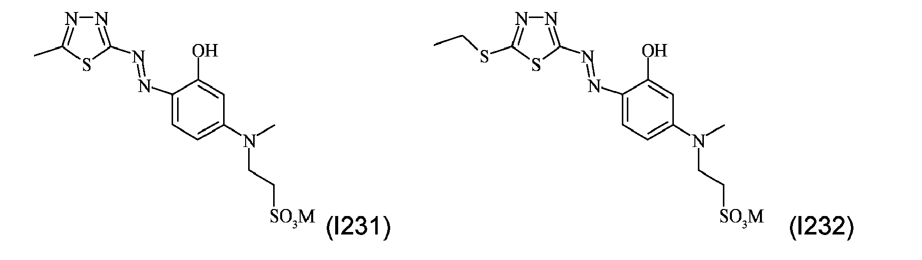 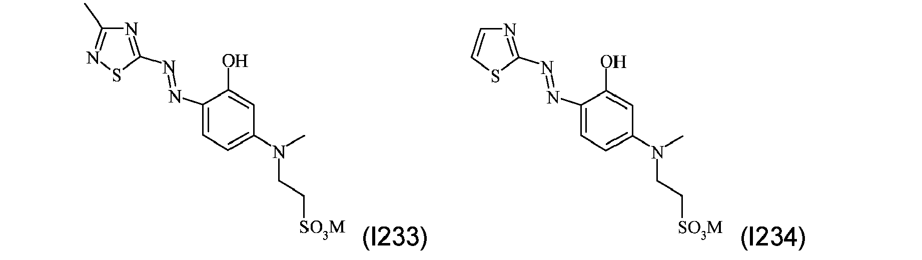 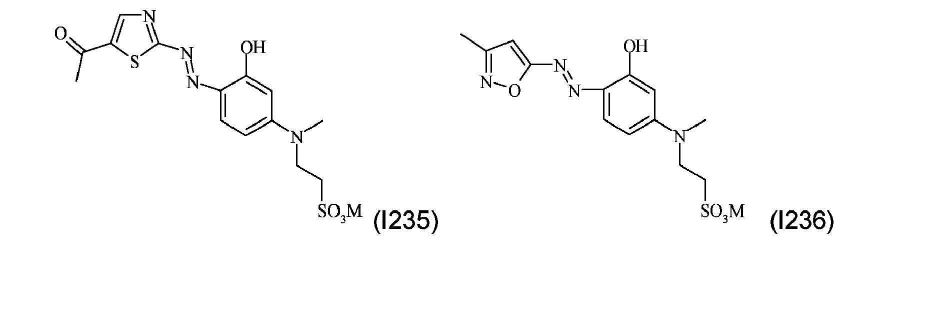 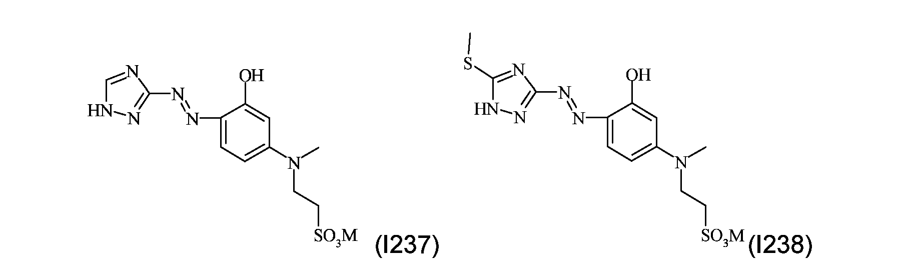 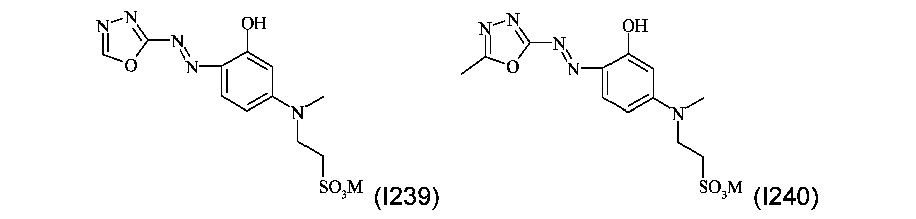 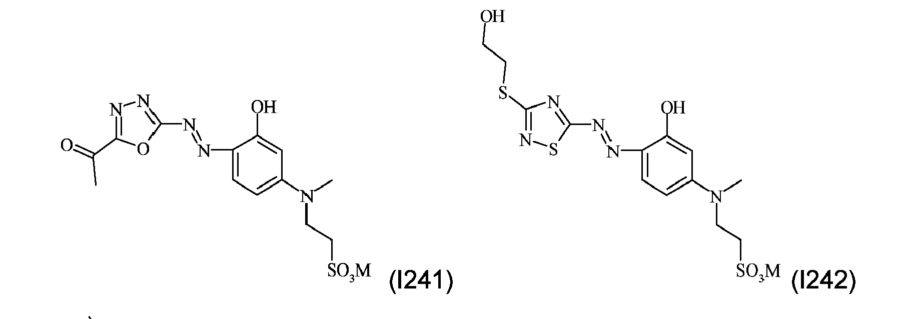 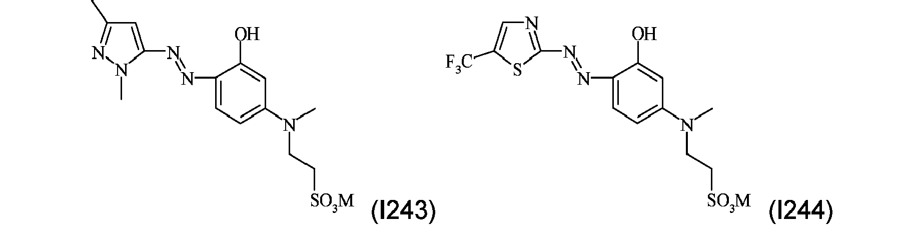 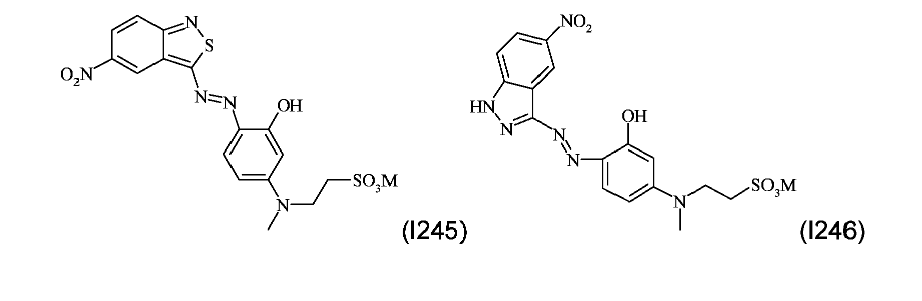 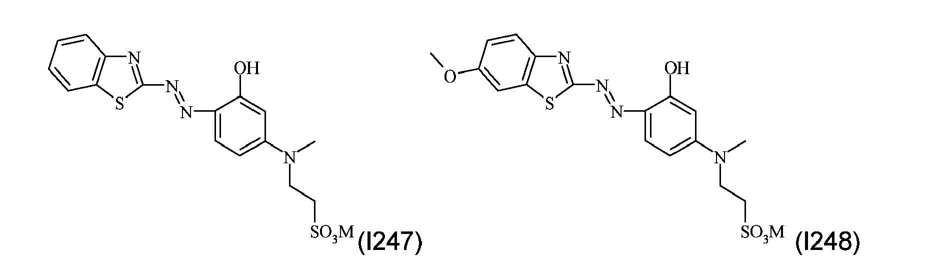 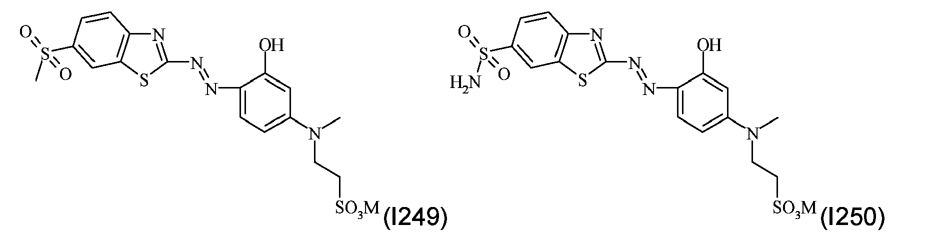 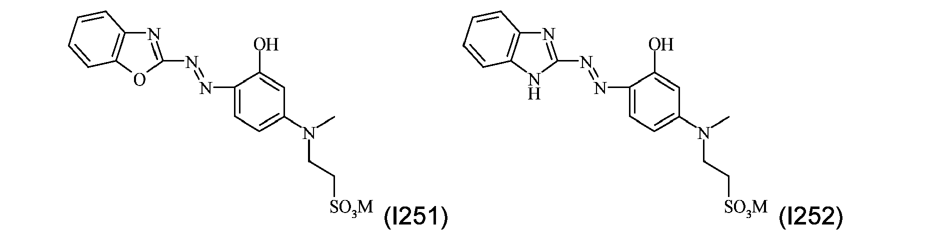 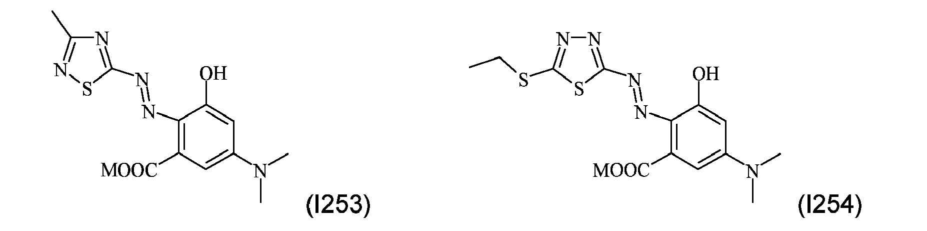 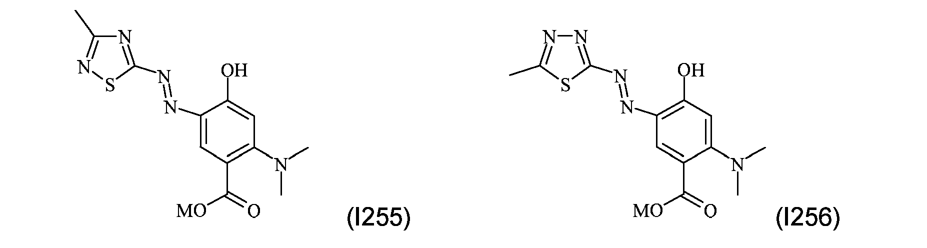 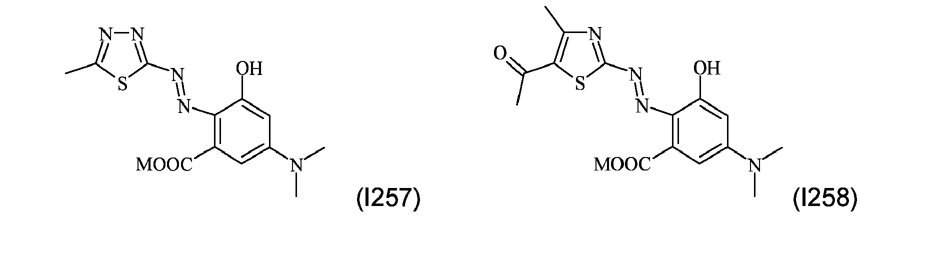 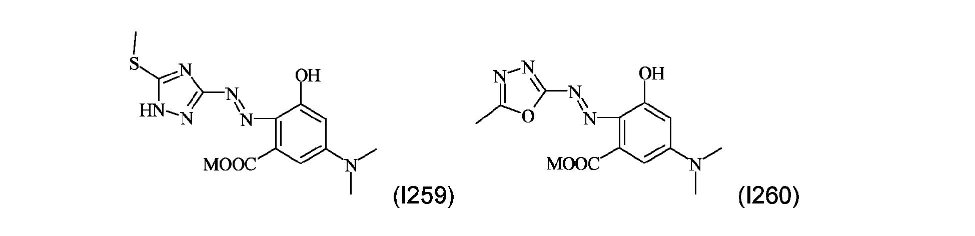 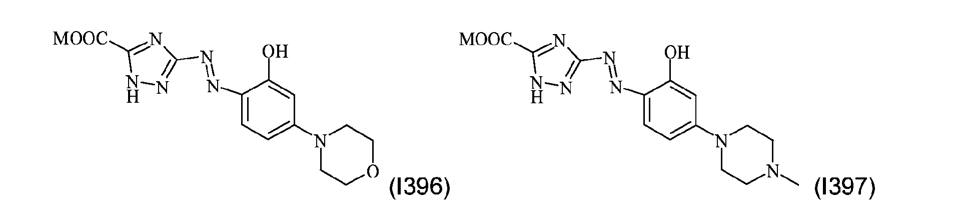 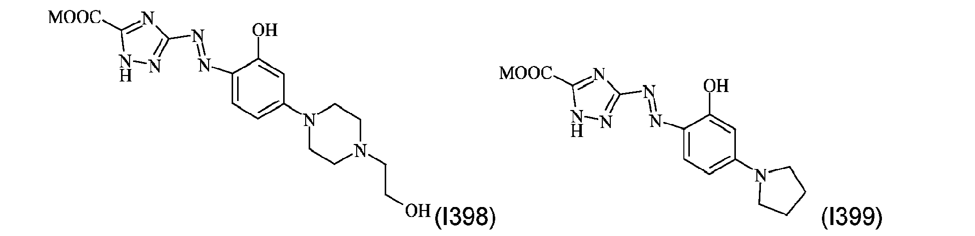 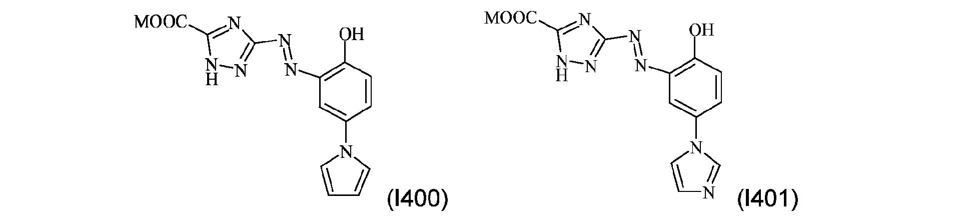 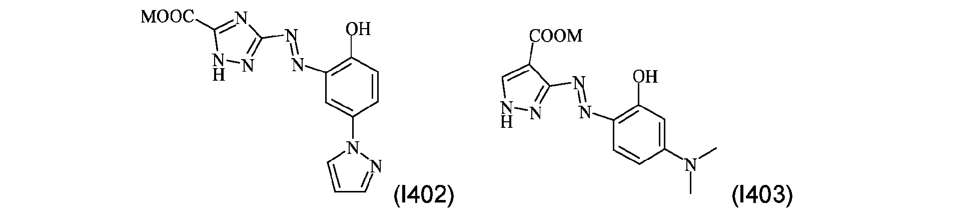 wherein M is hydrogen, sodium, potassium, lithium or ammonium. In still further preferred dyestuffs of the formula (I) D corresponds to the formula (D³)
wherein
G is defined as given above;
n is an integer from 0 to 1;
m is an integer from 0 to 2;
t is an integer from 0 to 2;
R⁵ is hydrogen, halogen, cyano, nitro, formyl, (C₁-C₄)-alkyl; (C₁-C₄)-alkoxy, (C₂-C₄)-acyl, (C₁-C₄)-alkoxycarbonyl, (C₂-C₄)-acyloxy, (C₂-C₄)-acylamino, N-(C₁-C₄)-alkyl-(C₂-C₄)-acylamino, (C₁-C₄)-alkylsulfonylamino, N-(C₁-C₄)-alkyl-(C₁-C₄)-alkylsulfonylamino, carbamoyl, N-mono-(C₁-C₄)-alkylcarbamoyl, N,N-di-(C₁-C₄)-alkylcarbamoyl, amino, mono-(C₁-C₄)-alkyl-amino, mono-(C₃-C₈)-cycloalkyl-amino, monoarylamino, diarylamino, mono-(C₁-C₄)-alkylmono-arylamino, mono-(C₃-C₈)-cycloalkylmono-arylamino, aminothiocarbonylamino, aminocarbonylamino, N,N-di-(C₁-C₄)-alkylaminothiocarbonylamino, N,N-di-(C₁-C₄)-alkylaminocarbonylamino, N,N,N-tri-(C₁-C₄)-alkylaminothiocarbonylamino, N,N,N-tri-(C₁-C₄)-alkylaminocarbonylamino, sulfamoyl, N-mono-(C₁-C₄)-alkyl-sulfamoyl, N,N-di-(C₁-C₄)-alkylsulfamoyl, aminosulfonylamino, N,N-di-(C₁-C₄)-alkylaminosulfonylamino, sulphur substituted by - SO₃M, (C₁-C₄)-alkylthio, (C₁-C₄)-alkylsulfonyl, iminomethyl, N-(C₁-C₄)-alkylimino-methyl or hydroxyiminomethyl; and wherein the (C₁-C₄)-alkyl groups mentioned above can be substituted by one or more substituents selected from the group consisting of hydroxyl, (C₁-C₄)-alkoxy, thio-(C₁-C₄)-alkoxy, poly(oxyalkylene), -SO₃M, halogen, cyano, nitro, (C₁-C₄)-alkoxythiocarbonyl, (C₁-C₄)-alkoxycarbonyl, (C₁-C₄)-acyloxy, carbamoyl, sulfamoyl, amino, N-(C₂-C₄)-acylamino, N-(C₁-C₄)-alkylsulfonylamino, aminothiocarbonylamino, aminocarbonylamino, N-mono-(C₁-C₄)-alkyl-amino, N,N-di-(C₁-C₄)-alkyl-amino, (C₁-C₄)-alkoxy-(C₁-C₄)-alkylamino, thio-(C₁-C₄)-alkoxy-(C₁-C₄)-alkyl-amino, amino-(C₁-C₄)-alkoxy, N-mono-(C₁-C₄)-alkylamino-(C₁-C₄)-alkoxy, N,N-di-(C₁-C₄)-alkyl-amino-(C₁-C₄)-alkoxy, amino-(C₁-C₄)-alkylthioxy, N-mono-(C₁-C₄)-alkylamino-(C₁-C₄)-alkylthioxy and N,N-di-(C₁-C₄)-alkylamino-(C₁-C₄)-alkylthioxy.

In especially preferred dyestuffs of the formula (I) D corresponds to a group of the formula (D³a) 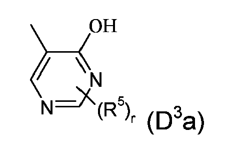 wherein
R⁵ is hydroxy, amino, chlorine, (C₁-C₄)-alkyl, (C₁-C₄)-alkoxy, (C₁-C₄)-thioalkoxy, phenyl, phenoxy or phenylamino; and r is 1 or 2.

Examples of dyestuffs of this type are the dyestuffs of the formulae (I261) to (I286). 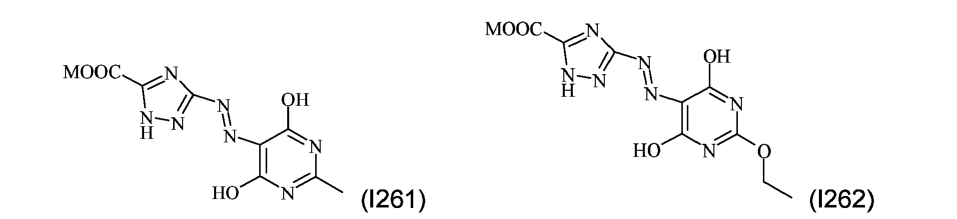 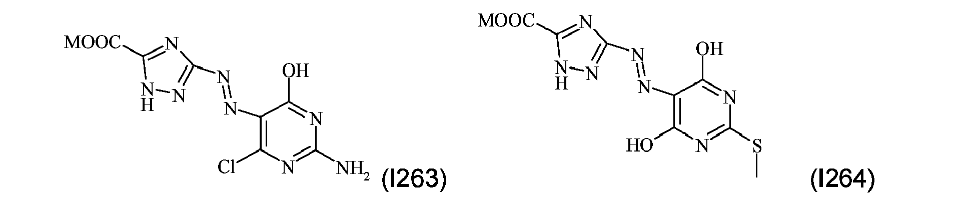 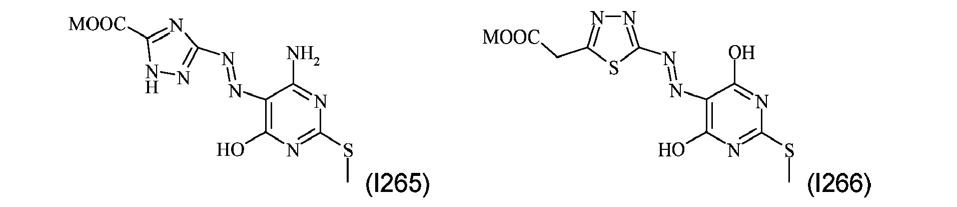 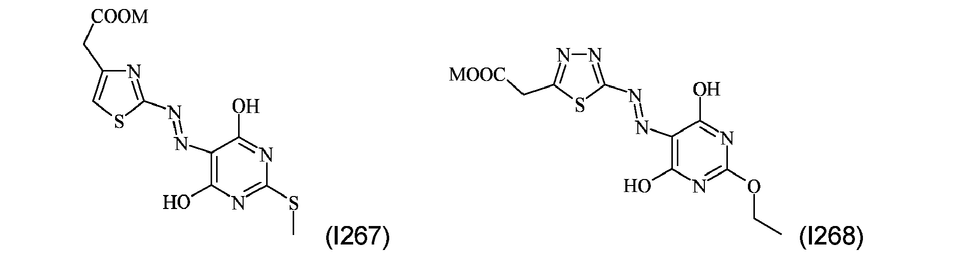 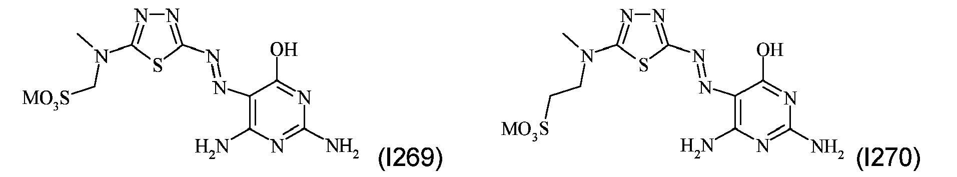 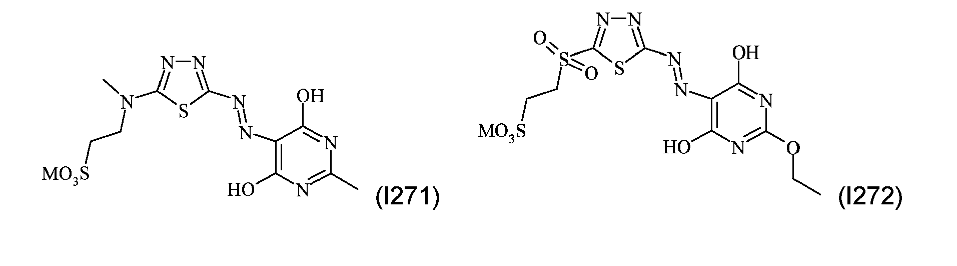 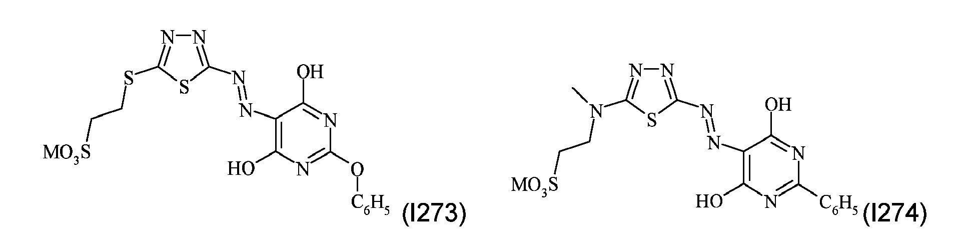 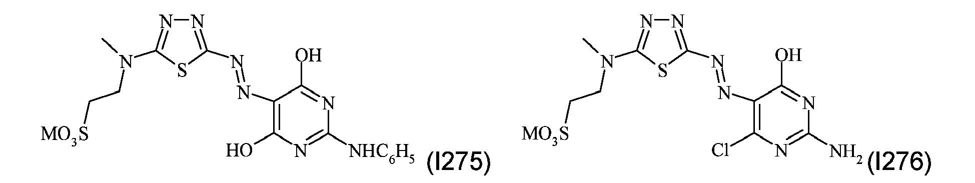 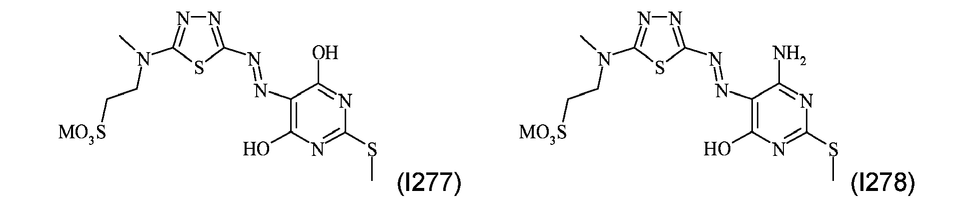 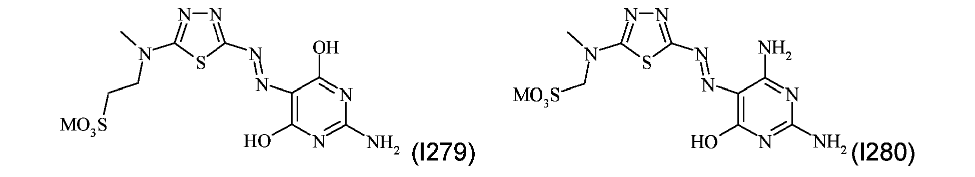 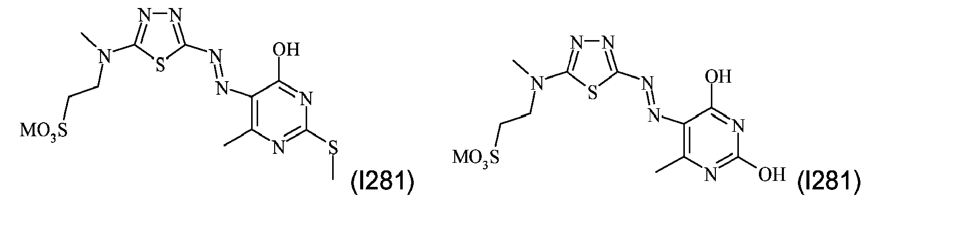 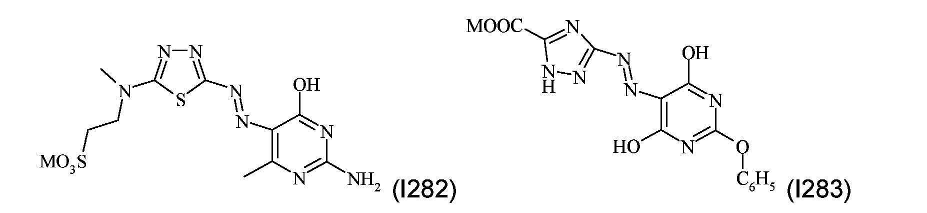 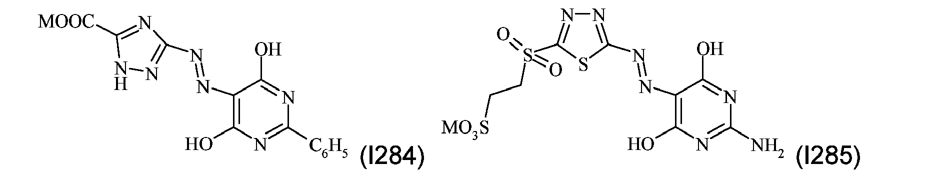 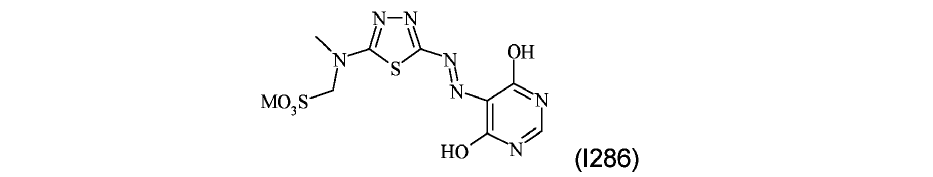 wherein M is hydrogen, sodium, potassium, lithium or ammonium.

In still further preferred dyestuffs of the formula (I) D corresponds to the formula (D⁴)
wherein
G is defined as given above;
n is an integer from 0 to 2;
s is an integer from 0 to 2;
R⁷ is (C₁-C₄)-alkyl or -COOM; and
R⁸ is halogen, nitro, amino, acylamino, alkylsulfonylamino, aminocarbonylamino, aminosulfonylamino, dialkylaminosulfonylamino, carbamoyl, sulfamoyl, N-(C₁-C₄)-monoalkyl-amino, N,N-di-(C₁-C₄)-alkyl-amino and (C₁-C₄)-alkylsukfonyl, whereby the alkyl group can be substituted by one or more substituents selected from the group consisting of hydroxyl, (C₁-C₄)-alkoxy, (C₁-C₄)-alkylthio, poly(oxyalkylene), -SO₃M, halogen, cyano, nitro, amino, halogen, carbamoyl, sulfamoyl, aminocarbonylamino, aminosulfonylamino, N-(C₁-C₄)-monoalkyl-amino, N,N-di-(C₁-C₄)-alkyl-amino; and wherein the substituents themselves can be substituted by one or more substituents selected from the group consisting of hydroxyl and -SO₃M; and
M is defined as given above.

In especially preferred dyestuffs of the formula (I) D corresponds to a group of the formula (D⁴a) to (D⁴c) 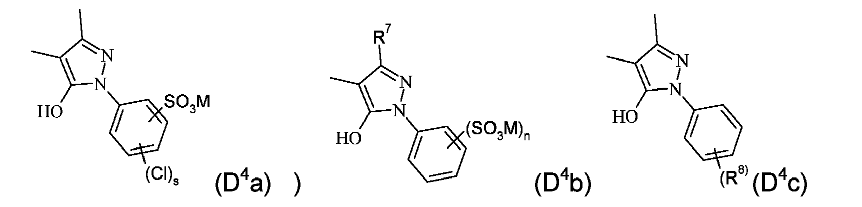 wherein
s is and integer from 0 to 2;
n is an integer from 1 to 2;
R⁷ is (C₁-C₄)-alkyl or -COOM;
R⁸ is acylamino, alkylsulfonylamino, dialkylaminosulfonylamino, N-(C₁-C₄)-monoalkyl-amino or (C₁-C₄)-alkylsukfonyl, whereby the alkyl group can be substituted by one or more substituents selected from the group consisting of hydroxyl, -SO₃M, N-(C₁-C₄)-monoalkyl-amino, N,N-di-(C₁-C₄)-alkyl-amino; and wherein the substituents themselves can be substituted by one or more substituents selected from the group consisting of hydroxyl and -SO₃M; and
M is defined as given above.

Examples of dyestuffs of this type are the dyestuffs of the formulae (I287) to (I331 ) and (I404) to (I417). 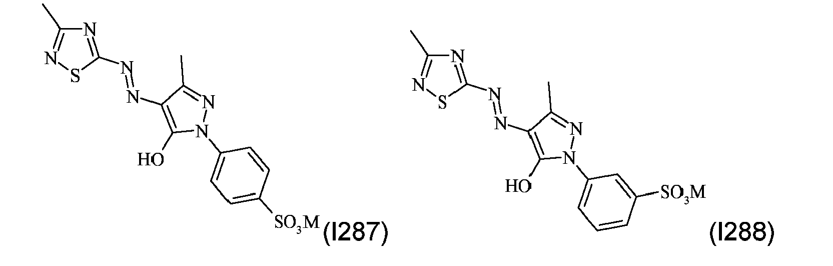 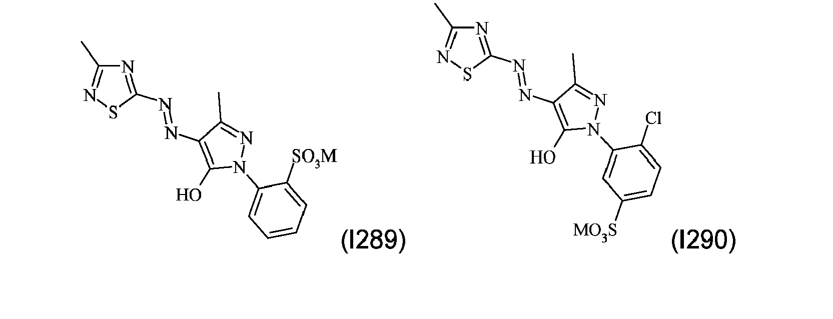 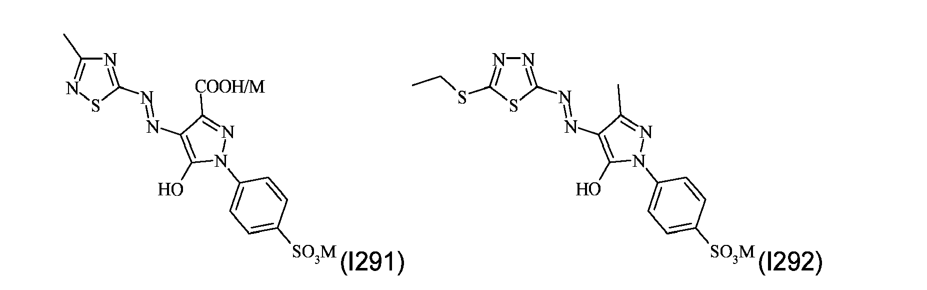 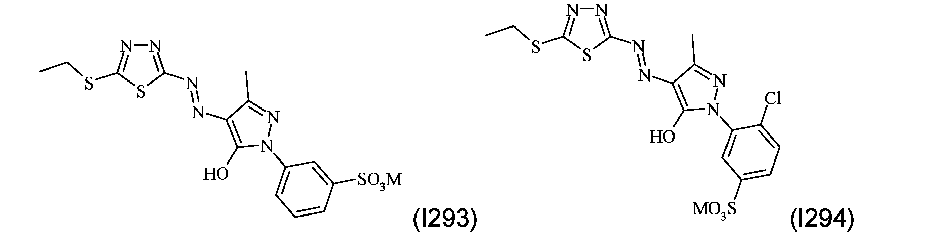 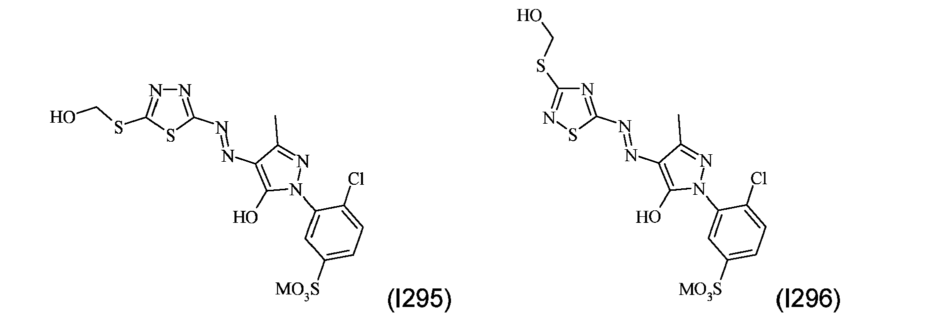 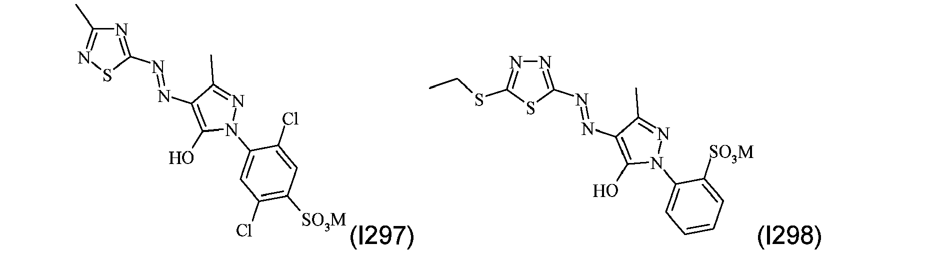 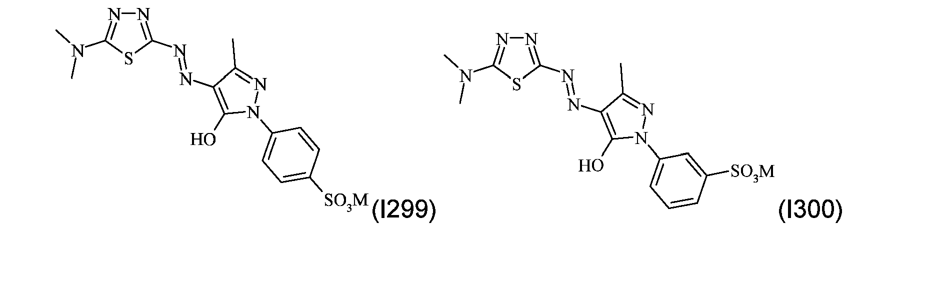 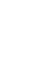 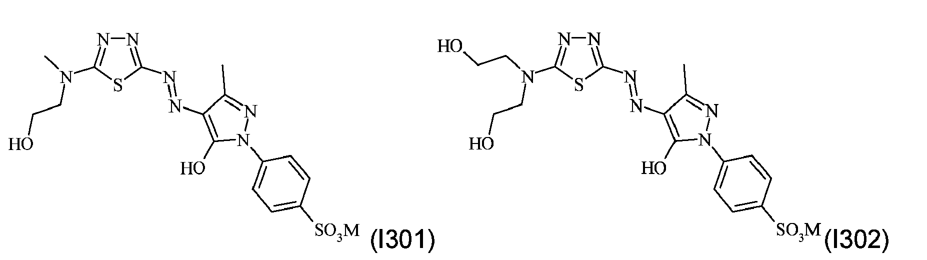 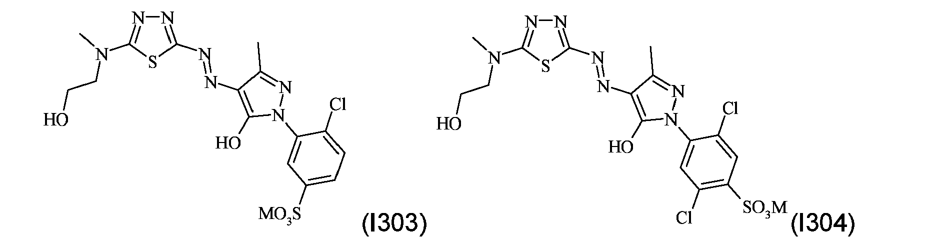 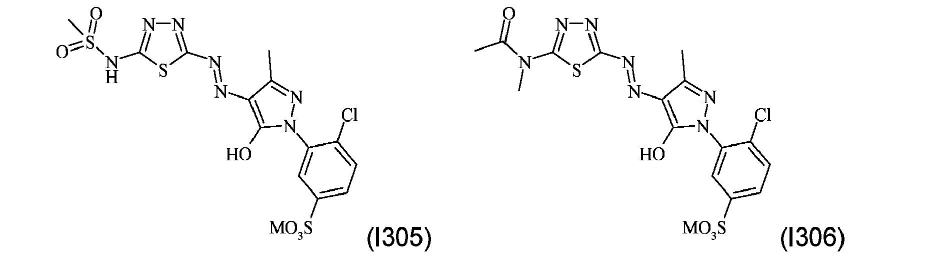 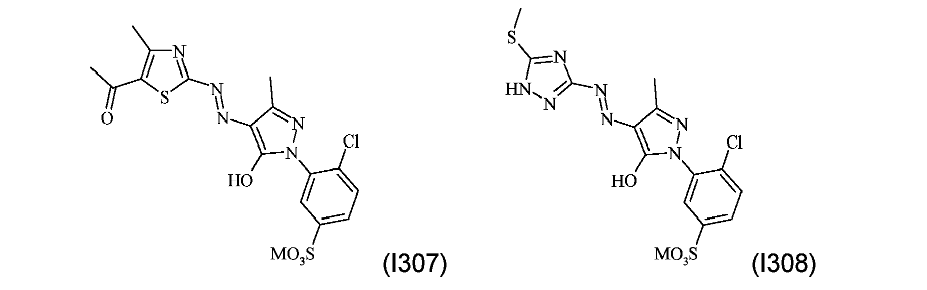 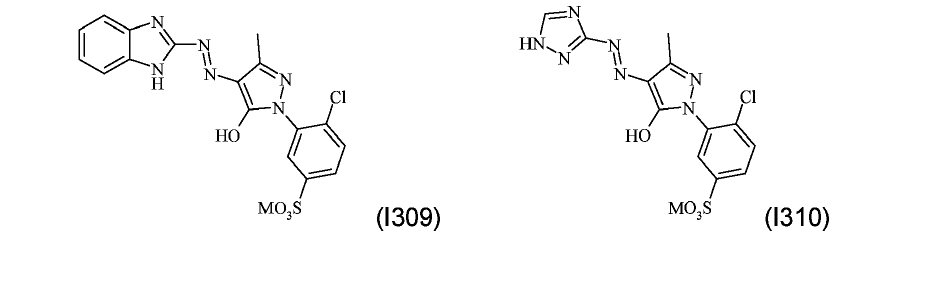 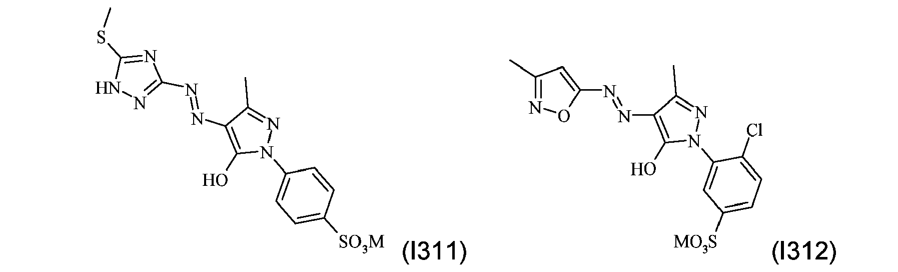 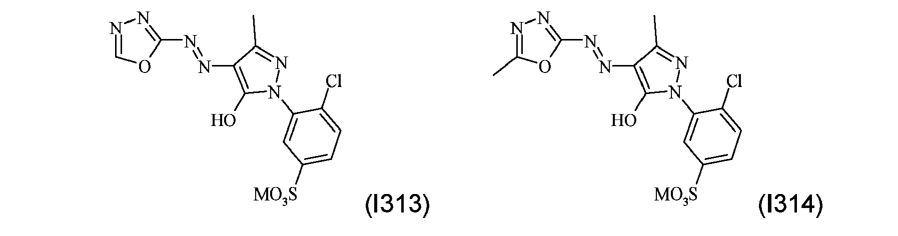 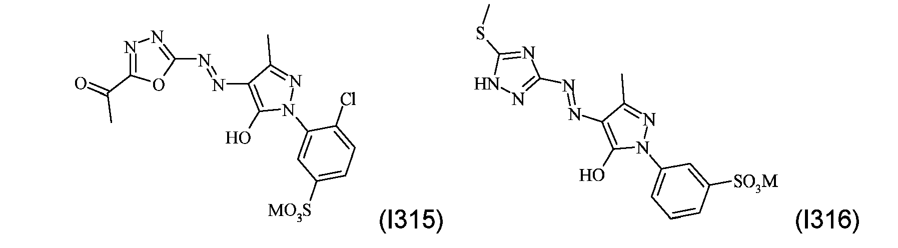 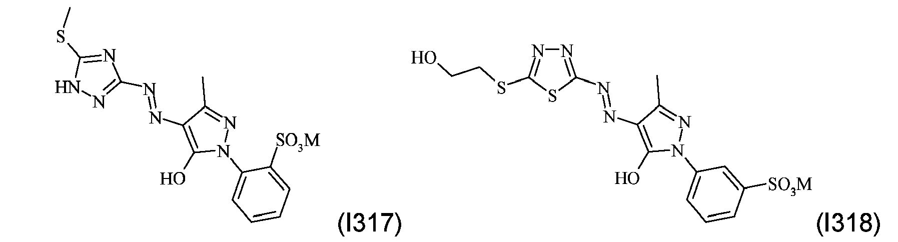 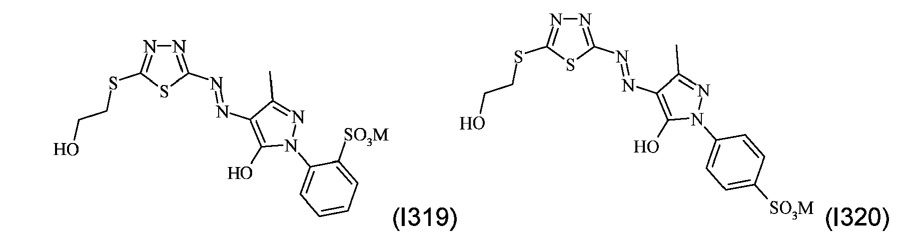 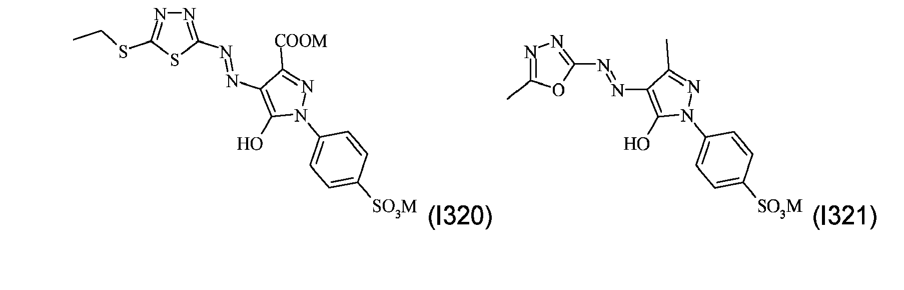 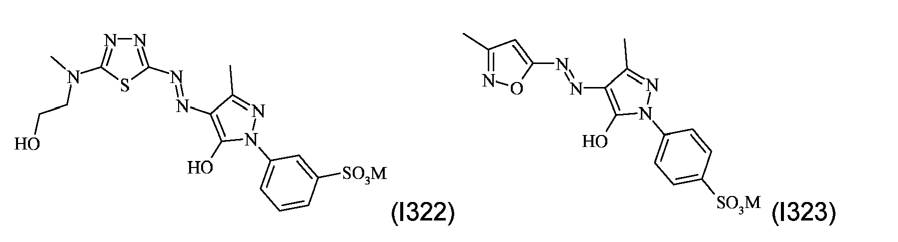 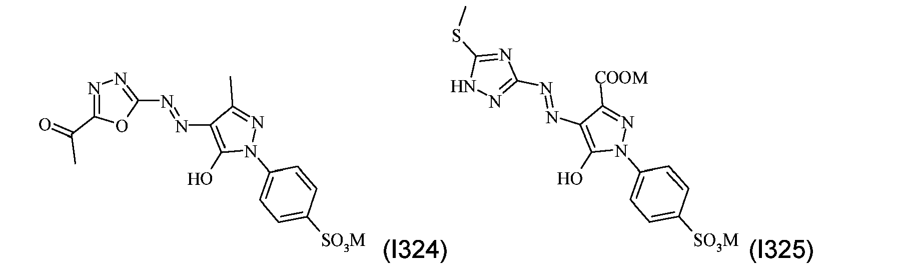 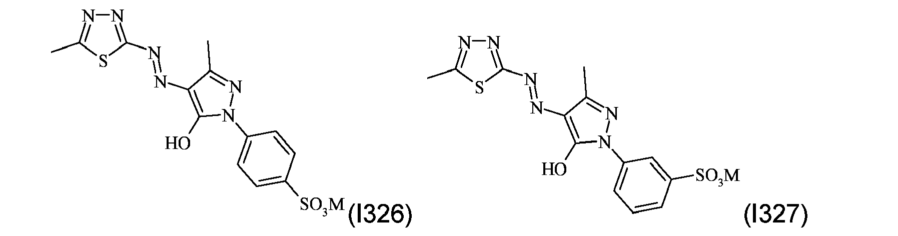 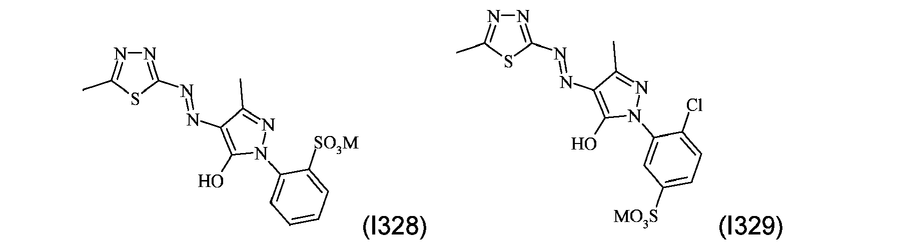 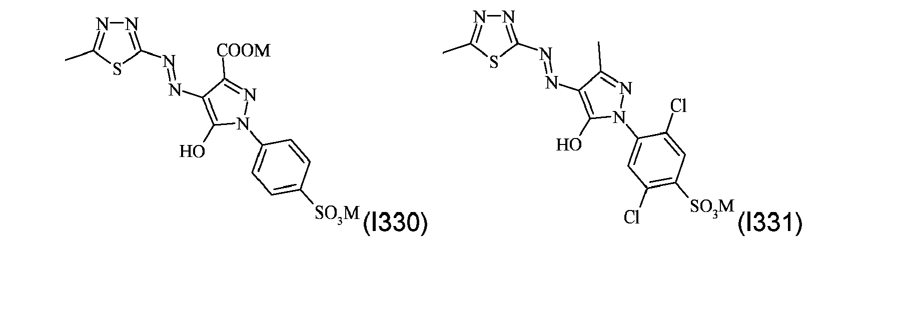 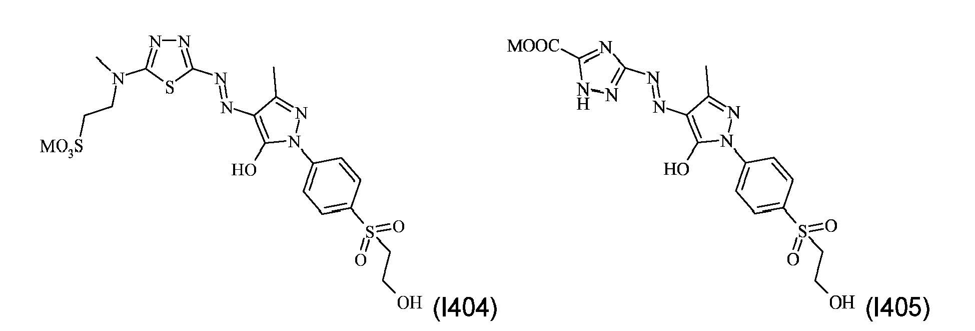 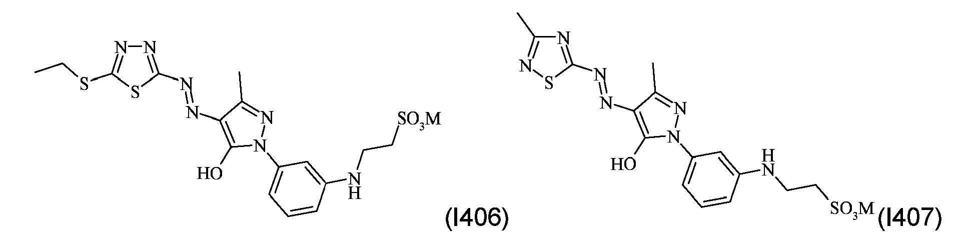 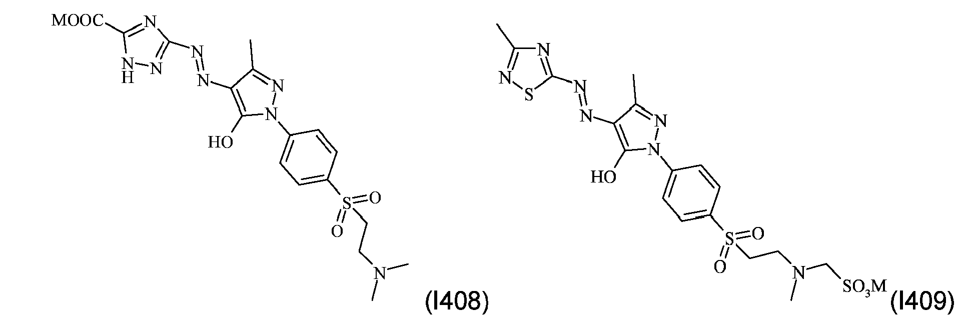 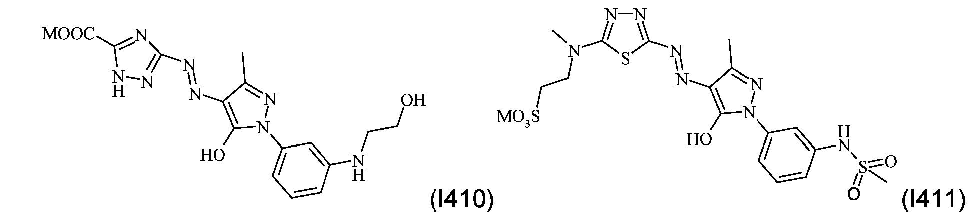 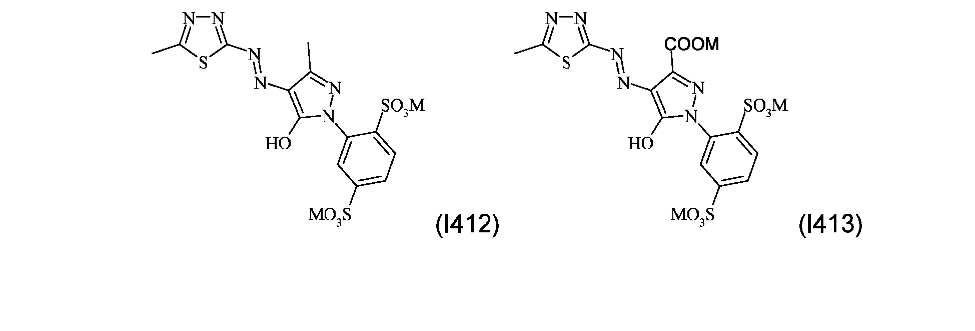 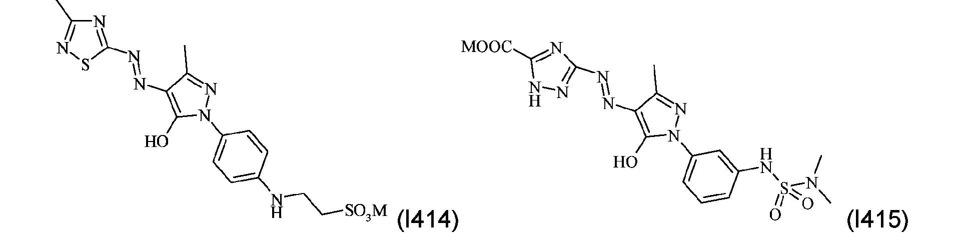 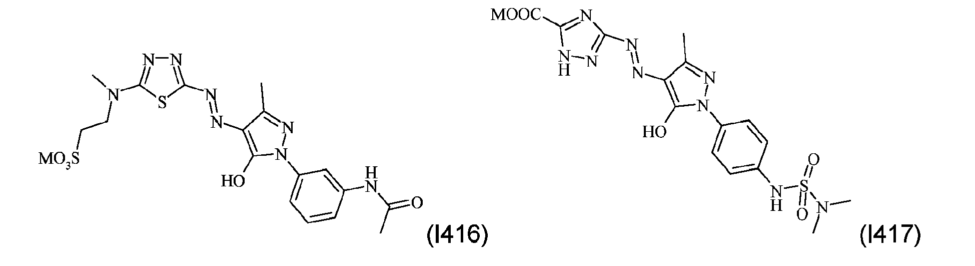 wherein M is hydrogen, sodium, potassium, lithium or ammonium.

In still further preferred dyestuffs of the formula (I) D corresponds to the formula (D⁵)
wherein
R⁹ and R¹⁰, independently, are hydrogen, halogen, cyano, nitro, (C₁-C₄)-alkyl, (C₁-C₄)-alkoxy, formyl, (C₂-C₄)-acyl, (C₁-C₄)-alkoxycarbonyl, (C₂-C₄)-acyloxy, (C₂-C₄)-acylamino, N-(C₁-C₄)-alkyl-(C₂-C₄)-acylamino, (C₁-C₄)-alkylsulfonylamino, N-(C₁-C₄)-alkyl-(C₁-C₄)-alkylsulfonylamino, carbamoyl, N-mono-(C₁-C₄)-alkylcarbamoyl, N,N-di-(C₁-C₄)-alkylcarbamoyl, amino, mono-(C₁-C₄)-alkylamino, mono-(C₃-C₈)-cydoalkylamino, di-(C₁-C₄)-alkylamino, di-(C₃-C₈)-cycloalkylamino, monoarylamino, diarylamino, mono-(C₁-C₄)-alkyl-monoarylamino, mono-(C₃-C₈)-cydoalkyl-monoarylamino, aminocarbonylamino, aminothiocarbonylamino, N,N-di-(C₁-C₄)-alkylaminothiocarbonylamino, N,N-di-(C₁-C₄)-alkylaminocarbonylamino, N,N,N-tri-(C₁-C₄)-alkylaminothiocarbonylamino, N,N,N-tri-(C₁-C₄)-alkylaminocarbonylamino, sulfamoyl, N-mono-(C₁-C₄)-alkyl-sulfamoyl, N,N-di-(C₁-C₄)-alkylsulfamoyl, aminosulfonylamino, N,N-di-(C₁-C₄)-alkylaminosulfonylamino, sulphur substituted by -SO₃M, (C₁-C₄)-alkylthio, (C₁-C₄)-alkylsulfonyl, iminomethyl, N-(C₁-C₄)-alkylimino-methyl, hydroxyiminomethyl or G;
and wherein the (C₁-C₄)-alkyl groups mentioned above can be substituted by one or more substituents selected from the group consisting of hydroxyl, (C₁-C₄)-alkoxy, thio-(C₁-C₄)-alkoxy, poly(oxyalkylene), -SO₃M, -COOM, halogen, cyano, nitro, (C₁-C₄)-alkoxythio-carbonyl, (C₁-C₄)-alkoxycarbonyl, (C₁-C₄)-acyloxy, carbamoyl, sulfamoyl, amino, N-(C₂-C₄)-acylamino, N-(C₁-C₄)-alkylsulfonylamino, aminothiocarbonylamino, aminocarbonylamino, N-mono-(C₁-C₄)-alkyl-amino, N,N-di-(C₁-C₄)-alkyl-amino, (C₁-C₄)-alkoxy-(C₁-C₄)-alkylamino, thio-(C₁-C₄)-alkoxy-(C₁-C₄)-alkyl-amino, amino-(C₁-C₄)-alkoxy, N-mono-(C₁-C₄)-alkylamino-(C₁-C₄)-alkoxy, N,N-di-(C₁-C₄)-alkyl-amino-(C₁-C₄)-alkoxy, amino-(C₁-C₄)-alkylthioxy, N-mono-(C₁-C₄)-alkylamino-(C₁-C₄)-alkylthioxy and N,N-di-(C₁-C₄)-alkylamino-(C₁-C₄)-alkylthioxy; and
R¹¹ is (C₁-C₄)-alkyl, (C₃-C₈)-cydoalkyl, (C₃-C₈)-cydoalkyl-(C₁-C₄)-alkyl, aryl, aryloxy-(C₁-C₄)-alkyl, arylthioxy-(C₁-C₄)-alkyl, or (C₁-C₄)-alkyl which is substituted by one or more substituents selected from the group consisting of aryl, hydroxyl, (C₁-C₄)-alkoxy, thio-(C₁-C₄)-alkoxy, poly(oxyalkylene), halogen, cyano, nitro, -SO₃M, -COOM, (C₁-C₄)-alkoxycarbonyl, (C₁-C₄)-alkoxythiocarbonyl, (C₂-C₄)-acyloxy, carbamoyl, sulfamoyl, amino, N-(C₂-C₄)-acylamino, N-(C₁-C₄)-alkylsulfonylamino, aminocarbonylamino, aminothiocarbonylamino, N,N-di-(C₁-C4)-alkylamino, (C₁-C₄)-alkoxy-(C₁-C₄)-alkylamino, thio-(C₁-C₄)-alkoxy-(C₁-C₄)-alkylamino, amino-(C₁-C₄)-alkyloxy, N,N-di-(C₁-C₄)-alkylamino-(C₁-C₄)-alkoxy, amino-(C₁-C₄)-alkylthioxy and N,N-di-(C₁-C₄)-alkylamino-(C₁-C₄)-alkylthioxy.

In especially preferred dyestuffs of the formula (I) D corresponds to a group of the formula (D⁵), wherein
R⁹ is (C₁-C₄)-alkyl or (C₁-C₄)-alkyl substituted by -COOM and is most preferably methyl; R¹⁰ is hydrogen, aminocarbonyl, -COOM or cyano; R¹¹ is (C₁-C₄)-alkyl or (C₁-C₄)-alkyl substituted by hydroxy, -SO₃M or -COOM; and
M is hydrogen, sodium, potassium, lithium or ammonium.

Examples of dyestuffs of this type are the dyestuffs of the formulae (I332) to (I387). 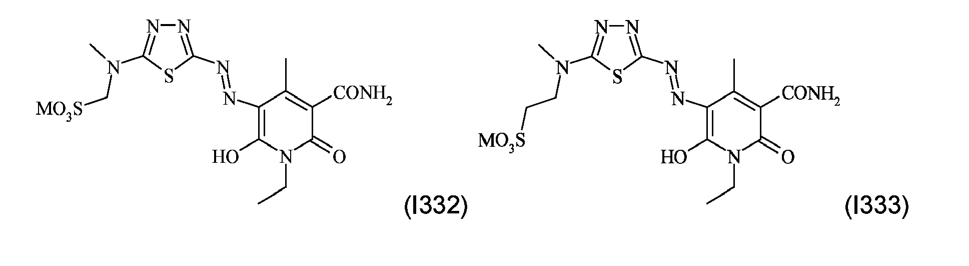 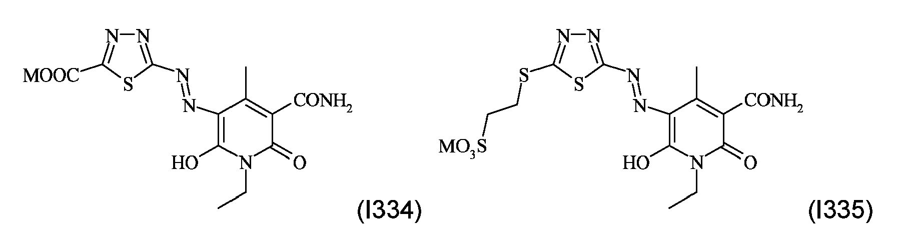 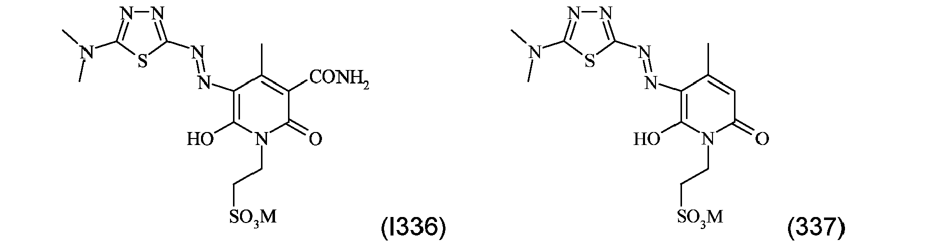 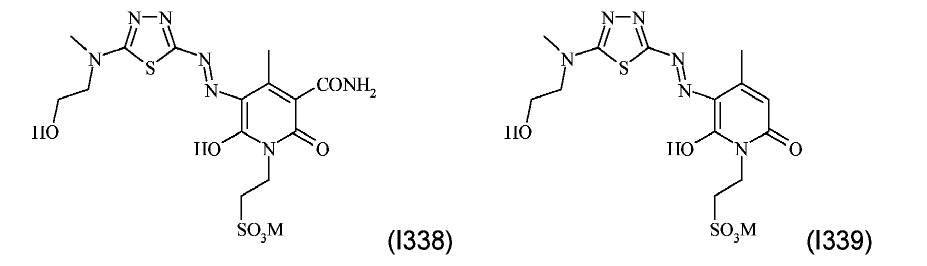 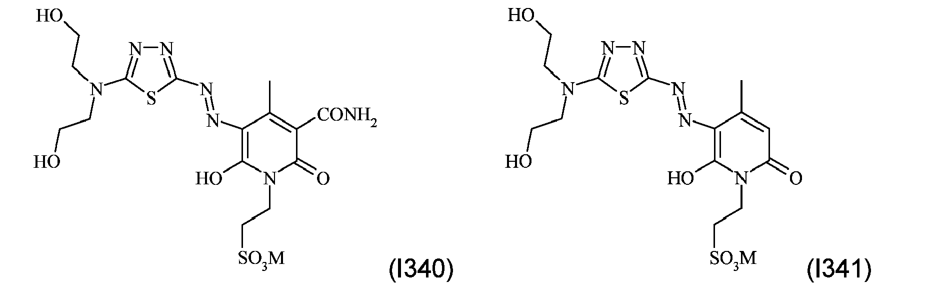 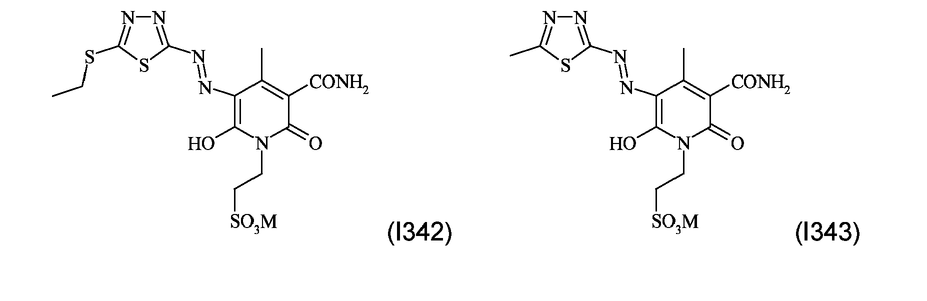 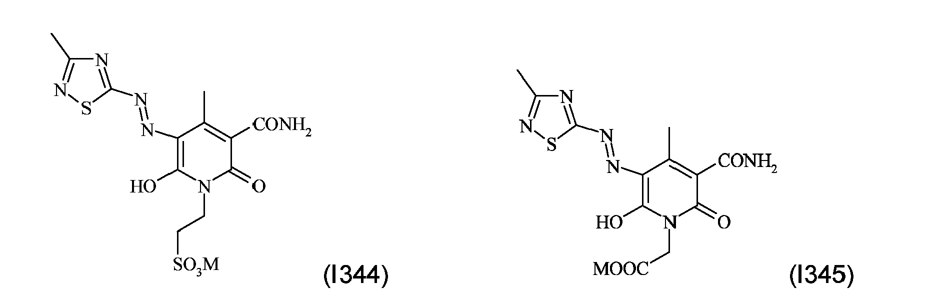 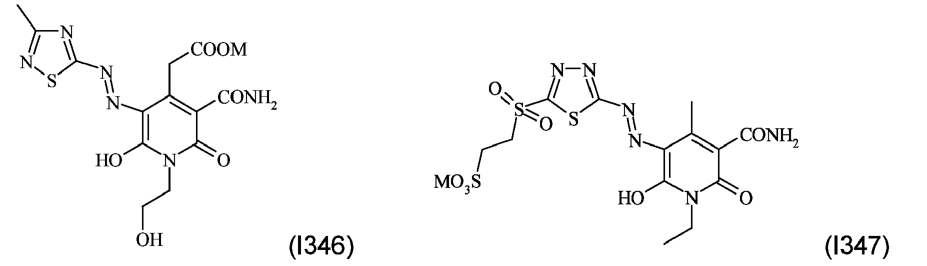 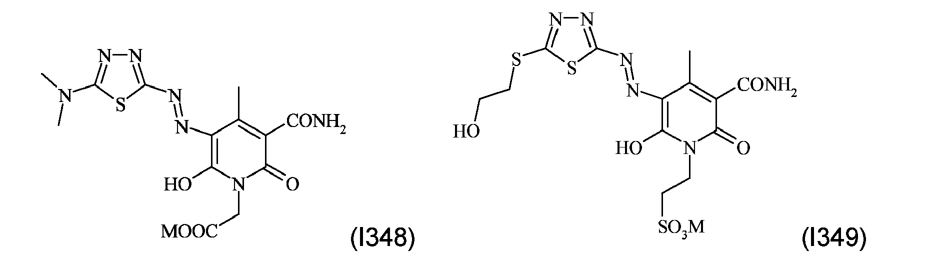 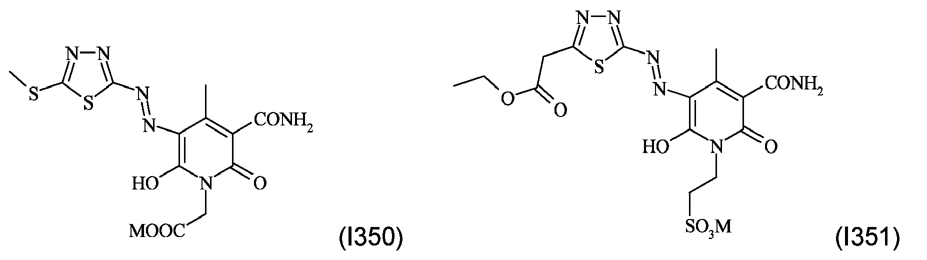 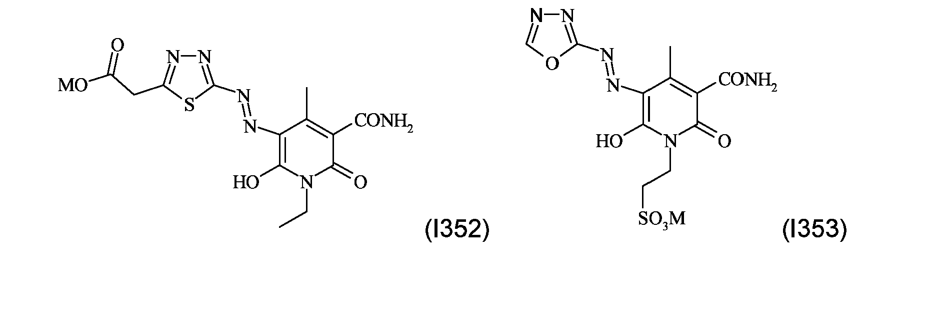 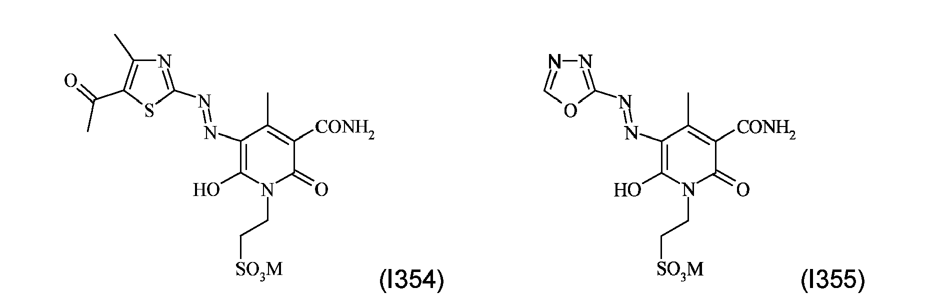 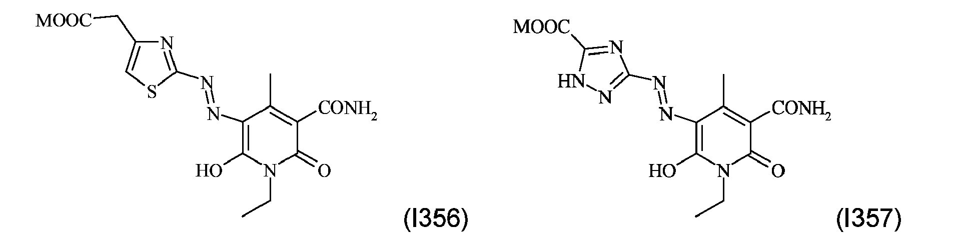 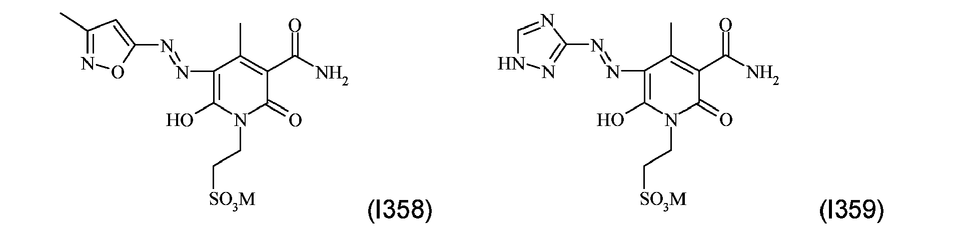 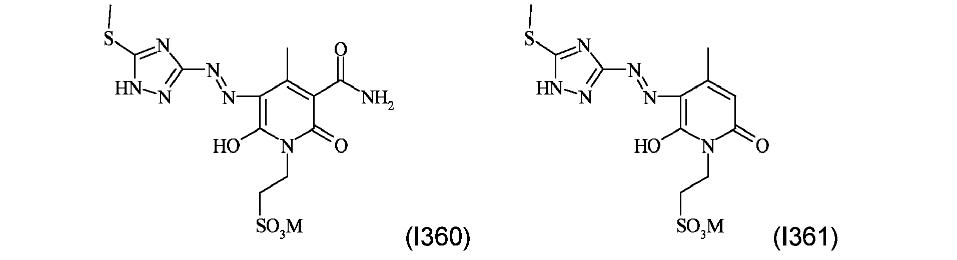 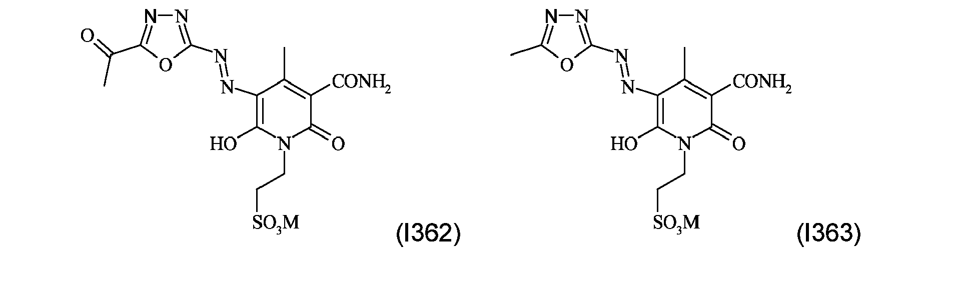 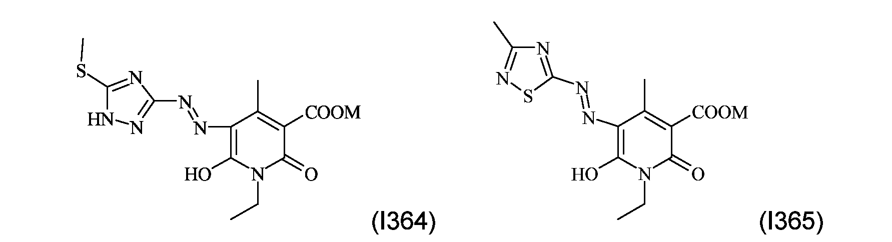 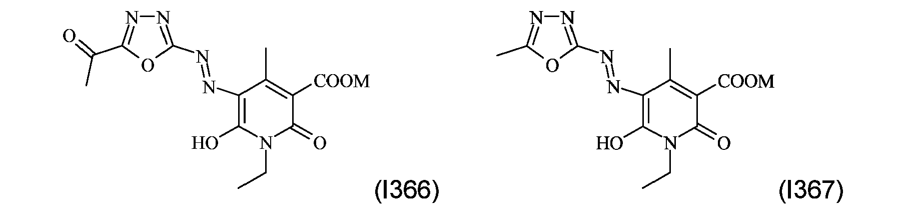 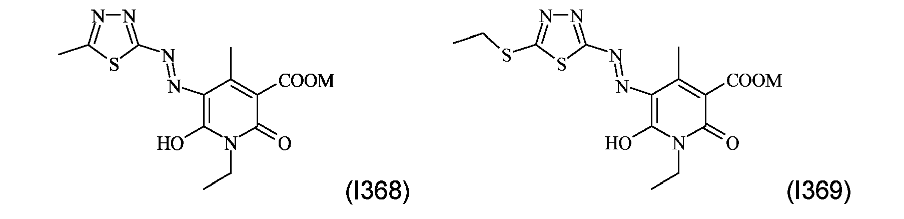 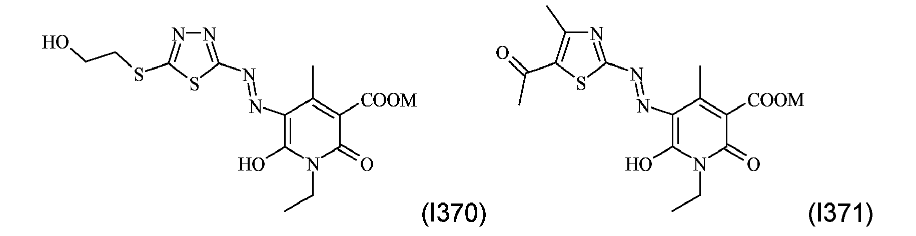 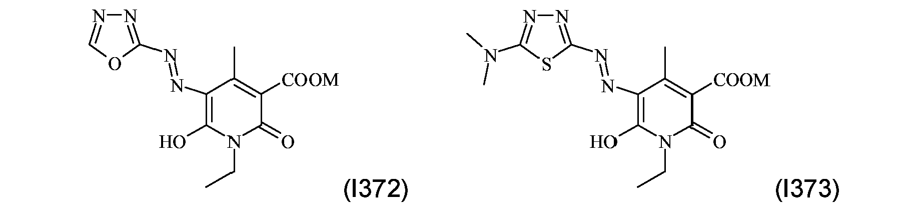 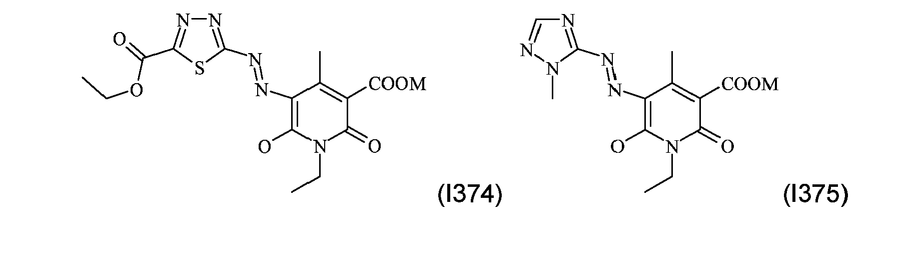 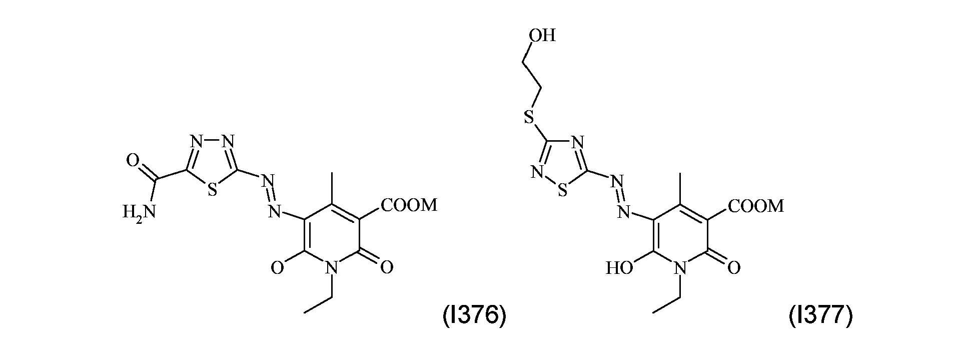 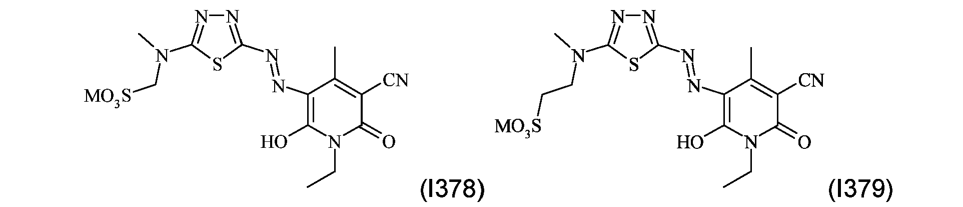 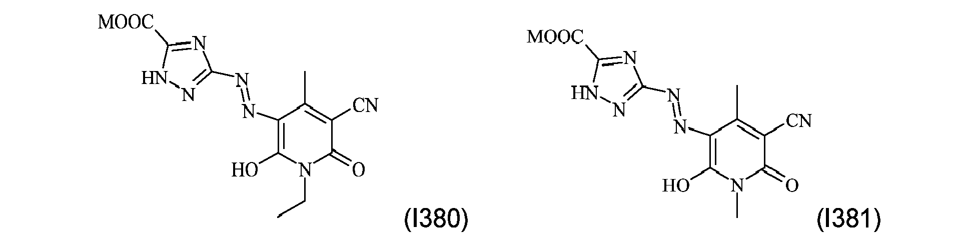 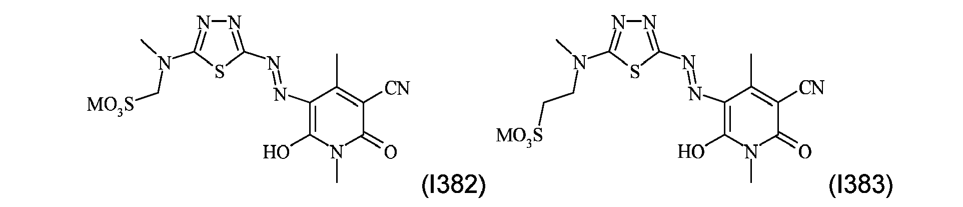 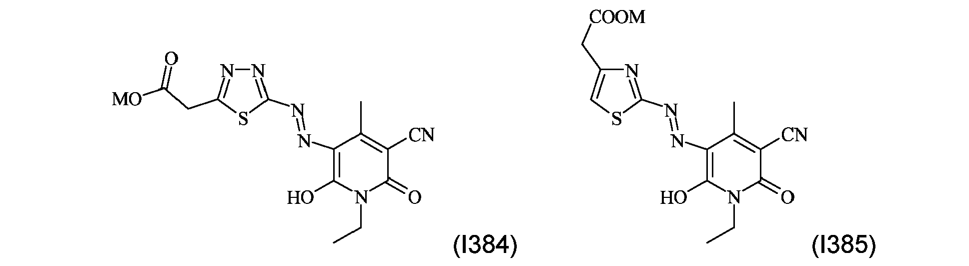 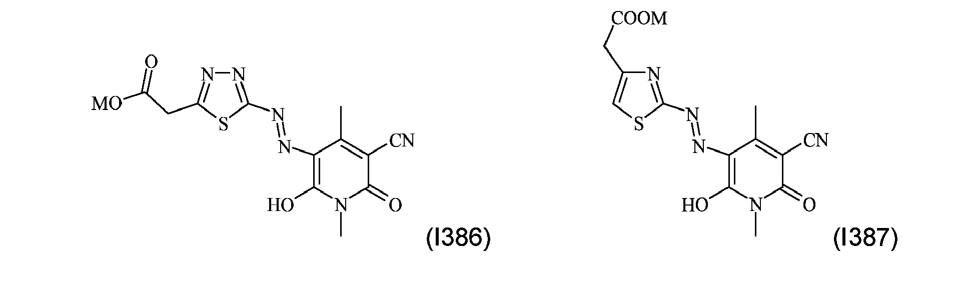 wherein M is hydrogen sodium, potassium, lithium or ammonium.

The dyestuffs of the formula (I) can be obtained by generally known azocoupling reactions, for instance by diazotization of a compound of the formula (II) 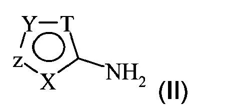 wherein T, X, Y and Z are defined as given above and coupling of the resulting diazo component with a compound of the formula (III¹), (III²), (III³), (III⁴) or (III⁵) 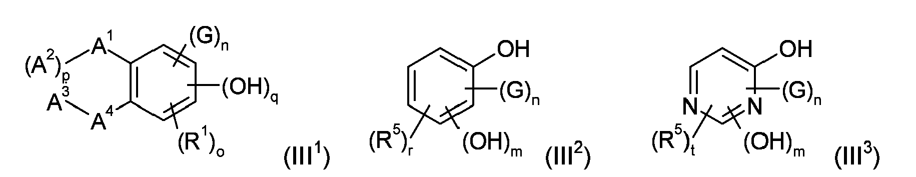 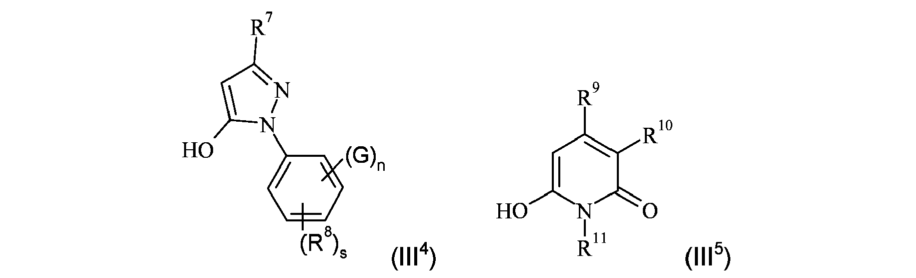 wherein A¹, A², A³, A⁴, G, R¹, R⁵, R⁷, R⁸, Rd⁹, R¹⁰, R¹¹, m, n, o, p, r, s and t are defined as given above.
The diazotization can preferably be carried out with nitrosylsulphuric acid or sodium nitrite in inorganic or organic acids or mixtures thereof such as hydrochloric acid, 85% phosphoric acid, mixtures of 85% phosphoric acid and acetic acid or sulphuric acid or a mixture of acetic and propionic acid. The coupling sequence is likewise carried out in a known manner, for example allowing the acid diazonium salt solution to enter the solution of the coupling component dissolved in aqueous, aqueous-organic or pure organic media such as aqueous-alcohols or pure alcohols media. In some cases it is of advantage to carry out the coupling sequence with the coupling compounds dissolved in aqueous-alcohols media. The optimum pH value during the coupling reaction can be achieved and maintained by adding bases such as alkali hydroxides, sodium carbonate, sodium hydrogen carbonate, ammonia, calcium carbonate, magnesium oxide, sodium acetate, sodium formiate or sodium phosphate.

The dyes of the formula (I) can be used alone, in mixtures with each other or in mixtures with other direct or precursors forming oxidation dyestuffs. Accordingly, the present invention also refers to dyestuff mixtures comprising one or more dyestuffs of the formula (I) and one or more direct dyestuffs or precursors of oxidation dyestuffs.

Preferred direct (neutral, acidic or basic) dyes are chosen from the group consisting of nitro dyes, natural direct dyes, azo dyes, anthraquinone dyes, triphenylmethane dyes, oxychinophthalone dyes, cumarin dyes, naphthalimide dyes, flavone dyes, anthrapyridone dyes, chinacridone dyes, xanthen dyes, thioxanthen dyes, benzoxanthen dyes, benzothioxanthen dyes, perylen dyes, perinone dyes, acridone dyes, phthalocyanine dyes, methine dyes, methine type cationic dyes having a cyanine structure, diketopyrrolopyrrole dyes, triphendioxazine dyes, naphthostyril dyes, phenoxazine dyes, phenothiazine dyes or metal complex compounds thereof. Especially preferred direct dyes include dyes from the publicly available dyestuff list issued by The European Cosmetic, Toiletry and Perfumery Association (COLIPA) and especially the dyestuffs C.I. Acid Yellow 1, C.I. Disperse Red 17, C.I. Basic Brown 17, C.I. Acid Black I, 4-Nitro-o-phenylenediamine, Picramic acid, HC Red 13, N,N-bis(2-hyrdoxyethyl)-2-nitro-p-phenylenediamine, HC Red 7, HC Blue 2, HC Yellow 4, HC Yellow 2, HC Orange 1, HC Red 1, HC Red 3, 4-Amino-3-nitrophenol, 2-Hydroxyethylamino-5-nitroanisole, 3-Nitro-p-hydroxyethylaminophenol, 3-Methylamino-4-nitrophenoxytheanol, 2-nitro-5-glyceryl methylaniline, HC Violet 1, HC Orange 2, HC Yellow 9, 4-Nitrophenylaminoethylurea, HC Red 10, HC Red 11, 2-Hydroxy-ethyl picramic acid, HC Blue 12, Hydroxyethyl-2-nitro-p-toluidine, HC Blue 11, HC Yellow 7, HC Yellow 10, 4-Amino-2-nitrophenylamine-2-carboxylic acid, 2-Chloro-6-ethylamino-4-nitrophenol, HC Violet 2, 2-Amino-6-chloro-4-nitrophenol, 4-Hydroxpropylamino-3-nitrophenol, HC Yellow 13, 2,6-Diamino-3-((pyridin-3-yl)azo)pyridine, N-(2-nitro-4-aminophenyl)-allylamine, Basic Violet 2, Basic Red 51, Basic Yellow 87, Basic Orange 31, Basic Red 76, Basic Brown 16, Basic Yellow 57, Acid Orange 7, Acid Red 33, Acid Yellow 23, Acid Blue 9, Acid Red 92, Acid Yellow 3, Acid Violet 43, Disperse Violet 1, Acid Blue 62, Disperse Black 9, Hydroxyanthraquinoneaminopropylmethyl morpholinium methosulfate, Lawsone, HC Blue 14, Curry Red, C.I. Acid Red 18, C.I. Acid Red 52, C.I. Acid Green 25, C.I. Disperse Blue 377, C.I. Pigment Red 57, HC Blue 15, Tetrabromophenol Blue, C.I.

Basic Blue 7, C.I. Basic Blue 26, C.I. Basic Blue 99, C.I. Basic Violet 10 and C.I. Basic Violet 14.

Preferred oxidation dye precursors are developers and couplers or self-coupling precursors which are ordinarily employed for an oxidation hair dye. Examples of the developer include paraphenylenediamine, toluene-2,5-diamine, 2-chloro-paraphenylenediamine, N-methoxyethyl-paraphenylenediamine, N,N-bis(2-hydroxyethyl)-paraphenylenediamine, 2-(2-hydroxyethyl)-paraphenylenediamine, 2,6-dimethyl-para-phenylenediamine, 4,4'-diaminodiphenylamine, 1,3-bis(N-(2-hydroxyethyl)-N-(4-aminophenyl)amino)-2-propanol, PEG-3,2,2'-paraphenylenediamine, paraaminophenol, paramethylaminophenol, 3-methyl-4-aminophenol, 2-aminomethyl-4-aminophenol, 2-(2-hydroxyethylaminomethyl)-4-aminophenol, orthoaminophenol, 2-amino-5-methylphenol, 2-amino-6-methylphenol, 4-amino-2-fluorophenol, 2-amino-5-acetamidophenol, 2,5-diaminopyridine, 3,4-diaminobenzoic acid, 5-aminosalicylic acid, 2,4,5,6-tetraaminopyrimidine, 2,5,6-triamino-4-hydroxypyrimidine and 4,5-diamino-1-(4'-chlorobenzyl)pyrazole and salts thereof.

Examples of coupler compounds include metaphenylenediamine, 2,4-diaminophenoxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, and 2,6-diaminopyridine and salts thereof.
Examples of self-coupling compounds include 2-amino-5-methylphenol and 2-amino-6-methylphenol.

The inventive dyestuff mixtures usually contain one or more dyestuffs of the formula (I) in an amount 90 to 10 wt.% and one or more direct dyestuff or precursors of oxidation dyestuff in an amount of 10 to 90 wt.% based on the total weight of the dyestuff mixture.
Preferably, the inventive dyestuff mixtures contain one ore more dyestuffs of the formula (I) in an amount 80 to 20 wt.% and one or more direct dyestuff or precursors of oxidation dyestuff in an amount of 20 to 80 wt.% based on the total weight of the dyestuff mixture.
Especially preferably, the inventive dyestuff mixtures contain one ore more dyestuffs of the formula (I) in an amount 65 to 35 wt.% and one or more direct dyestuff or precursors of oxidation dyestuff in an amount of 35 to 65 wt.% based on the total weight of the dyestuff mixture.

The dyestuffs of the general formula (I), as well as dyestuff mixtures comprising one or more dyestuffs of the formula (I) and one or more direct dyestuff or prescursors of oxidation dyestuff can advantageously be used to dye hair, especially human hair. They show a high tinctorial strength and partially strong fluorescence providing very vivid hair dyeing also at very low concentrations, e.g. 0.1 %. Also, they provide enhanced stability of the hair dyeing towards pH changes.
Further, use of an oxidation dye in combination with an inventive dyestuff of the formula (I) enables considerably vivid and intense dyeing which cannot be accomplished by the single use of the oxidation dye.

Accordingly, the present invention also refers to a process for coloring hair characterized in that one or more dyestuffs of the formula (I) or a dyestuff mixture comprising one or more dyestuffs of the formula (I) and one or more direct or oxidation dyestuff is applied to hair. Typically, the dyestuff is left on the hair for 1 to 45 minutes and is then rinsed off.

Preferably, the inventive dyestuffs of the formula (I) or the mixtures as defined are applied to hair in form of a hair dye composition.

The present invention thus also refers to hair dye compositions comprising one or more dyestuffs of the formula (I) 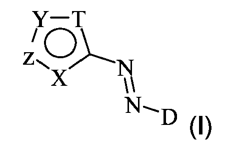 and its tautomeric forms, wherein D is a group of the formula D¹, D², D³, D⁴ or D⁵ 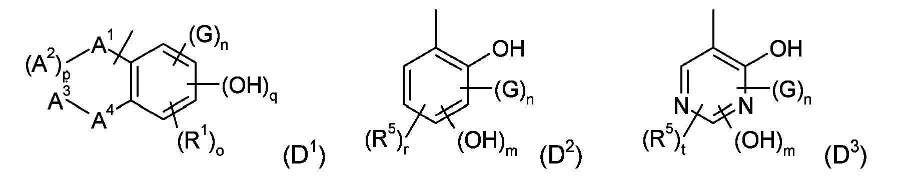 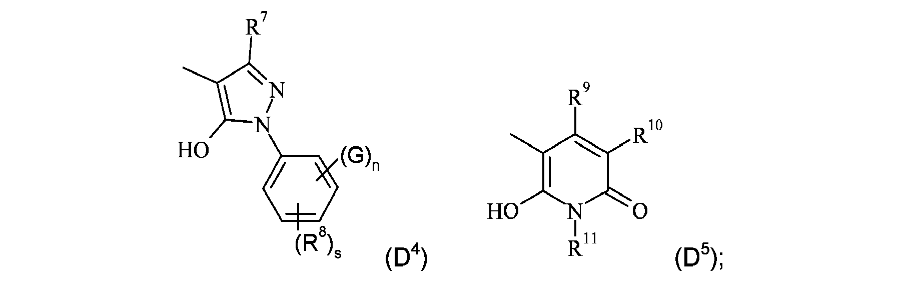 X is oxygen, sulphur, nitrogen, -N(R¹²)- or -C(R¹³)-;
Y is oxygen, sulphur, -C(R¹⁴)-, nitrogen or -N(R¹⁵)-;
Z is nitrogen or -C(R¹⁶)-;
T is nitrogen, sulphur or -C(R¹⁷)-; with the proviso that at least two of the groups X, Y, Z and T are heteroatoms; each of A¹, A², A³ and A⁴, independently, is oxygen, nitrogen, -N(R¹⁸)-, -C(R¹⁹)=, -C(R²⁰)(R²¹)- or -C(=R²²)-;
G is -SO₃M or -COOM;
M is hydrogen or a cosmetically acceptable, water solubilizing inorganic or organic cation;
n is an integer from 0 to 4, with the proviso, that when n is 0, at least one of the
groups X, Y Z and T carries at least one group G;
m is an integer from 0 to 3;
p is an integer from 0 to 1;
q is an integer from 1 to 4;
r is an integer from 0 to 4;
s is an integer from 0 to 3;
t is an integer from 0 to 2;
o is an integer from 1 to 3;
wherein in the group of the formula D² m+n+r is from 0 to 4 and in the group of the formula D³ m+n+r is from 0 to 2;
each of R¹¹, R¹², R¹⁵, R¹⁸ and R²³, independently, is hydrogen; alkyl; arylalkyl; alkoxyalkyl; alkylthioalkyl; poly(oxyalkylene)alkyl; N-acyl-aminoalkyl; N-alkylsulfonyl-aminoalkyl; aminothiocarbonyl-aminoalkyl; aminocarbonyl-aminoalkyl; alkoxyalkylaminoalkyl; alkylthioalkyl-aminoalkyl; aminoalkyloxyalkyl; N-monoalkylamino-alkyloxyalkyl; N,N-dialkyl-aminoalkoxyalkyl; N-arylamino-alkoxyalkyl; N,N-diarylamino-alkoxyalkyl; N-alkyl-N-aryl-amino-alkoxyalkyl; aminoalkylthioalkyl; N-monoalkylamino-alkylthioalkyl; N,N-dialkylamino-alkylthioalkyl; N-arylamino-alkylthioalkyl; N,N-diarylamino-alkylthioalkyl; N-alkyl-N-aryl-amino-alkylthioalkyl; alkenyl; alkynyl; cycloalkyl; cycloalkylalkyl; aryl; aryloxyalkyl; arylthioalkyl; heteroaryl; heteroarylalkyl; heterocycloalkyl; heterocycloalkylalkyl; acyl; benzoyl; alkoxycarbonyl; aryloxycarbonyl; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; acylthio; alkylsulfonyl or arylsulfonyl; or is alkyl substituted by one or more substituents selected from the group consisting of G, hydroxy, amino, N-monoalkyl-amino, N,N-dialkyl-amino, N-monoaryl-amino, N,N-diaryl-amino, N-alkyl-N-aryl-amino, halogen, cyano, nitro, alkoxycarbonyl, alkoxythiocarbonyl; acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoaryl-carbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl and N-monoalkyl-N-monoarylsulfamoyl; each of R¹, R⁵, R⁷, R⁸, R⁹, R¹⁰, R¹³, R¹⁴, R¹⁶, R¹⁷, R¹⁹, R²⁰ and R²¹, independently, is hydrogen; alkyl; trifluoromethyl; nitro, amino, N-acylamino, N-alkylsulfonylamino, aminocarbonyl-amino, aminothiocarbonylamino, N-monoalkyl-amino, N-monoaryl-amino, N,N-dialkyl-amino, N,N-diaryl-amino, N-alkyl-N-aryl-amino, alkoxyalkylamino, alkylthioalkylamino, aminoalkyloxy, N-monoalkylamino-alkyloxy, N,N-dialkyl-aminoalkoxy, N-arylamino-alkoxy, N,N-diarylaminoalkoxy, N-alkyl-N-aryl-amino-alkoxy, aminoalkylthio, N-monoalkylamino-alkylthio, N,N-dialkylamino-alkylthio, N-arylamino-alkylthio, N,N-diarylaminoalkylthio and N-alkyl-N-aryl-amino-alkylthio; alkenyl; alkynyl; cycloalkyl; cycloalkylalkyl; aryl; aryloxyalkyl; arylthioalkyl; heteroaryl; heteroarylalkyl; heterocycloalkyl; heterocycloalkylalkyl; halogen; cyano; nitro; alkoxy; aryloxy; formyl; iminomethyl; N-alkyl-iminomethyl; N-cycloalkyl-iminomethyl; N-aryl-iminomethyl; hydroxyiminomethyl; thioacyl; arylcarbonyl, arylthiocarbonyl; acyloxy; arylcarbonyloxy; acylamino; N-alkyl-acylamino; N-cycloalkyl-acylamino; N-arylacylamino; alkylsulfonylamino; cycloalkylsulfonylamino, N-alkyl-alkylsulfonylamino; N-alkyl-cycloalkylsulfonylamino; N-cycloalkyl-alkylsulfonylamino; N-cycloalkyl-cycloalkyl-sulfonylamino; N-arylalkylsulfonylamino; N-arylcycloalkylsulfonylamino; arylsulfonylamino; N-alkyl-arylsulfonylamino; N-cycloalkyl-arylsulfonylamino; N-arylarylsulfonylamino; arylcarbonylamino; arylthiocarbonylamino; N-alkyl-arylthiocarbonylamino; N-alkyl- arylcarbonylamino; N-cycloalkyl-arylthiocarbonylamino; N-cycloalkyl-arylcarbonylamino; N-aryl-arylthiocarbonylamino; N-aryl-arylcarbonylamino; carbamoyl; N-monocycloalkyl-carbamoyl; N-monoalkyl-carbamoyl; N,N-dicycloalkyl-carbamoyl; N,N-dialkyl-carbamoyl; N-monoaryl-carbamoyl; N,N-diaryl-carbamoyl; N-monocycloalkyl-N-monoarylcarbamoyl; N-monoalkyl-N-monoarylcarbamoyl; alkoxycarbonyl; aryloxycarbonyl; amino; monocycloalkyl-amino; monoalkyl-amino; di(cyclo)alkyl-amino; dialkyl-amino; monoaryl-amino; diaryl-amino; monocycloalkylmonoarylamino; monoalkylmonoarylamino; aminothiocarbonylamino; aminocarbonylamino; N-N-dicycloalkyl-aminothiocarbonylamino; N-N-dialkyl-aminothiocarbonylamino; N-N-dicycloalkyl-aminocarbonylamino; N-N-dialkyl-aminocarbonylamino; N,N-diaryl-aminothiocarbonylamino; N,N-diaryl-aminocarbonylamino; N,N-dialkylaminothio-carbonyl-N-alkylamino; N,N-dialkylaminocarbonyl-N-alkylamino; alkoxycarbonylamino; aryloxycarbonylamino; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; aminosulfonylamino; N-monocycloalkyl-aminosulfonylamino; N-monoalkyl-aminosulfonylamino; N,N-dicycloalkyl-aminosulfonylamino; N,N-dialkyl-aminosulfonylamino; N-monoaryl-aminosulfonylamino; N,N-diaryl-aminosulfonylamino; N-monocycloalkyl-N-monoarylaminosulfonyl-amino; N-monoalkyl-N-monoarylaminosulfonyl-amino; -S-G; alkylthio; arylthio; alkylsulfonyl; arylsulfonyl;
or is alkyl or alkylsulfonyl substituted by one or more substituents selected from the group consisting of hydroxy, aryl, alkoxy, alkylthio, poly(oxyalkylene), G, halogen, cyano, alkoxycarbonyl, alkoxythiocarbonyl, acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoarylcarbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl and N-monoalkyl-N-monoarylsulfamoyl;
and wherein the substituents mentioned above can themselves be substituted by G; and R⁷, R⁹, R¹⁹, R¹³, R¹⁴, R¹⁶ and R¹⁷ in addition can represent G;
R²² is oxygen, sulphur or -NR²³-;
wherein when X denotes -C-R¹³ and Z denotes -C-R¹⁶, or Y denotes -C-R¹⁴ and Z denotes -C-R¹⁶, or Y denotes -C-R¹⁴ and T denotes -C-R¹⁷, the groups R¹³ and R¹⁶ and R¹⁴ and R¹⁶ and R¹⁴ and R¹⁷, respectively, together with the carbon atoms to which they are bonded can form a 3- to 8 membered carbocyclic or heterocylic ring, which is saturated, unsaturated or partially unsaturated, and which is unsubstituted or substituted by one or more substituents selected from the group consisting of the substituents for R¹, R², R³, R⁴, R⁵, R⁷, R⁹, R¹⁰, R¹³, R¹⁴, R¹⁶, R¹⁷, R¹⁹, R²⁰ and R²¹ given above.
Such inventive hair dye compositions usually contain one or more dyestuffs of the formula (I) in amounts of 0.0001 to 20 wt.%, preferably 0.001 to 20 wt.%, more preferably from 0.01 to 10 wt.% and especially preferably from 0.05 to 5 wt.%, based on the weight of the hair dye composition.

In a further embodiment of the present invention, the hair dye compositions comprise in addition to one or more dyestuffs of the formula (I) one or more direct dyestuffs or precursors of oxidation dyestuffs
In the latter case the inventive hair dye compositions usually have a total dyestuff content between 0.001 to 20 wt.%, preferably from 0.01 to 20 wt.%, more preferably from 0.05 to 10 wt.% and especially preferably from 0.1 to 5 wt.%, based on the weight of the hair dye composition.

Preferred hair dye compositions comprise preferred or especially preferred dyestuffs of the formula (I) as defined above.

In the hair dye compositions according to the present invention, the inventive dyestuffs of the formula (I) exhibit a high storage stability within a wide pH range from 2 to 11, which is a pH range ordinarily employed for hair dyes, so that the hair dye compositions of the present invention can be used at any pH in the above-described pH range. Use in a pH range of from 2 or greater is however preferred from the viewpoint of dyeing property. Hair dye compositions of a pH range of 2 to 8 and hair dye compositions of a pH range of 8 to 11 are preferred.

The desired pH value is usually adjusted using an alkali agent. Examples of alkali agents include ammonia, alkanolamines such as monoethanolamine and isopropanolamine or salts thereof, guanidium salts such as guanidine carbonate and hydroxides such as sodium hydroxide. The inventive hair dye compositions contain an alkali agent preferably in an amount of from 0.01 to 20 wt.%, more preferably from 0.1 to 10 wt.%, especially preferably from 0.5 to 5 wt.% based on the total weight of the composition.

The inventive hair dye compositions may also be applied to hair together with an oxidizing agent. In other words, it can be provided as a two-part composition composed of a first part containing the dyestuff of the (I) and a second part containing an oxidizing agent. In this case, hair dyeing and bleaching can be carried out simultaneously, which facilitates more vivid hair dyeing.

Examples of oxidizing agent include hydrogen peroxide, persulfates such as ammonium persulfate, potassium persulfate and sodium persulfate, perborates such as sodium perborate, percarbonates such as sodium percarbonate and bromates such as sodium bromate and potassium bromate. Hydrogen peroxide is especially preferred from the viewpoints of hair bleaching property, stability and effectiveness of the inventive dyestuffs of the formulae (I). Hydrogen peroxide may be used in combination with another oxidizing agent. The oxidizing agent is preferably used in an amount of from 0.5 to 10 wt.%, especially preferably from 1 to 8 wt.%, based on the hair dye composition.
The first part containing the inventive dyestuff of the formulae (I) and the second part containing the oxidizing agent are mixed at a volume ratio preferably ranging from 2:1 to 1:3.

The hair dye compositions of the present invention may also comprise an autoxidation dye typified by an indole or an indoline, or a disperse dye.

Preferred hair dye compositions of the present invention further comprise cosmetically acceptable additives, like for example a polyol, polyol alkyl ether, anionic, cationic or amphoteric natural or synthetic polymer and/or silicone. Such compositions can dye the hair uniformly and provide improved cosmetic effects of the hair.
They may also comprise further additives which are ordinarily employed as a raw material for cosmetics. Examples of such optional components include hydrocarbons, animal or vegetable fats and oils, higher fatty acids, organic solvents, penetration promoters, anionic surfactants, natural or synthetic polymers, higher alcohols, ethers, amphoteric surfactants, nonionic surfactants, cationic surfactants, protein derivatives, amino acids, antiseptics, chelating agents, stabilizers, antioxidants, plant extracts, crude drug extracts, vitamins, colorants, perfumes and ultraviolet absorbers or further light stability enhancing agents.

The hair dye composition of the present invention can be prepared in a conventional manner to form a one-part composition or a two-part composition having a first part containing an alkali agent and a second part containing an oxidizing agent. The inventive dyestuff of the formula (I) may be incorporated in at least one of these parts of the two-part or three-part composition. When the hair dye composition of the present invention is of the one-part type, it is applied to the hair directly, while when it is of the two- or three-part type, these parts are mixed just before hair dyeing and the mixture is applied to the hair.
In the case of preparation of a two-part type hair dye composition, the first part is typically prepared by mixing the inventive dyestuff of the formulae (I) and optionally an oxidation dye and adjusting the pH of the mixture to 8 to 12 with an alkali agent such as ammonia. The second part is prepared by incorporating about 2 to 6 wt.% of hydrogen peroxide, adjusting the mixture to weakly acidic with phosphoric acid.
The hair dye composition of the present invention can be provided in the form of powder, transparent liquid, emulsion, cream, gel, paste, aerosol, aerosol foam or the like. It preferably has a viscosity of 2000 to 100000 mPa·s upon its application to the hair (after mixing of all the parts when the composition is a two-part or three-part type). The above-described viscosity is measured at 20°C by using a Brookfield rotary viscometer (No. 5 spindle, 5 rpm).

In the following, embodiments of the invention are described in detail for the purpose of illustrating only and not for the purpose of limiting the same.

### Example 1

5-(5-acetyl-4-methyl-thiazol-2-ylazo)-4-hydroxy-2-propenyl-3-vinyl-benzenesulfonic acid (dyestuff I1) 3.5 parts of 1-(2-amino-4-methyl-thiazol-5-yl)-ethanone were dissolved in a mixture of 30 parts of acetic acid 90% and 7 parts of propionic acid. After cooling at 0-5°C 3.7 parts of nitrosylsulphuric acid 37.5 % were gradually added during 1 hour and the resulted reaction mixture was stirred for further 4 hours at the same temperature. 5 parts of 4-hydroxy-naphthalene-1-sulfonic acid were dissolved in a mixture of 30 parts of water and 30 parts of methanol by adjusting the pH with sodium hydroxide solution. To this solution was gradually added at 0-5°C the diazo solution prepared before during 30 min. After stirring under cooling for 1 hour and at room temperature for another 1 hour, 250 parts of water were added and the pH was adjusted to 2.3 with hydrochloric acid. The precipitate was filtered off and purified by re-slurrying it in 200 parts of water/ice. Upon drying 4.5 parts of the dyestuff of the formula (I1) were obtained. The analytic data are consistent with the assigned structure for dye (I1). ¹H NMR (500 MHz, DMSO-d6): δ = 2.46 (3H), 2.55 (3H), 6.64 (1 H), 7.62 (1 H), 7.78 (1 H), 8.04 (1 H), 8.18 (1 H), 8.38 (1 H).

### Example 2

3-(5-acetyl-4-methyl-thiazol-2-ylazo)-4-hydroxy-naphthalene-2-sulfonic acid (dyestuff 12) 3.5 parts of 1-(2-amino-4-methyl-thiazol-5-yl)-ethanone were dissolved in a mixture of 30 parts of acetic acid 90% and 7 parts of propionic acid. After cooling at 0-5°C 3.7 parts of nitrosylsulphuric acid 37.5 % were gradually added during 1 hour and the resulted reaction mixture was stirred for further 3 hours at the same temperature. 5.6 parts of 3-hydroxy-naphthalene-1-sulfonic acid were dissolved in a mixture of 30 parts of water and 30 parts of methanol by adjusting the pH with sodium hydroxide solution. To this solution was gradually added at 0-5°C the diazo solution prepared before during 30 min. After stirring under cooling for 1 hour and at room temperature for another 1 hour, 120 parts of water were added and the pH was adjusted to 1.2 with hydrochloric acid. The precipitate was filtered off and washed with water. Upon drying 5.3 parts of the dyestuff of the formula (12) were obtained. The analytic data are consistent with the assigned structure for dye (12). ¹H NMR (500 MHz, DMSO-d6): δ = 2.48 (3H), 2.57 (3H), 7.08 (1 H), 7.62 (1 H), 7.82 (1 H), 7.95 (1 H), 8.34 (1 H), 14.50 (1 H).

### Example 3

(5-ethylsulfanyl-4-methyl-thiazol-2-ylazo)-4-hydroxy-naphthalene-1-sulfonic acid (dyestuff 13) 1 part of 5-ethylsulfanyl-4-methyl-thiazol-2-ylamine was dissolved in a mixture of 8 parts of acetic acid 90% and 2 parts of propionic acid. After cooling at 0-5°C 2.2 parts of nitrosylsulphuric acid 37.5 % were gradually added during 1 hour and the resulted reaction mixture was stirred for further 3 hours at the same temperature. 1.4 parts of 4-hydroxy-naphthalene-1-sulfonic acid were dissolved in a mixture of 10 parts of water and 10 parts of methanol by adjusting the pH with sodium hydroxide solution. To this solution was gradually added at 0-5°C the diazo solution prepared before during 30 min. After stirring under cooling for 1 hour and at room temperature for another 1 hour the pH was adjusted to 1.7 with hydrochloric acid. The resulted precipitate was filtered off and washed on the suction filter with 500 parts of water. Upon drying 1.2 parts of the dyestuff of the formula (13) were obtained. The analytic data are consistent with the assigned structure for dye (13). ¹H NMR (500 MHz, DMSO-d6): δ = 1.41 (3H), 3.35 (2H), 6.96 (1 H), 7.60 (1 H), 7.77 (1 H), 8.10 (1 H), 8.18 (1 H), 8.55 (1 H).

### Example 4

5-hydroxy-6-(thiazol-2-ylazo)-naphthalene-2-sulfonic acid (dyestuff 15) 2.1 parts of thiazol-2-ylamine were dissolved in a mixture of 25 parts of acetic acid 90% and 6.5 parts of propionic acid. After cooling at 0-5°C 6.7 parts of nitrosylsulphuric acid 37.5 % were gradually added during 1½ hours and the resulted reaction mixture was stirred for further 4 hours at the same temperature. 4.5 parts of 5-hydroxy-naphthalene-2-sulfonic acid were dissolved in a mixture of 30 parts of water and 30 parts of methanol by adjusting the pH with sodium hydroxide solution. To this solution was gradually added at 0-5°C the diazo solution prepared before during 30 min. After stirring under cooling for 1 hour and at room temperature for another 30 min the reaction mixture was poured into 100 parts of water and the pH was adjusted to 2.0 with hydrochloric acid. The resulted precipitate was filtered off and washed salt-free on the suction filter with water. Upon drying 4.9 parts of the dyestuff of the formula (15) were obtained. The analytic data are consistent with the assigned structure for dye (15). ¹H NMR (500 MHz, DMSO-d6): δ = 6.97 (1 H), 7.65 (1 H), 7.81 (1 H), 7.91 (1 H), 8.11 (1 H), 8.15 (1 H), 8.93 (1 H).

### Example 5

4-hydroxy-3-(thiazol-2-ylazo)-naphthalene-1-sulfonic acid (dyestuff 17) 2.1 parts of thiazol-2-ylamine were dissolved in a mixture of 25 parts of acetic acid 90% and 6.5 parts of propionic acid. After cooling at 0-5°C 6.7 parts of nitrosylsulphuric acid 37.5 % were gradually added during 1½ hours and the resulted reaction mixture was stirred for further 4 hours at the same temperature. 5.0 parts of 4-hydroxy-naphthalene-1-sulfonic acid were dissolved in a mixture of 30 parts of water and 30 parts of methanol by adjusting the pH with sodium hydroxide solution. To this solution was gradually added at 0-5°C the diazo solution prepared before during 30 min. After stirring under cooling for 1 hour and at room temperature for another 30 min the reaction mixture was poured in 1000 parts of water and the pH was adjusted to 2.0 with hydrochloric acid. The resulted precipitate was filtered off and washed salt-free on the suction filter with water. Upon drying 4.7 parts of the dyestuff of the formula (I7) were obtained. The analytic data are consistent with the assigned structure for dye (I7). ¹H NMR (500 MHz, DMSO-d6): δ = 6.96 (1 H), 7.58-7.60 (2H), 7.78 (1 H), 7.90 (1 H), 8.11 (1 H), 8.19 (1 H), 8.63 (1 H).

### Example 6

4-hydroxy-3-(5-methyl-thiazol-2-ylazo)-naphthalene-1-sulfonic acid (dyestuff I125) 5.8 parts of 5-methyl-thiazol-2-ylamine were dissolved in a mixture of 55 parts of acetic acid 90% and 15 parts of propionic acid. After cooling at 0-5°C 17 parts of nitrosylsulphuric acid 37.5 % were gradually added during 1½ hours and the resulted reaction mixture was stirred for further 4 hours at 5-10°C. 11.2 parts of 4-hydroxynaphthalene-1-sulfonic acid were dissolved in a mixture of 75 parts of water and 75 parts of methanol by adjusting the pH with sodium hydroxide solution. To this solution was gradually added at 0-5°C the diazo solution prepared before during 30 min. After stirring under cooling for 1 hour and at room temperature for another 30 min the reaction mixture was diluted with 200 parts of water and the pH was adjusted to 2.5 with hydrochloric acid. The resulted precipitate was filtered off and purified by re-slurrying it in 1500 parts of water. Upon drying 6.7 parts of the dyestuff of the formula (1125) were obtained. The analytic data are consistent with the assigned structure for dye (I125). ¹H NMR (500 MHz, DMSO-d6): δ = 2.47 (3H), 6.96 (1 H), 7.58 (1 H), 7.65 (1 H), 7.74 (1 H), 8.11 (1 H), 8.19 (1 H), 8.65 (1 H).

### Example 7

4-hydroxy-3-(3-methyl-[1,2,4]thiadiazol-5-ylazo)-naphthalene-1-sulfonic acid (dyestuff 18) 3.0 parts of 3-methyl-[1,2,4]thiadiazol-5-ylamine were dissolved in a mixture of 22 parts of acetic acid 90% and 5.6 parts of propionic acid. After cooling at 0-5°C 8.5 parts of nitrosylsulphuric acid 37.5 % were gradually added during 1 hour and the resulted reaction mixture was stirred for further 5 hours at 0-5°C. 6.3 parts of 4-hydroxy-naphthalene-1-sulfonic acid were dissolved in a mixture of 37.5 parts of water and 37.5 parts of methanol by adjusting the pH with sodium hydroxide solution. To this solution was gradually added at 0-5°C the diazo solution prepared before during 30 min. After stirring under cooling for 1 hour and at room temperature for another 30 min the reaction mixture was poured in 1000 parts of water and the pH was adjusted to 2.2 with hydrochloric acid. The resulted precipitate was filtered off and washed salt-free on the suction filter with water. Upon drying 2.0 parts of the dyestuff of the formula (18) were obtained. The analytic data are consistent with the assigned structure for dye (I8). ¹H NMR (500 MHz, DMSO-d6): δ = 2.39 (3H), 6.70 (1 H), 7.60 (1 H), 7.74 (1 H), 8.00 (1 H), 8.17 (1 H), 8.21 (1 H).

### Example 8

5-hydroxy-6-(5-methyl-thiazol-2-ylazo)-naphthalene-2-sulfonic acid (dyestuff I10) 4.1 parts of 5-methyl-thiazol-2-ylamine were dissolved in a mixture of 39 parts of acetic acid 90% and 10,5 parts of propionic acid. After cooling at 0-5°C 12 parts of nitrosylsulphuric acid 37.5 % was gradually added during 1½ hours and the resulted reaction mixture was stirred for further 4 hours at 5-10°C. 7.8 parts of 5-hydroxynaphthalene-2-sulfonic acid was dissolved in a mixture of 52.5 parts of water and 52.5 parts of methanol by adjusting the pH with sodium hydroxide solution. To this solution was gradually added at 0-5°C the diazo solution prepared before during 30 min. After stirring under cooling for 1 hour and at room temperature for another 30 min the reaction mixture was diluted with 200 parts of water and the pH was adjusted to 2.0 with hydrochloric acid. The resulted precipitate was filtered off and washed salt-free on the suction filter with water. Upon drying 4.5 parts of the dyestuff of the formula (110) were obtained. The analytic data are consistent with the assigned structure for dye (I10). ¹H NMR (500 MHz, DMSO-d6): δ = 2.50 (3H), 7.01 (1 H), 7.69 (1 H), 7.81 (1 H), 8.05 (1 H), 8.16 (1 H), 8.94 (1 H).

### Example 9

4-[5-hydroxy-3-methyl-4-(5-methyl-[1,3,4]thiadiazol-2-ylazo)-pyrazol-1-yl]-benzenesulfonic acid (dyestuff 1326) 29.1 parts of 5-methyl-[1,3,4]thiadiazol-2-ylamine were dissolved in a mixture of 175 parts of acetic acid 90% and 48 parts of propionic acid. After cooling at 0-5°C 85 parts of nitrosylsulphuric acid 37.5 % was gradually added during 3 hours and the resulted reaction mixture was stirred for another 1 hour at 0-5°C. 44.8 parts of 4-(5-hydroxy-3-methyl-pyrazol-1-yl)-benzenesulfonic acid was dissolved in 600 parts of water by adjusting the pH with sodium hydroxide solution. To this solution was gradually added at 0-5°C the diazo solution prepared before during 1 hour. After stirring under cooling for 1 hour and at room temperature for another 30 min the reaction mixture was diluted with water and desalt. Upon drying 47.0 parts of the dyestuff of the formula (1326) were obtained. The analytic data are consistent with the assigned structure for dye (I326). ¹H NMR (500 MHz, DMSO-d6): δ = 2.59 (3H), 2.23 (3H), 7.64 (2H), 7.87 (2H).

### Example 10

4-[4-(5-dimethylamino-[1,3,4]thiadiazol-2-ylazo)-5-hydroxy-3-methyl-pyrazol-1-yl]-benzenesulfonic acid (dyestuff 1299) 4.3 parts of N,N-dimethyl-[1,3,4]thiadiazole-2,5-diamine were dissolved in a mixture of 26 parts of acetic acid 90% and 6.7 parts of propionic acid. After cooling at 0-5°C 5.7 parts of nitrosylsulphuric acid 37.5 % was gradually added during 1 hour and the resulted reaction mixture was stirred for further 3 hours at 0-5°C. 7.5 parts of 4-(5-Hydroxy-3-methyl-pyrazol-1-yl)-benzenesulfonic acid was dissolved in a mixture of 50 parts of water and 10 parts of methanol by adjusting the pH with sodium acetate solution solution. To this solution was gradually added at 0-5°C the diazo solution prepared before during 1 hour. After stirring under cooling for 1½ hours and at room temperature for another 30 min the resulted precipitate was filtered off and purified by re-slurrying it in 100 parts of water. Upon drying 7.0 parts of the dyestuff of the formula (1299) were obtained. The analytic data are consistent with the assigned structure for dye (1299). ¹H NMR (500 MHz, DMSO-d6): δ = 2.29 (3H), 3.10 (6H), 7.62 (2H), 7.86 (2H).

### Example 11

2-{[5-(4-dimethylamino-2-hydroxy-phenylazo)-[1,3,4]thiadiazol-2-yl]-methyl-amino}-ethanesulfonic acid (dyestuff 1193) 5.2 parts of 2-[(5-amino-[1,3,4]thiadiazol-2-yl)-methyl-amino]-ethanesulfonic acid sodium salt was dissolved in a mixture of 26 parts of acetic acid 90% and 7.0 parts of propionic acid. After cooling at 0-5°C 3.6 parts of nitrosylsulphuric acid 37.5 % was gradually added during 1 hour and the resulted reaction mixture was stirred for further 2 hours at 0-5°C. 2.5 parts of 3-dimethylamino-phenol were dissolved in 35 parts of conc. hydrochloric acid. To this solution was gradually added at 0-5°C the diazo solution prepared before during 30 min. After stirring under cooling for 1 hour the reaction mixture was poured into 180 parts of cold water and the pH was adjusted to 3.0 with sodium hydroxide solution. The solution was concentrated in vacuum and the resulted precipitate was filtered off and washed salt-free on the suction filter with water. Upon drying 4.5 parts of the dyestuff of the formula (1193) were obtained. The analytic data are consistent with the assigned structure for dye (I193). ¹H NMR (500 MHz, DMSO-d6): δ = 2.79 (2H), 3.09 (6H), 3.15 (3H), 3.73 (2H), 6.20 (1 H), 6.51 (1 H), 7.51 (1 H), 11.66 (1 H).

### Example 12

3-(benzothiazol-2-ylazo)-4-hydroxy-naphthalene-1-sulfonic acid (dyestuff 1111) 5.0 parts of benzothiazol-2-ylamine were dissolved in a mixture of 41.5 parts of acetic acid 90% and 10.8 parts of propionic acid. After cooling at 0-5°C 6.25 parts of nitrosylsulphuric acid 37.5 % were gradually added during 45 min and the resulted reaction mixture was stirred for further 2½ hours at 0-5°C. 7.45 parts of 4-hydroxynaphthalene-1-sulfonic acid were dissolved in a mixture of 44 parts of methanol and 33 parts of water by adjusting the pH with sodium acetate solution. To this solution was gradually added at 0-5°C the diazo solution prepared before during 30 min. After stirring under cooling for 30 min and at room temperature for further 30 min the resulted precipitate was filtered off and washed salt-free. Upon drying 6.7 parts of the dyestuff of the formula (I111) were obtained. The analytic data are consistent with the assigned structure for dye (I111). ¹H NMR (500 MHz, DMSO-d6): δ = 6.71 (1 H), 7.23 (1 H), 7.38 (1 H), 7.45 (1 H), 7.60 (1 H), 7.75-7.81 (2H), 8.05 (1 H), 8.20 (1 H), 8.44 (1 H).

The remaining dyestuffs I1 to 1387 were synthesised in line with the procedures given in Examples 1 to 12.

Dyeing Examples
1. The dyestuffs of the formula (I) were dissolved into the following Base Formulation 1:

| | |
|---|---|
| Dyestuff of formula (I) | 0.5g |
| 2-Propanol | 5.0g |
| Ammonium Hydroxide (25%) | 5.0g |
| Water | up to 100g |
| pH | 10.2 |

Approximately 1.5 g of the Base Formulation I was applied to 1g of undamaged white goat hair at 30°C for 30 min. Afterwards the tresses were rinsed with lukewarm water, shampooed and dried and then recorded by measuring L, a, b and delta E values of the tresses using a hand held Datacolor Mercury color-measuring instrument. Delta E, which is a known measure for the chroma, was calculated according to the well-known equation: ΔE = (ΔL² + Δa² + Δb²)^{1/2}. For comparison, the tresses were recorded before coloration as well.
2. To verify the stability towards hydrogen peroxide as well as the color effect using an oxidizing agent the dyestuffs of the formula (I) were dissolved into the following Base Formulation 2, containing alkaline hydrogen peroxide:

| | |
|---|---|
| Dyestuff of formula (I) | 0.5g |
| iso-Propanol | 5.0g |
| Ammonium Hydroxide (25%) | 5.0g |
| Hydrogen Peroxide (50%) | 6.0g |
| Water | up to 100g |
| pH | 10.2 |

Approximately 1.5 g of the Base Formulation 2 was applied to 1g of undamaged white goat hair at 30°C for 30mins. Afterwards the tresses were rinsed with lukewarm water, shampooed, dried and then recorded as described above.
3. To check the wash fastness the colored tresses were shampooed in 20 cycles, dried and then recorded as above described. The dyes of the formula (I) show very good to excellent wash fastness.

The dyeing results are summarized in Table 1.

**Table 1: Dyeing effects on undamaged goat hair:**

| Dyestuff | Hair color | delta E in base formulation 1 | delta E in base formulation 2 | delta E color loss after 20 washing cycles |
|---|---|---|---|---|
| (16) | vivid violet | 49 | 43 | 10 |
| (17) | intense purple | 66 | 65 | 9 |
| (13) | brilliant violet | 59 | 59 | 8 |
| (15) | intense violet | 55 | 60 | 6 |
| (I1) | blue | 50 | 50 | 10 |
| (12) | blue | 46 | 48 | 8 |
| (I8) | very intense violet | 68 | 68 | 11 |
| (I10) | violet | 52 | 53 | 7 |
| (I125) | very intense bluish violet | 67 | 67 | 8 |
| (19) | violet | 46 | 46 | 12 |
| (1287) | brilliant yellow | 40 | 42 | 4 |
| (1294) | yellow | 40 | 42 | 10 |
| (1329) | yellow | 38 | 39 | 8 |
| (I111) | very intense bluish violet | 67 | 67 | 9 |
| (I13) | very intense reddish violet | 70 | 70 | 9 |
| (I17) | reddish violet | 51 | 51 | 10 |
| (118) | bluish violet | 50 | 50 | 10 |
| (1326) | yellow | 30 | 32 | 4 |
| (1299) | yellow | 32 | 32 | 5 |
| (I11) | reddish violet | 52 | 52 | 8 |

To investigate the dye ability in acidic media, the dyestuffs of the formulae (I) were dissolved into the following Base Formulation 3:

| | |
|---|---|
| Dyestuff of formula (I) | 0.4g |
| Propylen carbonate | 25.0g |
| Ethanol | 5.0g |
| Emulgator | 3.0 g |
| acidification agent | 5.0 g |
| Water | up to 100g |
| adjusted pH | 3.0 |

To check the wash fastness the colored tresses were shampooed in 20 cycles, dried and then recorded as above described. The dyeings performed in acidic media with the dyestuffs of formula (I) show very good to excellent wash fastness as well.

The dyeing results are summarized in Table 2.

| Dyestuff | Hair color | delta E in base formulation 3 | delta E color after 20washing cycles |
|---|---|---|---|
| (I357) | intensive fluorescent yellow | 59 | 59 |
| (I193) | intensive fluorescent pink | 79 | 79 |
| (I104) | reddish brown | 81 | 82 |
| (I171) | brownish red | 81 | 81 |
| (I80) | yellowish red | 85 | 87 |
| (182) | reddish brown | 78 | 81 |

## Claims

1. Dyestuff of the formula (I) 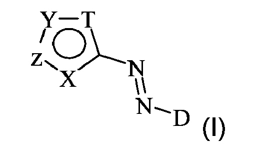 and its tautomeric forms, wherein D is a group of the formula D¹, D², D³, D⁴ or D⁵ 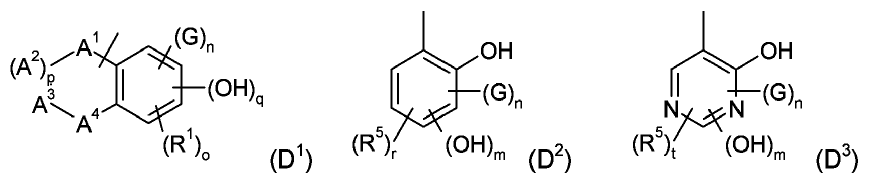 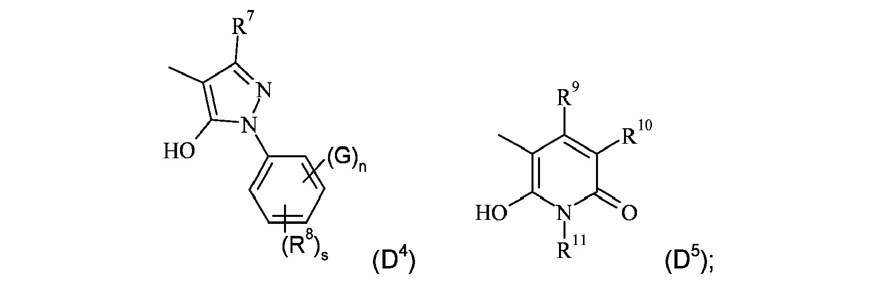 X is oxygen, sulphur, nitrogen, -N(R¹²)- or -C(R¹³)-;
Y is oxygen, sulphur, -C(R¹⁴)-, nitrogen or -N(R¹⁵)-;
Z is nitrogen or -C(R¹⁶)-;
T is nitrogen, sulphur or -C(R¹⁷)-; with the proviso that at least two of the groups X, Y, Z and T are heteroatoms; each of A¹, A², A³ and A⁴, independently, is oxygen, nitrogen, -N(R¹⁸)-, -C(R¹⁹)=, -C(R²⁰)(R²¹)- or -C(=R²²)-;
G is -SO₃M or -COOM;
M is hydrogen or a cosmetically acceptable, water solubilizing inorganic or organic cation;
n is an integer from 0 to 4, with the proviso, that when n is 0, at least one of the groups A¹, A², A³, A⁴, X, Y Z and T carries a group G;
m is an integer from 0 to 3;
p is an integer from 0 to 1;
q is an integer from 1 to 4;
r is an integer from 0 to 4;
s is an integer from 0 to 3;
t is an integer from 0 to 2;
o is an integer from 1 to 3;
wherein in the group of the formula D² m+n+r is from 0 to 4 and in the group of the formula D³ m+n+r is from 0 to 2; each of R¹¹, R¹², R¹⁵, R¹⁸ and R²³, independently, is hydrogen; alkyl; arylalkyl; alkoxyalkyl; alkylthioalkyl; poly(oxyalkylene)alkyl; N-acyl-aminoalkyl; N-alkylsulfonyl-aminoalkyl; aminothiocarbonyl-aminoalkyl; aminocarbonyl-aminoalkyl; alkoxyalkylaminoalkyl; alkylthioalkyl-aminoalkyl; aminoalkyloxyalkyl; N-monoalkylamino-alkyloxyalkyl; N,N-dialkyl-aminoalkoxyalkyl; N-arylamino-alkoxyalkyl; N,N-diarylamino-alkoxyalkyl; N-alkyl-N-aryl-amino-alkoxyalkyl; aminoalkylthioalkyl; N-monoalkylamino-alkylthioalkyl; N,N-dialkylamino-alkylthioalkyl; N-arylamino-alkylthioalkyl; N,N-diarylamino-alkylthioalkyl; N-alkyl-N-aryl-amino-alkylthioalkyl; alkenyl; alkynyl; cycloalkyl; cycloalkylalkyl; aryl; aryloxyalkyl; arylthioalkyl; heteroaryl; heteroarylalkyl; heterocycloalkyl; heterocycloalkylalkyl; acyl; benzoyl; alkoxycarbonyl; aryloxycarbonyl; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; acylthio; alkylsulfonyl or arylsulfonyl; or is alkyl substituted by one or more substituents selected from the group consisting of G, hydroxy, amino, N-monoalkyl-amino, N,N-dialkyl-amino, N-monoaryl-amino, N,N-diaryl-amino, N-alkyl-N-aryl-amino, halogen, cyano, nitro, alkoxycarbonyl, alkoxythiocarbonyl; acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoaryl-carbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl and N-monoalkyl-N-monoarylsulfamoyl; each of R¹, R⁵, R⁷, R⁸, R⁹, R¹⁰, R¹³, R¹⁴, R¹⁶, R¹⁷, R¹⁹, R²⁰ and R²¹, independently, is hydrogen; alkyl; trifluoromethyl; alkenyl; alkynyl; cycloalkyl; aryl; heteroaryl; heterocycloalkyl; halogen; cyano; nitro; alkoxy; aryloxy; formyl; iminomethyl; N-alkyl-iminomethyl; N-cycloalkyl-iminomethyl; N-aryl-iminomethyl; hydroxyiminomethyl; thioacyl; arylcarbonyl, arylthiocarbonyl; acyloxy; arylcarbonyloxy; acylamino; N-alkyl-acylamino; N-cycloalkyl-acylamino; N-arylacylamino; alkylsulfonylamino; cycloalkylsulfonylamino, N-alkyl-alkylsulfonylamino; N-alkyl-cycloalkylsulfonylamino; N-cycloalkyl-alkylsulfonylamino; N-cycloalkyl-cycloalkyl-sulfonylamino; N-arylalkylsulfonylamino; N-arylcycloalkylsulfonylamino; arylsulfonylamino; N-alkyl-arylsulfonylamino; N-cycloalkyl-arylsulfonylamino; N-arylarylsulfonylamino; arylcarbonylamino; arylthiocarbonylamino; N-alkyl- arylthiocarbonylamino; N-alkyl-arylcarbonylamino; N-cycloalkyl-arylthiocarbonylamino; N-cycloalkyl-arylcarbonylamino; N-aryl-arylthiocarbonylamino; N-aryl-arylcarbonylamino; carbamoyl; N-monocycloalkyl-carbamoyl; N-monoalkyl-carbamoyl; N,N-dicycloalkyl-carbamoyl; N,N-dialkyl-carbamoyl; N-monoaryl-carbamoyl; N,N-diaryl-carbamoyl; N-monocycloalkyl-N-monoarylcarbamoyl; N-monoalkyl-N-monoarylcarbamoyl; alkoxycarbonyl; aryloxycarbonyl; amino; monocycloalkyl-amino; monoalkyl-amino; di(cyclo)alkyl-amino; dialkyl-amino; monoaryl-amino; diaryl-amino; monocycloalkylmonoarylamino; monoalkylmonoarylamino; aminothiocarbonylamino; aminocarbonylamino; N-N-dicycloalkyl-aminothiocarbonylamino; N-N-dialkyl-aminothiocarbonylamino; N-N-dicycloalkyl-aminocarbonylamino; N-N-dialkyl-aminocarbonylamino; N,N-diaryl-aminothiocarbonylamino; N,N-diaryl-aminocarbonylamino; N,N-dialkylaminothio-carbonyl-N-alkylamino; N,N-dialkylaminocarbonyl-N-alkylamino; alkoxycarbonylamino; aryloxycarbonylamino; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; aminosulfonylamino; N-monocycloalkyl-aminosulfonylamino; N-monoalkyl-aminosulfonylamino; N,N-dicycloalkyl-aminosulfonylamino; N,N-dialkyl-aminosulfonylamino; N-monoaryl-aminosulfonylamino; N,N-diaryl-aminosulfonylamino; N-monocycloalkyl-N-monoarylaminosulfonyl-amino; N-monoalkyl-N-monoarylaminosulfonyl-amino; -S-G; alkylthio; arylthio; alkylsulfonyl; arylsulfonyl; or is alkyl, N-monoalkylamino, N-monocycloalkylamino, N,N-dialkylamino, N,N-dicycloalkylamino, N-alkyl-N-cycloalkylamino, N-alkyl-N-arylamino, N-aryl-N-cycloalkylamino, alkoxy, alkylthio or alkylsulfonyl substituted by one or more substituents selected from the group consisting of hydroxy, cycloalkyl, heteroaryl, heterocycloalkyl, aryl, aryloxy, alkoxy, alkylthio, arykthio. poly(oxyalkylene), G, halogen, cyano, alkoxycarbonyl, alkoxythiocarbonyl, acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoarylcarbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl, N-monoalkyl-N-monoarylsulfamoyl, nitro, amino, acylamino, arylcarbonylamino, alkylsulfonylamino, arylsulfonylamino, aminocarbonylamino, aminothiocarbonylamino, N-monoalkylamino, N-mono(hydroxyalkyl)amino, N,N-bis(hydroxyalkyl)amino, N-mono(alkoxyalkyl)amino, N,N-bis(alkoxyalkyl)amino, N-mono(alkoxyalkyl)amino, N-mono(alkylthioalkyl)amino, N-bis(alkylthioalkyl)amino, N-monocycloalkylamino, N-monoaryl-amino, N,N-dialkyl-amino, N,N-dicycloalkylamino, N,N-diaryl-amino, N-alkyl-N-aryl-amino, N-cycloalkyl-N-aryl-amino, aminosulfonylamino, N-monocycloalkyl-aminosulfonylamino, N-monoalkyl-aminosulfonylamino, N,N-dicycloalkyl-aminosulfonylamino, N,N-dialkylaminosulfonylamino, N-monoaryl- aminosulfonylamino, N,N-diaryl-aminosulfonylamino, N-monocycloalkyl-N-monoarylaminosulfonyl-amino, N-monoalkyl-N-monoarylaminosulfonyl-amino and -S-G; and wherein the substituents mentioned above can themselves be substituted by G; and
R⁷, R⁹, R¹⁹, R¹³, R¹⁴, R¹⁶ and R¹⁷ in addition can represent G; and
R¹⁹, R²⁰ and R²¹ in addition can represent hydroxyl;
R²² is oxygen, sulphur or -NR²³-;
wherein when X denotes -C-R¹³ and Z denotes -C-R¹⁶, or Y denotes -C-R¹⁴ and Z denotes -C-R¹⁶, or Y denotes -C-R¹⁴ and T denotes -C-R¹⁷, the groups R¹³ and R¹⁶ and R¹⁴ and R¹⁶ and R¹⁴ and R¹⁷, respectively, together with the carbon atoms to which they are bonded can form a 3- to 8 membered carbocyclic or heterocylic ring, which is saturated, unsaturated or partially unsaturated, and which is unsubstituted or substituted by one or more substituents selected from the group consisting of the substituents for R¹, R², R³, R⁴, R⁵, R⁷, R⁹, R¹⁰, R¹³, R¹⁴, R¹⁶, R¹⁷, R¹⁹, R²⁰ and R²¹ given above; and
wherein 3-hydroxy-4-(1H-imidazol-2-ylazo)-1-naphthalenesulfonic acid and 4-hydroxy-3-(1 H-imidazol-2-ylazo)-benzenesulfonic acid are excluded.

2. Dyestuff according to claim 1, which correspond to one of the formulae (Ia) to (Im) 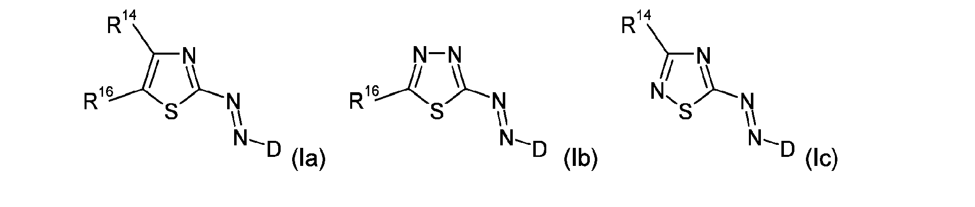 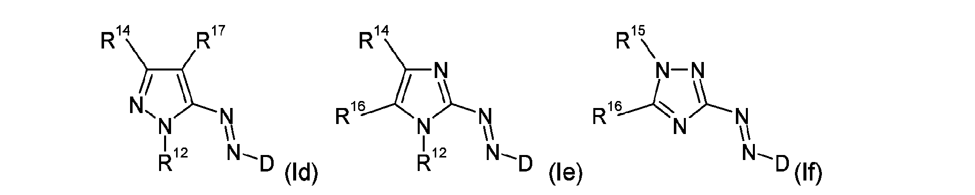 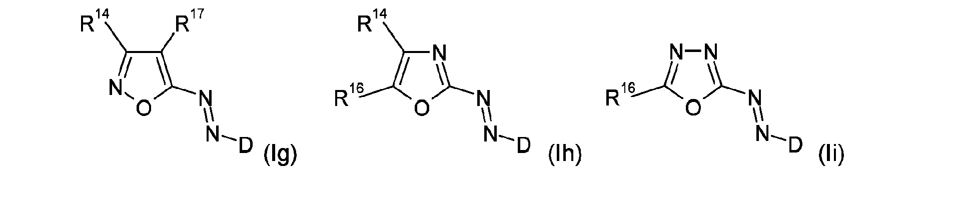 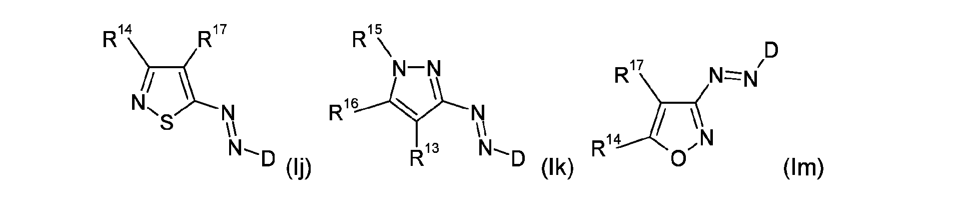 wherein R¹² to R¹⁷ are defined as given above.

3. Dyestuff according to claim 1 and/or 2 wherein D corresponds to a group of the formula (D¹a) to (D¹w) 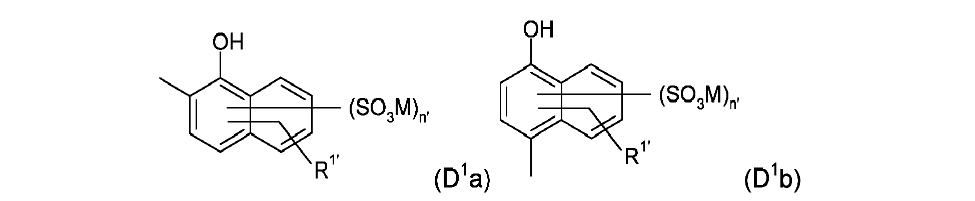 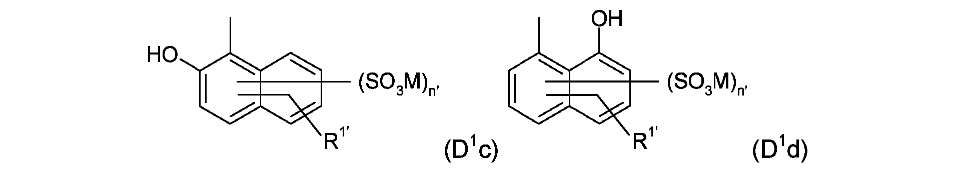 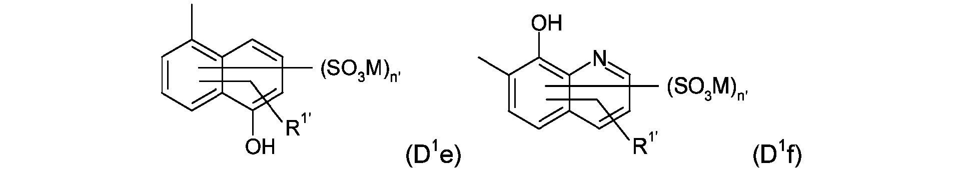 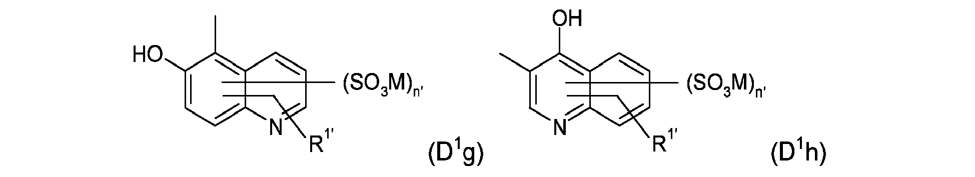 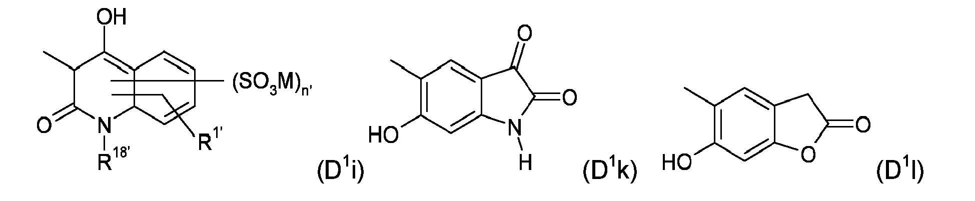 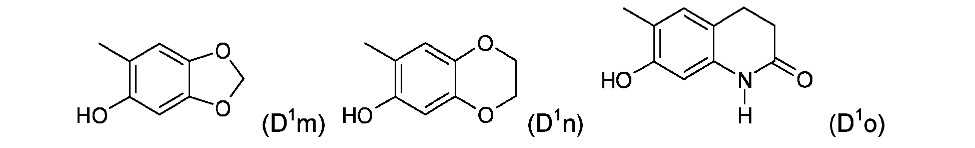 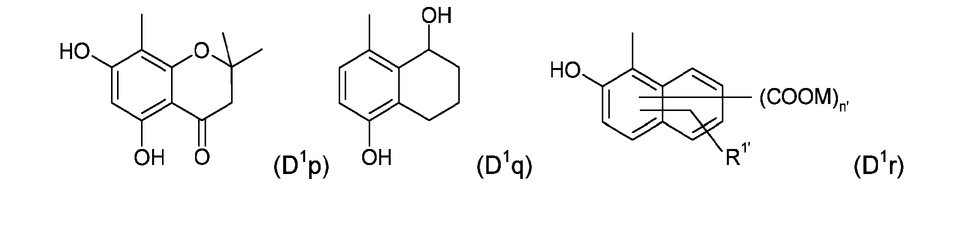 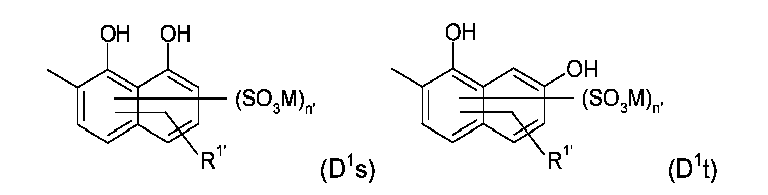 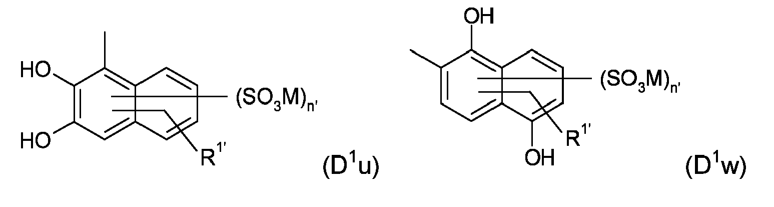 wherein
R^{1'} is hydrogen, (C₁-C₄)-alkoxy, amino, (C₁-C₄)-alkylcarbonyl-amino, benzoylamino, hydroxy, (C₁-C₄)-alkylamino or chlorine;
R^{18'} is hydrogen or (C₁-C₄)-alkyl;
M is defined as given above; and
n' is an integer from 0 to 2.

4. Dyestuff according to claim 1 and/or 2 wherein D corresponds to a group of the formula (D²a) to (D¹c) 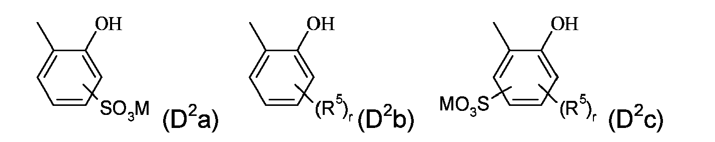 wherein
R⁵ is cyano, -COOM, (C₁-C₄)-alkyl, heteroaryl, heterocycloalkyl, (C₁-C₄)-alkoxy, N,N-di-(C₁-C₄)-alkyl-amino or N,N-di-(C₁-C₄)-alkyl-amino in which one or both alkyl groups
are substituted by hydroxy, cyano, (C₁-C₄)-alkoxycarbonyl, aminocarbonyl, (C₁-C₄)-alkylcarbonyloxy, -COOM, -SO₃M, carboxyl or sulfo.
r is an intteger form 1 to 3; and
M is defined as given above.

5. Dyestuff according to claim 1 and/or 2 wherein D corresponds to a group of the formula (D³a) 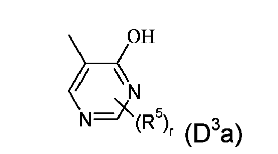 wherein
R⁵ is hydroxy, amino, chlorine, (C₁-C₄)-alkyl, (C₁-C₄)-alkoxy, (C₁-C₄)-thioalkoxy, phenyl, phenoxy or phenylamino; and r is 1 or 2.

6. Dyestuff according to claim 1 and/or 2 wherein D corresponds to a group of the formula (D⁴a) to (D⁴c) 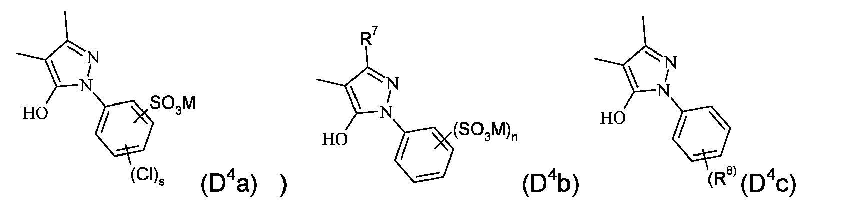 wherein
s is and integer from 0 to 2;
n is an integer from 1 to 2;
R⁷ is (C₁-C₄)-alkyl or -COOM;
R⁸ is acylamino, alkylsulfonylamino, dialkylaminosulfonylamino, N-(C₁-C₄)-monoalkyl-amino or (C₁-C₄)-alkylsukfonyl, whereby the alkyl group can be substituted by one or more substituents selected from the group consisting of hydroxyl, -SO₃M, N-(C₁-C₄)-monoalkyl-amino, N,N-di-(C₁-C₄)-alkyl-amino; and wherein the substituents themselves can be substituted by one or more substituents selected from the group consisting of hydroxyl and -SO₃M; and
M is defined as given above.

7. Dyestuff according to claim 1 and/or 2 wherein D corresponds to a group to a group of the formula (D⁵), wherein
R⁹ is (C₁-C₄)-alkyl or (C₁-C₄)-alkyl substituted by -COOM and is most preferably methyl; R¹⁰ is hydrogen, aminocarbonyl, -COOM or cyano;
R¹¹ is (C₁-C₄)-alkyl or (C₁-C₄)-alkyl substituted by hydroxy, -SO₃M or -COOM; and
M is hydrogen, sodium, potassium, lithium or ammonium.

8. Process for the manufacture of a dyestuff of the formula (I) according to claim 1 which comprises diazotization of a compound of the formula (II) 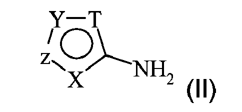 wherein T, X, Y and Z are defined as given in claim 1 and coupling of the resulting diazo component with a compound of the formula (III¹), (III²), (III³), (III⁴) or (III⁵) 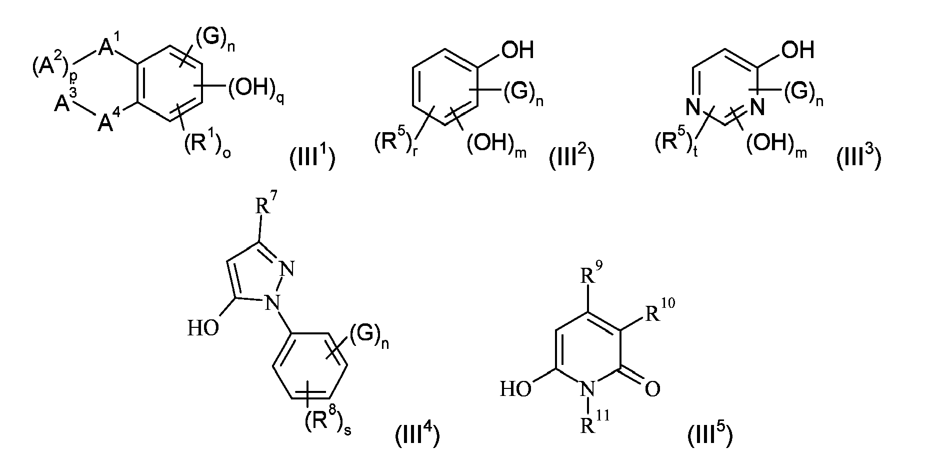 wherein A¹, A², A³, A⁴, G, R¹, R⁵, R⁷, R⁸, R⁹, R¹⁰, R¹¹, m, n, o, p, r, s and t are defined as given in claim 1.

9. Dyestuff mixture comprising one or more dyestuffs of the formula (I) according to claim 1 and one or more direct dyestuffs or precursors of oxidation dyestuffs.

10. Hair dye composition comprising one or more dyestuffs of the formula (I) 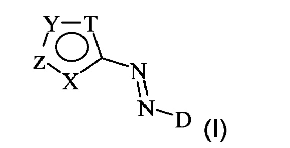 and its tautomeric forms, wherein
D is a group of the formula D¹, D², D³, D⁴ or D⁵ 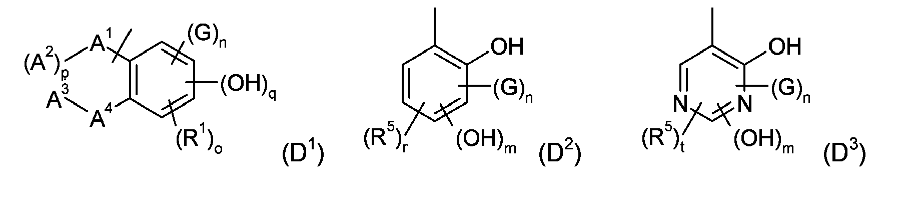 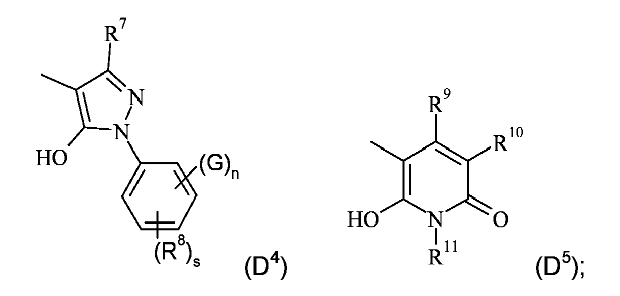 X is oxygen, sulphur, nitrogen, -N(R¹²)- or -C(R¹³)-;
Y is oxygen, sulphur, -C(R¹⁴)-, nitrogen or -N(R¹⁵)-;
Z is nitrogen or -C(R¹⁶)-;
T is nitrogen, sulphur or -C(R¹⁷)-; with the proviso that at least two of the groups X, Y, Z and T are heteroatoms;
each of A¹, A², A³ and A⁴, independently, is oxygen, nitrogen, -N(R¹⁸)-, -C(R¹⁹)=, -C(R²⁰)(R²¹)- or -C(=R²²)-;
G is -SO₃M or -COOM;
M is hydrogen or a cosmetically acceptable, water solubilizing inorganic or organic cation;
n is an integer from 0 to 4, with the proviso, that when n is 0, at least one of the
groups A¹, A², A³, A⁴, X, Y Z and T carries a group G;
m is an integer from 0 to 3;
p is an integer from 0 to 1;
q is an integer from 1 to 4;
r is an integer from 0 to 4;
s is an integer from 0 to 3;
t is an integer from 0 to 2;
o is an integer from 1 to 3;
wherein in the group of the formula D² m+n+r is from 0 to 4 and in the group of the formula D³ m+n+r is from 0 to 2; each of R¹¹, R¹², R¹⁵, R¹⁸ and R²³, independently, is hydrogen; alkyl; arylalkyl; alkoxyalkyl; alkylthioalkyl; poly(ooyalkylene)alkyl; N-acyl-aminoalkyl; N-alkylsulfonyl-aminoalkyl; aminothiocarbonyl-aminoalkyl; aminocarbonyl-aminoalkyl; alkoxyalkylaminoalkyl; alkylthioalkyl-aminoalkyl; aminoalkyloxyalkyl; N-monoalkylamino-alkyloxyalkyl; N,N-dialkyl-aminoalkoxyalkyl; N-arylamino-alkoxyalkyl; N,N-diarylamino-alkoxyalkyl; N-alkyl-N-aryl-amino-alkoxyalkyl; aminoalkylthioalkyl; N-monoalkylamino-alkylthioalkyl; N,N-dialkylamino-alkylthioalkyl; N-arylamino-alkylthioalkyl; N,N-diarylamino-alkylthioalkyl; N-alkyl-N-aryl-amino-alkylthioalkyl; alkenyl; alkynyl; cycloalkyl; cycloalkylalkyl; aryl; aryloxyalkyl; arylthioalkyl; heteroaryl; heteroarylalkyl; heterocycloalkyl; heterocycloalkylalkyl; acyl; benzoyl; alkoxycarbonyl; aryloxycarbonyl; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; acylthio; alkylsulfonyl or arylsulfonyl; or is alkyl substituted by one or more substituents selected from the group consisting of G, hydroxy, amino, N-monoalkyl-amino, N,N-dialkyl-amino, N-monoaryl-amino, N,N-diaryl-amino, N-alkyl-N-aryl-amino, halogen, cyano, nitro, alkoxycarbonyl, alkoxythiocarbonyl; acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoaryl-carbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl and N-monoalkyl-N-monoarylsulfamoyl; each of R¹, R⁵, R⁷, R⁸, R⁹, R¹⁰, R¹³, R¹⁴, R¹⁶, R¹⁷, R¹⁹, R²⁰ and R²¹, independently, is hydrogen; alkyl; trifluoromethyl; alkenyl; alkynyl; cycloalkyl; aryl; heteroaryl; heterocycloalkyl; halogen; cyano; nitro; alkoxy; aryloxy; formyl; iminomethyl; N-alkyl-iminomethyl; N-cycloalkyl-iminomethyl; N-aryl-iminomethyl; hydroxyiminomethyl; thioacyl; arylcarbonyl, arylthiocarbonyl; acyloxy; arylcarbonyloxy; acylamino; N-alkyl-acylamino; N-cycloalkyl-acylamino; N-arylacylamino; alkylsulfonylamino; cycloalkylsulfonylamino, N-alkyl-alkylsulfonylamino; N-alkyl-cycloalkylsulfonylamino; N-cycloalkyl-alkylsulfonylamino; N-cycloalkyl-cycloalkyl-sulfonylamino; N-arylalkylsulfonylamino; N-arylcycloalkylsulfonylamino; arylsulfonylamino; N-alkyl-arylsulfonylamino; N-cycloalkyl-arylsulfonylamino; N-arylarylsulfonylamino; arylcarbonylamino; arylthiocarbonylamino; N-alkyl- arylthiocarbonylamino; N-alkyl-arylcarbonylamino; N-cycloalkyl-arylthiocarbonylamino; N-cycloalkyl-arylcarbonylamino; N-aryl-arylthiocarbonylamino; N-aryl-arylcarbonylamino; carbamoyl; N-monocycloalkyl-carbamoyl; N-monoalkyl-carbamoyl; N,N-dicycloalkyl-carbamoyl; N,N-dialkyl-carbamoyl; N-monoaryl-carbamoyl; N,N-diaryl-carbamoyl; N-monocycloalkyl-N-monoarylcarbamoyl; N-monoalkyl-N-monoarylcarbamoyl; alkoxycarbonyl; aryloxycarbonyl; amino; monocycloalkyl-amino; monoalkyl-amino; di(cyclo)alkyl-amino; dialkyl-amino; monoaryl-amino; diaryl-amino; monocycloalkylmonoarylamino; monoalkylmonoarylamino; aminothiocarbonylamino; aminocarbonylamino; N-N-dicycloalkyl-aminothiocarbonylamino; N-N-dialkyl-aminothiocarbonylamino; N-N-dicycloalkyl-aminocarbonylamino; N-N-dialkyl-aminocarbonylamino; N,N-diaryl-aminothiocarbonylamino; N,N-diaryl-aminocarbonylamino; N,N-dialkylaminothio-carbonyl-N-alkylamino; N,N-dialkylaminocarbonyl-N-alkylamino; alkoxycarbonylamino; aryloxycarbonylamino; sulfamoyl; N-monocycloalkyl-sulfamoyl; N-monoalkyl-sulfamoyl; N,N-dicycloalkyl-sulfamoyl; N,N-dialkyl-sulfamoyl; N-monoaryl-sulfamoyl; N,N-diaryl-sulfamoyl; N-monocycloalkyl-N-monoarylsulfamoyl; N-monoalkyl-N-monoarylsulfamoyl; aminosulfonylamino; N-monocycloalkyl-aminosulfonylamino; N-monoalkyl-aminosulfonylamino; N,N-dicycloalkyl-aminosulfonylamino; N,N-dialkyl-aminosulfonylamino; N-monoaryl-aminosulfonylamino; N,N-diaryl-aminosulfonylamino; N-monocycloalkyl-N-monoarylaminosulfonyl-amino; N-monoalkyl-N-monoarylaminosulfonyl-amino; -S-G; alkylthio; arylthio; alkylsulfonyl; arylsulfonyl; or is alkyl, N-monoalkylamino, N-monocycloalkylamino, N,N-dialkylamino, N,N-dicycloalkylamino, N-alkyl-N-cycloalkylamino, N-alkyl-N-arylamino, N-aryl-N-cycloalkylamino, alkoxy, alkylthio or alkylsulfonyl substituted by one or more substituents selected from the group consisting of hydroxy, cycloalkyl, heteroaryl, heterocycloalkyl, aryl, aryloxy, alkoxy, alkylthio, arykthio. poly(oxyalkylene), G, halogen, cyano, alkoxycarbonyl, alkoxythiocarbonyl, acyloxy, carbamoyl, N-monocycloalkyl-carbamoyl, N-monoalkyl-carbamoyl, N,N-dicycloalkyl-carbamoyl, N,N-dialkyl-carbamoyl, N-monoaryl-carbamoyl, N,N-diaryl-carbamoyl, N-monocycloalkyl-N-monoarylcarbamoyl, N-monoalkyl-N-monoarylcarbamoyl, sulfamoyl, N-monocycloalkyl-sulfamoyl, N-monoalkyl-sulfamoyl, N,N-dicycloalkyl-sulfamoyl, N,N-dialkyl-sulfamoyl, N-monoaryl-sulfamoyl, N,N-diaryl-sulfamoyl, N-monocycloalkyl-N-monoarylsulfamoyl, N-monoalkyl-N-monoarylsulfamoyl, nitro, amino, acylamino, arylcarbonylamino, alkylsulfonylamino, arylsulfonylamino, aminocarbonylamino, aminothiocarbonylamino, N-monoalkylamino, N-mono(hydroxyalkyl)amino, N,N-bis(hydroxyalkyl)amino, N-mono(alkoxyalkyl)amino, N,N-bis(alkoxyalkyl)amino, N-mono(alkoxyalkyl)amino, N-mono(alkylthioalkyl)amino, N-bis(alkylthioalkyl)amino, N-monocycloalkylamino, N-monoaryl-amino, N,N-dialkyl-amino, N,N-dicycloalkylamino, N,N-diaryl-amino, N-alkyl-N-aryl-amino, N-cycloalkyl-N-aryl-amino, aminosulfonylamino, N-monocycloalkyl-aminosulfonylamino, N-monoalkyl-aminosulfonylamino, N,N-dicycloalkyl-aminosulfonylamino, N,N-dialkylaminosulfonylamino, N-monoaryl- aminosulfonylamino, N,N-diaryl-aminosulfonylamino, N-monocycloalkyl-N-monoarylaminosulfonyl-amino, N-monoalkyl-N-monoarylaminosulfonyl-amino and -S-G; and wherein the substituents mentioned above can themselves be substituted by G; and
R⁷, R⁹, R¹⁹, R¹³, R¹⁴, R¹⁶ and R¹⁷ in addition can represent G; and
R¹⁹, R²⁰ and R²¹ in addition can represent hydroxyl;
R²² is oxygen, sulphur or -NR²³-;
wherein when X denotes -C-R¹³ and Z denotes -C-R¹⁶, or Y denotes -C-R¹⁴ and Z denotes -C-R¹⁶, or Y denotes -C-R¹⁴ and T denotes -C-R¹⁷, the groups R¹³ and R¹⁶ and R¹⁴ and R¹⁶ and R¹⁴ and R¹⁷, respectively, together with the carbon atoms to which they are bonded can form a 3- to 8 membered carbocyclic or heterocylic ring, which is saturated, unsaturated or partially unsaturated, and which is unsubstituted or substituted by one or more substituents selected from the group consisting of the substituents for R¹, R², R³, R⁴, R⁵, R⁷, R⁹, R¹⁰, R¹³, R¹⁴, R¹⁶, R¹⁷, R¹⁹, R²⁰ and R²¹ given above.
